# EUROPEAN PATENT SPECIFICATION

(11) **EP 3 713 530 B1**
(45) Date of publication and mention of the grant of the patent: **21.10.2026**
(21) Application number: 18881427.1
(22) Date of filing: 21.11.2018
(51) Int. Cl.: A61K 9/08, A61K 9/06, A61K 47/14, A61K 47/18, A61K 47/26, A61K 47/38, A61K 47/16, A61K 47/10, A61K 47/12, A61M 31/00, A61F 9/00, A61L 12/08, A61M 35/00, B05B 1/02, C07D 211/44, A61M 37/00, A61L 2/00, C07D 451/10

(54) **OPHTHALMIC COMPOSITION AND DELIVERY DEVICE THEREOF**
OPHTHALMISCHE ZUSAMMENSETZUNG UND ABGABEVORRICHTUNG DAFÜR
COMPOSITION OPHTALMIQUE ET DISPOSITIF D'ADMINISTRATION ASSOCIÉ

(30) Priority: 21.11.2017 US 201762589503 P; 11.04.2018 US 201862656174 P
(43) Date of publication of application: 30.09.2020
(73) Proprietor: Sydnexis, Inc., Del Mar, CA 92014 (US)
(72) Inventor: OSTROW, Gregory I., Del Mar California 92014 (US); WIDDER, Kenneth J., Del Mar California 92014 (US); BAKER, David S., Del Mar California 92014 (US)
(74) Representative: Mewburn Ellis LLP
(86) International application number: PCT/US2018/062279
(87) International publication number: WO 2019/104191

(56) References cited:
- WO-A1-2016/025560
- WO-A1-2016/196367
- WO-A2-2010/083129
- WO-A2-2016/172712
- US-A- 5 490 938
- US-A- 5 716 952
- US-A1- 2012 015 035
- US-A1- 2016 339 007
- ABAK: "Pure technology in a bottle The history of a technological r -evolution LABORATOIRES THEA -12 RUE LOUIS BLERIOT -63017 CLERMONT FERRAND -FRANCE Tél", 18 April 2011 (2011-04-18), F-Clermont-Ferrand, pages 1 - 53, XP055822917, Retrieved from the Internet <URL:https://www.laboratoires-thea.com/medias/abak_brochure_eng.pdf>

## Description

### TECHNICAL FIELD

The present invention relates to ophthalmic products for dispensing ophthalmic compositions, more specifically, an ophthalmic product comprising: a) a fluid-dispensing device comprising an internal filter or membrane, a reservoir and a dispensing tip fitted onto the reservoir; and b) an ophthalmic composition comprising from about 0.001 wt% to about 0.05 wt% of a muscarinic antagonist and deuterated water, at a pD of from about 4.2 to about 7.9, in the reservoir; wherein the muscarinic antagonist is atropine or atropine sulfate; wherein the ophthalmic composition is dispensable from the dispensing tip into an eye of an individual in need thereof, and wherein the dispensed ophthalmic composition is preservative-free.

### BACKGROUND OF THE DISCLOSURE

Pharmaceutical formulations have an expiration date which is based on the degradation of the active ingredient.

WO 2016/172712 A2 (Sydnexis Inc.; 27 October 2016) describes an ophthalmic composition comprising a muscarinic antagonist and deuterated water.

### SUMMARY OF THE INVENTION

The invention is an ophthalmic product, comprising:
a) a fluid-dispensing device comprising an internal filter or membrane, a reservoir and a dispensing tip fitted onto the reservoir; and
b) an ophthalmic composition comprising from about 0.001 wt% to about 0.05 wt% of a muscarinic antagonist and deuterated water, at a pD of from about 4.2 to about 7.9, in the reservoir;

wherein the muscarinic antagonist is atropine or atropine sulfate;
wherein the ophthalmic composition is dispensable from the dispensing tip into an eye of an individual in need thereof, and wherein the dispensed ophthalmic composition is preservative-free.

In one embodiment:
the ophthalmic composition has a pD of one of: less than 7.3, less than 7.2, less than 7.1, less than 7, less than 6.8, less than 6.5, less than 6.4, less than 6.3, less than 6.2, less than 6.1, less than 6, less than 5.9, less than 5.8, less than 5.2, or less than 4.8 after an extended period of time under storage condition.

In one embodiment:
the ophthalmic composition comprises one of: less than 1 %, less than 0.5 %, less than 0.4 %, less than 0.3 %, less than 0.2 %, less than 0.1 %, less than 0.01 %, or less than 0.001 % of a preservative.

In one embodiment:
the ophthalmic composition is preservative-free.

In one embodiment:
the internal filter or membrane is located within the fluid-dispensing device at a position capable of removing a preservative from the ophthalmic composition prior to dispensing the ophthalmic composition into the eye of the individual.

In one embodiment:
the internal filter or membrane is located within the fluid-dispensing device at a position capable of removing a microorganism and/or an endotoxin from the ophthalmic composition prior to dispensing the ophthalmic composition into the eye of the individual.

In one embodiment:
the internal filter or membrane comprises cellulose acetate, cellulose nitrate, nylon, polyether sulfone (PES), polypropylene (PP), polyvinyl difluoride (PVDF), silicone, polycarbonate, or a combination thereof.

In one embodiment:
the ophthalmic composition comprises one of: at least 80 %, at least 85 %, at least 90 %, at least 93 %, at least 95 %, at least 97 %, at least 98 %, or at least 99 % of the muscarinic antagonist based on initial concentration after extended period of time under storage condition.

In one embodiment:
the ophthalmic composition has a potency of one of: at least 80 %, at least 85 %, at least 90 %, at least 93 %, at least 95 %, at least 97 %, at least 98 %, or at least 99 % after extended period of time under storage condition.

In one embodiment:
the extended period of time is one of: about 1 week, about 2 weeks, about 3 weeks, about 1 month, about 2 months, about 3 months, about 4 months, about 5 months, about 6 months, about 8 months, about 10 months, about 12 months, about 18 months, about 24 months, about 36 months, about 4 years, or about 5 years.

### BRIEF DESCRIPTION

### Ophthalmic Products

Provided herein is an ophthalmic product comprising an ophthalmic composition. In some embodiments, disclosed herein is an ophthalmic product, comprising: (a) a fluid-dispensing device comprising an internal filter or membrane, a reservoir and a dispensing tip fitted onto the reservoir; and (b) an ophthalmic composition comprising from about 0.001 wt% to about 0.05 wt% of a muscarinic antagonist and deuterated water, at a pD of from about 4.2 to about 7.9, in the reservoir; wherein the muscarinic antagonist is atropine or atropine sulfate; wherein the ophthalmic composition is dispensable from the dispensing tip into an eye of an individual in need thereof, and wherein the dispensed ophthalmic composition is preservative-free.

In some embodiments, the ophthalmic composition has a pD of one of: less than 7.3, less than 7.2, less than 7.1, less than 7, less than 6.8, less than 6.5, less than 6.4, less than 6.3, less than 6.2, less than 6.1, less than 6, less than 5.9, less than 5.8, less than 5.2, or less than 4.8 after an extended period of time under storage condition.

In some embodiments, the ophthalmic composition comprises one of: less than 1%, less than 0.5%, less than 0.4%, less than 0.3%, less than 0.2%, less than 0.1%, less than 0.01%, or less than 0.001% of a preservative.

In some embodiments, the ophthalmic composition is preservative-free.

In some embodiments, the internal filter or membrane is located within the fluid-dispensing device at a position capable of removing a preservative from the ophthalmic composition prior to dispensing the ophthalmic composition into the eye of the individual. In some embodiments, the internal filter or membrane is located within the fluid-dispensing device at a position capable of removing a microorganism and/or an endotoxin from the ophthalmic composition prior to dispensing the ophthalmic composition into the eye of the individual. In some embodiments, the internal filter or membrane comprises cellulose acetate, cellulose nitrate, nylon, polyether sulfone (PES), polypropylene (PP), polyvinyl difluoride (PVDF), silicone, polycarbonate, or a combination thereof.

In some embodiments, the reservoir is at least partially elastically deformable so as to dispense the ophthalmic composition by pressing on the reservoir.

In some embodiments, the fluid-dispensing device optionally comprises an atomizer, a pump, or a mister.

In some embodiments, the reservoir comprises a polymeric material. In some embodiments, the polymeric material comprises polyvinyl chloride (PVC) plastics. In some embodiments, the polymeric material comprises non-PVC plastics. In some embodiments, the polymeric material comprises high-density polyethylene (HDPE), low-density polyethylene (LDPE), polyethylene terephthalate (PET), polyvinyl chloride (PVC), polypropylene (PP), polystyrene (PS), fluorine treated HDPE, post-consumer resin (PCR), K-resin (SBC), or bioplastic. In some embodiments, the polymeric material comprises low-density polyethylene (LDPE).

In some embodiments, the reservoir comprises glass.

In some embodiments, the ophthalmic composition comprises one of: at least 80%, at least 85%, at least 90%, at least 93%, at least 95%, at least 97%, at least 98%, or at least 99% of the muscarinic antagonist based on initial concentration after extended period of time under storage condition.

In some embodiments, the ophthalmic composition has a potency of one of: at least 80%, at least 85%, at least 90%, at least 93%, at least 95%, at least 97%, at least 98%, or at least 99% after extended period of time under storage condition.

In some embodiments, the extended period of time is one of: about 1 week, about 2 weeks, about 3 weeks, about 1 month, about 2 months, about 3 months, about 4 months, about 5 months, about 6 months, about 8 months, about 10 months, about 12 months, about 18 months, about 24 months, about 36 months, about 4 years, or about 5 years.

In some embodiments, the storage condition has a storage temperature of from about 2°C to about 10°C or from about 16°C to about 26°C.

In some embodiments, the muscarinic antagonist is present in the composition at a concentration of one of: from about 0.001 wt% to about 0.04 wt%, from about 0.001 wt% to about 0.03 wt%, from about 0.001 wt% to about 0.025 wt%, from about 0.001 wt% to about 0.02 wt%, from about 0.001 wt% to about 0.01 wt%, from about 0.001 wt% to about 0.008 wt%, or from about 0.001 wt% to about 0.005 wt%.

In some embodiments, the ophthalmic composition further comprises an osmolarity adjusting agent. In some embodiments, the osmolarity adjusting agent is sodium chloride.

In some embodiments, the ophthalmic composition further comprises a buffer agent. In some embodiments, the buffer agent is selected from borates, borate-polyol complexes, phosphate buffering agents, citrate buffering agents, acetate buffering agents, carbonate buffering agents, organic buffering agents, amino acid buffering agents, or combinations thereof.

In some embodiments, the ophthalmic composition has one of: less than about 60 colony forming units (CFU), less than about 50 colony forming units, less than about 40 colony forming units, or less than about 30 colony forming units of microbial agents per gram of formulation.

In some embodiments, the ophthalmic composition is substantially free of microorganism.

In some embodiments, the ophthalmic composition is substantially free of endotoxins.

In some embodiments, the ophthalmic composition is essentially free of procaine and benactyzine, or pharmaceutically acceptable salts thereof.

In some embodiments, the ophthalmic composition has a dose-to-dose muscarinic antagonist concentration variation of one of: less than 50%, less than 40%, less than 30%, less than 20%, less than 10%, or less than 5%.

In some embodiments, the dose-to-dose muscarinic antagonist concentration variation is based on one of: 10 consecutive doses, 8 consecutive doses, 5 consecutive doses, 3 consecutive doses, or 2 consecutive doses.

In some embodiments, the ophthalmic composition further comprises a pD adjusting agent. In some embodiments, the pD adjusting agent comprises DCl, NaOD, CD₃COOD, or C₆D₈O₇.

In some embodiments, the ophthalmic composition comprises one of: less than 5% of H₂O, less than 4% of H₂O, less than 3% of H₂O, less than 2% of H₂O, less than 1% of H₂O, less than 0.5% of H₂O, less than 0.1% of H₂O, or 0% of H₂O.

In some embodiments, the ophthalmic composition is formulated as an ophthalmic solution for the treatment of pre-myopia, myopia, or progression of myopia.

In some embodiments, the fluid-dispensing device is a multi-dose preservative-free device.

In some embodiments, the fluid-dispensing device enables dispensing a preservative-free ophthalmic composition.

In some embodiments, disclosed herein is an ophthalmic product, comprising: (a) a multi-dose preservative free fluid-dispensing device comprising an internal filter or membrane, a reservoir and a dispensing tip fitted onto the reservoir; and (b) an ophthalmic composition comprising from about 0.001 wt% to about 0.05 wt% of a muscarinic antagonist and deuterated water, at a pD of from about 4.2 to about 7.9, in the reservoir; wherein the muscarinic antagonist is atropine or atropine sulfate; wherein the ophthalmic composition is dispensable from the dispensing tip into an eye of an individual in need thereof, and wherein the ophthalmic composition is preservative-free.

### Ophthalmic Composition suitable for being delivered

Also described herein is an ophthalmic composition suitable for being delivered to an eye of an individual in need thereof, comprising: (a) generating at least one droplet containing an ophthalmic composition comprising from about 0.001 wt% to about 0.05 wt% of a muscarinic antagonist and deuterated water, at a pD of from about 4.2 to about 7.9, via a fluid-dispensing device comprising an internal filter or membrane, a reservoir and a dispensing tip fitted onto the reservoir; and (b) delivering the at least one droplet containing said ophthalmic composition to the eye of the individual; wherein the muscarinic agonist is atropine or atropine sulfate, wherein the ophthalmic composition dispensed in step b) is substantially preservative-free.

In some instances, the individual has pre-myopia or myopia.

In some instances, the ophthalmic composition has a pD of one of: less than 7.3, less than 7.2, less than 7.1, less than 7, less than 6.8, less than 6.5, less than 6.4, less than 6.3, less than 6.2, less than 6.1, less than 6, less than 5.9, less than 5.8, less than 5.2, or less than 4.8 after an extended period of time under storage condition.

In some instances, the ophthalmic composition comprises one of: less than 1%, less than 0.5%, less than 0.4%, less than 0.3%, less than 0.2%, less than 0.1%, less than 0.01%, or less than 0.001% of a preservative.

In some instances, the ophthalmic composition is preservative-free.

In some instances, the internal filter or membrane is located within the fluid-dispensing device at a position capable of removing a preservative from the ophthalmic composition prior to dispensing the ophthalmic composition into the eye of the individual.

In some instances, the internal filter or membrane is located within the fluid-dispensing device at a position capable of removing a microorganism and/or an endotoxin from the ophthalmic composition prior to dispensing the ophthalmic composition into the eye of the individual. In some instances, the internal filter or membrane comprises cellulose acetate, cellulose nitrate, nylon, polyether sulfone (PES), polypropylene (PP), polyvinyl difluoride (PVDF), silicone, polycarbonate, or a combination thereof.

According to the invention, the dispensed ophthalmic composition is preservative-free.

In some instances, the reservoir is at least partially elastically deformable so as to dispense the ophthalmic composition by pressing on the reservoir.

In some instances, the fluid-dispensing device optionally comprises an atomizer, a pump, or a mister.

In some instances, the reservoir comprises a polymeric material. In some instances, the polymeric material comprises polyvinyl chloride (PVC) plastics. In some instances, the polymeric material comprises non-PVC plastics. In some instances, the polymeric material comprises high-density polyethylene (HDPE), low-density polyethylene (LDPE), polyethylene terephthalate (PET), polyvinyl chloride (PVC), polypropylene (PP), polystyrene (PS), fluorine treated HDPE, post-consumer resin (PCR), K-resin (SBC), or bioplastic. In some instances, the polymeric material comprises low-density polyethylene (LDPE).

In some instances, the reservoir comprises glass.

In some instances, the reservoir stores multiple unit doses of the ophthalmic composition.

In some instances, the ophthalmic composition comprises one of: at least 80%, at least 85%, at least 90%, at least 93%, at least 95%, at least 97%, at least 98%, or at least 99% of the muscarinic antagonist based on initial concentration after extended period of time under storage condition.

In some instances, the ophthalmic composition has a potency of one of: at least 80%, at least 85%, at least 90%, at least 93%, at least 95%, at least 97%, at least 98%, or at least 99% after extended period of time under storage condition.

In some instances, the extended period of time is one of: about 1 week, about 2 weeks, about 3 weeks, about 1 month, about 2 months, about 3 months, about 4 months, about 5 months, about 6 months, about 8 months, about 10 months, about 12 months, about 18 months, about 24 months, about 36 months, about 4 years, or about 5 years.

In some instances, the storage condition has a storage temperature of from about 2°C to about 10°C or from about 16°C to about 26°C.

In some instances, the muscarinic antagonist is present in the composition at a concentration of one of: from about 0.001 wt% to about 0.04 wt%, from about 0.001 wt% to about 0.03 wt%, from about 0.001 wt% to about 0.025 wt%, from about 0.001 wt% to about 0.02 wt%, from about 0.001 wt% to about 0.01 wt%, from about 0.001 wt% to about 0.008 wt%, or from about 0.001 wt% to about 0.005 wt%.

In some instances, the ophthalmic composition further comprises an osmolarity adjusting agent. In some instances, the osmolarity adjusting agent is sodium chloride.

In some instances, the ophthalmic composition further comprises a buffer agent. In some instances, the buffer agent is selected from borates, borate-polyol complexes, phosphate buffering agents, citrate buffering agents, acetate buffering agents, carbonate buffering agents, organic buffering agents, amino acid buffering agents, or combinations thereof.

In some instances, the ophthalmic composition has one of: less than about 60 colony forming units (CFU), less than about 50 colony forming units, less than about 40 colony forming units, or less than about 30 colony forming units of microbial agents per gram of formulation.

In some instances, the ophthalmic composition is substantially free of microorganism.

In some instances, the ophthalmic composition is substantially free of endotoxins.

In some instances, the ophthalmic composition is essentially free of procaine and benactyzine, or pharmaceutically acceptable salts thereof.

In some instances, the ophthalmic composition has a dose-to-dose muscarinic antagonist concentration variation of one of: less than 50%, less than 40%, less than 30%, less than 20%, less than 10%, or less than 5%.

In some instances, the dose-to-dose muscarinic antagonist concentration variation is based on one of: 10 consecutive doses, 8 consecutive doses, 5 consecutive doses, 3 consecutive doses, or 2 consecutive doses.

In some instances, the ophthalmic composition further comprises a pD adjusting agent. In some instances, the pD adjusting agent comprises DCl, NaOD, CD₃COOD, or C₆D₈O₇.

In some instances, the ophthalmic composition comprises one of: less than 5% of H₂O, less than 4% of H₂O, less than 3% of H₂O, less than 2% of H₂O, less than 1% of H₂O, less than 0.5% of H₂O, less than 0.1% of H₂O, or 0% of H₂O.

In some instances, the ophthalmic composition is an ophthalmic solution.

In some instances, at least 60%, 70%, 80%, 85%, 90%, 95%, or 99% of the ejected mass of the at least one droplet is deposited on the eye.

In some instances, the individual is a human.

### Ophthalmic Compositions in the Ophthalmic Product

Also described herein is an ophthalmic composition, comprising from about 0.001 wt% to about 0.05 wt% of a muscarinic antagonist and deuterated water, at a pD of from about 4.2 to about 7.9, wherein the muscarinic antagonist is atropine, or atropine sulfate, and wherein the ophthalmic composition is substantially preservative-free.

In some instances, the ophthalmic composition comprises one of: less than 1%, less than 0.5%, less than 0.4%, less than 0.3%, less than 0.2%, less than 0.1%, less than 0.01%, less than 0.001%, or less than 0.0001% of a preservative.

In some instances, the ophthalmic composition is preservative-free.

In some instances, the ophthalmic composition has a pD of one of: less than 7.3, less than 7.2, less than 7.1, less than 7, less than 6.8, less than 6.5, less than 6.4, less than 6.3, less than 6.2, less than 6.1, less than 6, less than 5.9, less than 5.8, less than 5.2, or less than 4.8 after extended period of time under storage condition.

In some instances, the ophthalmic composition comprises one of: at least 80%, at least 85%, at least 90%, at least 93%, at least 95%, at least 97%, at least 98%, or at least 99% of the muscarinic antagonist based on initial concentration after extended period of time under storage condition.

In some instances, the ophthalmic composition has a potency of one of: at least 80%, at least 85%, at least 90%, at least 93%, at least 95%, at least 97%, at least 98%, or at least 99% after extended period of time under storage condition.

In some instances, the extended period of time is one of: about 1 week, about 2 weeks, about 3 weeks, about 1 month, about 2 months, about 3 months, about 4 months, about 5 months, about 6 months, about 8 months, about 10 months, about 12 months, about 18 months, about 24 months, about 36 months, about 4 years, or about 5 years.

In some instances, the storage condition has a storage temperature of from about 2°C to about 10°C or from about 16°C to about 26°C.

In some instances, the muscarinic antagonist is present in the composition at a concentration of one of: from about 0.001 wt% to about 0.04 wt%, from about 0.001 wt% to about 0.03 wt%, from about 0.001 wt% to about 0.025 wt%, from about 0.001 wt% to about 0.02 wt%, from about 0.001 wt% to about 0.01 wt%, from about 0.001 wt% to about 0.008 wt%, or from about 0.001 wt% to about 0.005 wt%.

In some instances, the ophthalmic composition further comprises an osmolarity adjusting agent. In some instances, the osmolarity adjusting agent is sodium chloride.

In some instances, the ophthalmic composition further comprises a buffer agent. In some instances, the buffer agent is selected from borates, borate-polyol complexes, phosphate buffering agents, citrate buffering agents, acetate buffering agents, carbonate buffering agents, organic buffering agents, amino acid buffering agents, or combinations thereof.

In some instances, the ophthalmic composition is essentially free of procaine and benactyzine, or pharmaceutically acceptable salts thereof.

In some instances, the ophthalmic composition has one of: less than about 60 colony forming units (CFU), less than about 50 colony forming units, less than about 40 colony forming units, or less than about 30 colony forming units of microbial agents per gram of formulation.

In some instances, the ophthalmic composition is substantially free of microorganism.

In some instances, the ophthalmic composition is substantially free of endotoxins.

In some instances, the ophthalmic composition has a dose-to-dose muscarinic antagonist concentration variation of one of: less than 50%, less than 40%, less than 30%, less than 20%, less than 10%, or less than 5%.

In some instances, the dose-to-dose muscarinic antagonist concentration variation is based on one of: 10 consecutive doses, 8 consecutive doses, 5 consecutive doses, 3 consecutive doses, or 2 consecutive doses.

In some instances, the ophthalmic composition further comprises a pD adjusting agent. In some instances, the pD adjusting agent comprises DCl, NaOD, CD₃COOD, or C₆D₈O₇.

In some instances, the ophthalmic composition comprises one of: less than 5% of H₂O, less than 4% of H₂O, less than 3% of H₂O, less than 2% of H₂O, less than 1% of H₂O, less than 0.5% of H₂O, less than 0.1% of H₂O, or 0% of H₂O.

In some instances, the ophthalmic composition is not formulated as an injectable formulation.

In some instances, the ophthalmic composition is formulated as an ophthalmic solution for the treatment of pre-myopia, myopia, or progression of myopia.

Other features and technical effects of the methods and compositions described herein will become apparent from the following detailed description. It should be understood, however, that the the specific examples, while indicating specific embodiments, are given by way of illustration only.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

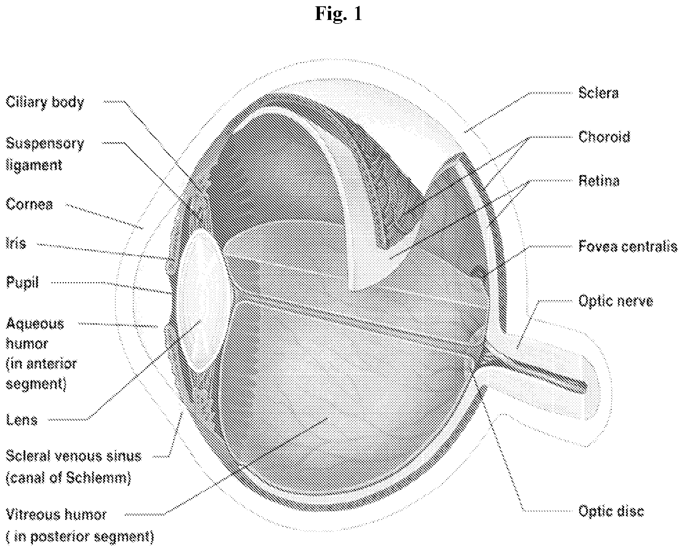

Fig. 1 illustrates a conceptual representation of the eye anatomy.

### DETAILED DESCRIPTION

The present disclosure recognizes that there is a need for a stabilized ophthalmic composition with extended shelf life upon storage. The present disclosure also recognizes that there is a need for stabilizing an ophthalmic composition through arresting or reducing hydrolysis of at least some of its active agents. The present disclosure further recognizes that there is a need for an ophthalmic composition that provides convenient and effective delivery of a muscarinic antagonist such as atropine in the eye of a patient.

Preservatives are added to an ophthalmic composition to prevent growth of microorganisms during storage and to maintain sterility of the solution. In some instances, sensitivity and/or allergic reactions have been attributed to the presence of preservatives. For example, the salts of benzalkonium have been classified as being moderately allergic whereas mercurial products are strongly allergic. In some cases, thimerosal causes ocular delayed hypersensitivity. Chlorhexidine has been shown to cause corneal endothelium damage. Parabens are capable of producing immunologically mediated, immediate systemic hypersensitivity reactions. In additional cases, the use of preservative containing eye drops has been implicated in the development or worsening of ocular surface disease.

The present disclosure recognizes that delivery of preservative-free compositions comprising an ophthalmic drug. In some instances, the ophthalmic drug comprises a muscarinic antagonist, atropine or atropine sulfate, for treating, preventing, and/or arresting the development of myopia in humans, for example as evidenced by reduction of the rate of increase of myopia in young people, while further minimizing the risk of developing an adverse effect (e.g., a sensitivity or an allergic reaction due to the presence of a preservative). The present disclosure also recognizes the effects of said muscarinic antagonist on reduction of axial elongation and myopia in visually impaired chick eyes, and on ocular growth and muscarinic cholinergic receptors in young rhesus monkeys.

In addition, the present disclosure recognizes that systemic absorption of said muscarinic antagonist sometimes leads to undesirable side effect, and that localized delivery of muscarinic antagonist (e.g. atropine or its pharmaceutically acceptable salts) reduces or prevents the aforementioned systemic exposure.

Further, the present disclosure recognizes that the claimed muscarinic antagonist compositions are formulated at a relatively lower pH range (e.g. less than 4.5) for stability of the muscarinic antagonist. For some individuals, the lower pH range in some instances causes discomfort or other side effects such as pain or burning sensation in the eye, which is prevented or alleviated by formulating muscarinic antagonist (e.g. atropine) compositions at higher pH ranges. For some individuals, the lower pH in some instances elicits a tear response which reduces the absorption of the drug in the eye and therefore the effectiveness.

Still further, the present disclosure recognizes that the claimed muscarinic antagonist (e.g. atropine) liquid compositions formulated at lower concentrations (e.g. 0.001% to 0.05%) present stability challenges that are less so in higher concentrations (e.g. 0.1-1%). Without wishing to be bound by any particular theory, it is contemplated that the muscarinic antagonist (e.g. atropine) contributes to the stability of an ophthalmic composition, such as an aqueous solution. For example, the concentration of the muscarinic antagonist (e.g. atropine) in some embodiments affects the pH or pD of the ophthalmic composition, such as with the muscarinic antagonist acting as a buffering agent. Furthermore, the concentration of the muscarinic antagonist (e.g. atropine) in some embodiments affects the interaction between the muscarinic antagonist and other ingredients of the ophthalmic composition, which in turn affects the stability of the ophthalmic composition.

Finally, the present disclosure recognizes that deuterated water stabilizes ophthalmic compositions. In some cases, the deuterated water is a weak acid as compared to H₂O, as such deuterated water comprises a lower concentration of the reactive species (e.g., -OD) which in some instances leads to base catalyzed hydrolysis of an active agent in the ophthalmic composition. As such, in some instances compositions comprising deuterated water leads to reduced base catalyzed hydrolysis when compared to compositions comprising H₂O. In some instances, deuterated water further lowers the buffering capacity of an ophthalmic composition, leading to less tear reflex in the eye.

Myopia, axial elongation of the eye, affects a large proportion of the population. The onset of myopia is generally during the grade school years and progresses until growth of the eye is completed. The present disclosure recognizes the importance of compositions and treatments for preventing or arresting the development of myopia, especially compositions and treatments that allow convenient administration, reduce potential side effects, have suitable stability, and/or provide relatively consistent therapeutic effects.

### Ophthalmic Muscarinic Antagonist Composition

Provided herein is an ophthalmic product comprising an ophthalmic composition containing low concentrations of an ophthalmic drug. In some embodiments, the ophthalmic composition includes from about 0.001 wt% to about 0.05 wt% of an ophthalmic drug for treatment of an ophthalmic disorder or condition; and an ophthalmically acceptable carrier, wherein the ophthalmic drug is distributed with substantial uniformity throughout the ophthalmically acceptable carrier. The ophthalmic drug is a muscarinic antagonist.

Provided herein is an ophthalmic product comprising an ophthalmic composition containing low concentrations of a muscarinic antagonist. In some embodiments, the ophthalmic composition includes from about 0.001 wt% to about 0.05 wt% of a muscarinic antagonist for treatment of an ophthalmic disorder or condition; and an ophthalmically acceptable carrier, wherein the muscarinic antagonist is distributed with substantial uniformity throughout the ophthalmically acceptable carrier.

The muscarinic antagonist is atropine or atropine sulfate. In some embodiments, the muscarinic antagonist is atropine. In some embodiments, the muscarinic antagonist is atropine sulfate.

The ophthalmic composition comprise a muscarinic antagonist selected from atropine and atropine sulfate.

In some embodiments, the ophthalmic composition comprises a muscarinic antagonist selected from atropine and atropine sulfate, in combination with one or more sympathetic agonists. In some embodiments, the sympathetic agonist is selected from phenylephrine or hydroxyamphetamine.

Provided herein is an ophthalmic product comprising an ophthalmic composition containing low concentrations of atropine or its pharmaceutically acceptable salts. In some embodiments, the ophthalmic composition includes from about 0.001 wt% to about 0.05 wt% of atropine or its pharmaceutically acceptable salts for treatment of an ophthalmic disorder or condition; and an ophthalmically acceptable carrier, wherein the ophthalmic drug is distributed with substantial uniformity throughout the ophthalmically acceptable carrier.

Provided herein is an ophthalmic product comprising an ophthalmic composition containing low concentrations of atropine sulfate. In some embodiments, the ophthalmic composition includes from about 0.001 wt% to about 0.05 wt% of atropine sulfate for treatment of an ophthalmic disorder or condition; and an ophthalmically acceptable carrier, wherein the ophthalmic drug is distributed with substantial uniformity throughout the ophthalmically acceptable carrier.

In some embodiments, the ophthalmic disorder or condition is pre-myopia, myopia or progression of myopia.

The present disclosure further recognizes that the clinical use of atropine as a therapy has been limited due to its ocular side effects including glare from pupillary dilation and blurred vision due to loss of accommodation. Without wishing to be bound by any particular theory, it is contemplated that the limited use of atropine against myopia development, include its ocular side effects, is attributable to the concentration of atropine used in known ophthalmic formulations (e.g. 1wt% or higher).

The present disclosure further recognizes the challenges present in formulation of compositions that contain low concentrations, especially very low concentrations (e.g. from about 0.001 wt% to about 0.5 wt%), of ophthalmic drugs, such as muscarinic antagonist (e.g. atropine or its pharmaceutically acceptable salts). In particular, pharmaceutical compositions with ophthalmic drug at such low concentrations are difficult to maintain dose-to-dose uniformity in term of ophthalmic drug content and/or distribution.

According to the invention, formulations or solutions of muscarinic antagonist (e.g., atropine) are formulated in deuterated water. In some embodiments, the formulations or solutions of muscarinic antagonist (e.g., atropine) formulated in deuterated water are stable at different temperatures, at different relative humidity, with an acidic pD, and with a potency of at least 80% relative to the ophthalmic drug. In additional embodiments, the formulations or solutions of muscarinic antagonist (e.g., atropine) formulated in deuterated water has a lowered buffering capacity. In such instances, the lowered buffering capacity of the ophthalmic formulations or solutions when administered into the eye allows the ophthalmic formulation or solution to reach physiological pH at a faster rate than compared to an equivalent ophthalmic formulation or solution formulated in H₂O.

In some instances, described herein are formulations or solutions of deuterated muscarinic antagonist (e.g., deuterated atropine). In some cases, formulations or solutions of deuterated muscarinic antagonist (e.g., atropine) are stable at different temperatures, at different relative humidity, with an acidic pD, and with a potency of at least 80% relative to the ophthalmic drug.

In some embodiments, the formulations of muscarinic antagonist (e.g. atropine or deuterated atropine) at low concentrations do not have a dose-to-dose variation. In some embodiments, the formulations of muscarinic antagonist (e.g. atropine) at low concentrations are stable at different temperatures, at different relative humidity, with an acidic pD, and with a potency of at least 80% relative to the ophthalmic drug.

The ophthalmic composition may be formulated as an ophthalmic gel or an ophthalmic ointment. For example, an ophthalmic gel or an ophthalmic ointment described herein may allow desirable dose-to-dose uniformity, reduced or limited systemic exposure, or combinations thereof.

### Ophthalmic Solution Muscarinic Antagonist Composition

Disclosed herein, in certain embodiments, is an ophthalmic composition formulated as an aqueous solution. The ophthalmic composition comprises from about 0.001 wt% to about 0.05 wt% of a muscarinic antagonist and deuterated water. As used herein, deuterated water refers to D₂O, DHO, heavy water, and/or deuterium oxide.

In some embodiments, the composition comprises at least about 80% of the ophthalmic drug (e.g. muscarinic antagonist) for an extended period of time under storage condition. In some embodiments, the composition comprises at least about 81% of the ophthalmic drug (e.g. muscarinic antagonist) for an extended period of time under storage condition. In some embodiments, the composition comprises at least about 82% of the ophthalmic drug (e.g. muscarinic antagonist) for an extended period of time under storage condition. In some embodiments, the composition comprises at least about 83% of the ophthalmic drug (e.g. muscarinic antagonist) for an extended period of time under storage condition. In some embodiments, the composition comprises at least about 84% of the ophthalmic drug (e.g. muscarinic antagonist) for an extended period of time under storage condition. In some embodiments, the composition comprises at least about 85% of the ophthalmic drug (e.g. muscarinic antagonist) for an extended period of time under storage condition. In some embodiments, the composition comprises at least about 86% of the ophthalmic drug (e.g. muscarinic antagonist) for an extended period of time under storage condition. In some embodiments, the composition comprises at least about 87% of the ophthalmic drug (e.g. muscarinic antagonist) for an extended period of time under storage condition. In some embodiments, the composition comprises at least about 88% of the ophthalmic drug (e.g. muscarinic antagonist) for an extended period of time under storage condition. In some embodiments, the composition comprises at least about 89% of the ophthalmic drug (e.g. muscarinic antagonist) for an extended period of time under storage condition. In some embodiments, the composition comprises at least about 90% of the ophthalmic drug (e.g. muscarinic antagonist) for an extended period of time under storage condition. In some embodiments, the composition comprises at least about 91% of the ophthalmic drug (e.g. muscarinic antagonist) for an extended period of time under storage condition. In some embodiments, the composition comprises at least about 92% of the ophthalmic drug (e.g. muscarinic antagonist) for an extended period of time under storage condition. In some embodiments, the composition comprises at least about 93% of the ophthalmic drug (e.g. muscarinic antagonist) for an extended period of time under storage condition. In some embodiments, the composition comprises at least about 94% of the ophthalmic drug (e.g. muscarinic antagonist) for an extended period of time under storage condition. In some embodiments, the composition comprises at least about 95% of the ophthalmic drug (e.g. muscarinic antagonist) for an extended period of time under storage condition. In some embodiments, the composition comprises at least about 96% of the ophthalmic drug (e.g. muscarinic antagonist) for an extended period of time under storage condition. In some embodiments, the composition comprises at least about 97% of the ophthalmic drug (e.g. muscarinic antagonist) for an extended period of time under storage condition. In some embodiments, the composition comprises at least about 98% of the ophthalmic drug (e.g. muscarinic antagonist) for an extended period of time under storage condition. In some embodiments, the composition comprises at least about 99% of the ophthalmic drug (e.g. muscarinic antagonist) for an extended period of time under storage condition.

In some embodiments, the composition has a potency of at least about 80% after extended period of time under storage condition. In some embodiments, the composition has a potency of at least about 81% after extended period of time under storage condition. In some embodiments, the composition has a potency of at least about 82% after extended period of time under storage condition. In some embodiments, the composition has a potency of at least about 83% after extended period of time under storage condition. In some embodiments, the composition has a potency of at least about 84% after extended period of time under storage condition. In some embodiments, the composition has a potency of at least about 85% after extended period of time under storage condition. In some embodiments, the composition has a potency of at least about 86% after extended period of time under storage condition. In some embodiments, the composition has a potency of at least about 87% after extended period of time under storage condition. In some embodiments, the composition has a potency of at least about 88% after extended period of time under storage condition. In some embodiments, the composition has a potency of at least about 89% after extended period of time under storage condition. In some embodiments, the composition has a potency of at least 90% after extended period of time under storage condition. In some embodiments, the composition has a potency of at least 91% after extended period of time under storage condition. In some embodiments, the composition has a potency of at least 92% after extended period of time under storage condition. In some embodiments, the composition has a potency of at least 93% after extended period of time under storage condition. In some embodiments, the composition has a potency of at least 94% after extended period of time under storage condition. In some embodiments, the composition has a potency of at least 95% after extended period of time under storage condition. In some embodiments, the composition has a potency of at least 96% after extended period of time under storage condition. In some embodiments, the composition has a potency of at least 97% after extended period of time under storage condition. In some embodiments, the composition has a potency of at least 98% after extended period of time under storage condition. In some embodiments, the composition has a potency of at least 99% after extended period of time under storage condition.

In some embodiments, the extended period of time is at least 1 week. In some embodiments, the extended period of time is at least 2 weeks. In some embodiments, the extended period of time is at least 3 weeks. In some embodiments, the extended period of time is at least 1 month. In some embodiments, the extended period of time is at least 2 months. In some embodiments, the extended period of time is at least 3 months. In some embodiments, the extended period of time is at least 4 months. In some embodiments, the extended period of time is at least 5 months. In some embodiments, the extended period of time is at least 6 months. In some embodiments, the extended period of time is at least 7 months. In some embodiments, the extended period of time is at least 8 months. In some embodiments, the extended period of time is at least 9 months. In some embodiments, the extended period of time is at least 10 months. In some embodiments, the extended period of time is at least 11 months. In some embodiments, the extended period of time is at least 12 months (i.e. 1 year). In some embodiments, the extended period of time is at least 18 months (i.e. 1.5 years). In some embodiments, the extended period of time is at least 24 months (i.e. 2 years). In some embodiments, the extended period of time is at least 36 months (i.e. 3 years). In some embodiments, the extended period of time is at least 3 years. In some embodiments, the extended period of time is at least 5 years, or more.

In some embodiments, the temperature of the storage condition is between about 20°C and about 70°C. In some embodiments, the temperature of the storage condition is between about 25°C and about 65°C, about 30°C and about 60°C, about 35°C and about 55°C, or about 40°C and about 50°C. In some embodiments, the temperature of the storage condition is about 25°C. In some embodiments, the temperature of the storage condition is about 40°C. In some embodiments, the temperature of the storage condition is about 60°C.

In some embodiments, the relative humidity of the storage condition is between about 50% and about 80%, or between about 60% and about 75%. In some embodiments, the relative humidity of the storage condition is about 60%. In some embodiments, the relative humidity of the storage condition is about 75%.

In some embodiments, the composition comprises less than 60% of H₂O. In some embodiments, the composition comprises less than 55% of H₂O. In some embodiments, the composition comprises less than 50% of H₂O. In some embodiments, the composition comprises less than 45% of H₂O. In some embodiments, the composition comprises less than 40% of H₂O. In some embodiments, the composition comprises less than 35% of H₂O. In some embodiments, the composition comprises less than 30% of H₂O. In some embodiments, the composition comprises less than 25% of H₂O. In some embodiments, the composition comprises less than 20% of H₂O. In some embodiments, the composition comprises less than 15% of H₂O. In some embodiments, the composition comprises less than 10% of H₂O.

In some embodiments, the composition comprises from less than 5% of H₂O to 0% of H₂O. In some embodiments, the composition comprises less than 5% of H₂O. In some embodiments, the composition comprises less than 4.5% of H₂O. In some embodiments, the composition comprises less than 4% of H₂O. In some embodiments, the composition comprises less than 3.5% of H₂O. In some embodiments, the composition comprises less than 3% of H₂O. In some embodiments, the composition comprises less than 2.5% of H₂O. In some embodiments, the composition comprises less than 2% of H₂O. In some embodiments, the composition comprises less than 1.5% of H₂O. In some embodiments, the composition comprises less than 1% of H₂O. In some embodiments, the composition comprises less than 0.5% of H₂O. In some embodiments, the composition comprises less than 0.4% of H₂O. In some embodiments, the composition comprises less than 0.3% of H₂O. In some embodiments, the composition comprises less than 0.2% of H₂O. In some embodiments, the composition comprises less than 0.1% of H₂O. In some embodiments, the composition comprises 0% of H₂O.

The composition has a pD of from about 4.2 to about 7.9. In some embodiments, the composition has a pD of between about 4.5 and about 7.8, about 5 and about 7.5, or about 5.5 and about 7. In some embodiments, the composition has a pD of less than about 7.5. In some embodiments, the composition has a pD of less than about 7.4. In some embodiments, the composition has a pD of less than about 7.3. In some embodiments, the composition has a pD of less than about 7.2. In some embodiments, the composition has a pD of less than about 7.1. In some embodiments, the composition has a pD of less than about 7. In some embodiments, the composition has a pD of less than about 6.9. In some embodiments, the composition has a pD of less than about 6.8. In some embodiments, the composition has a pD of less than about 6.7. In some embodiments, the composition has a pD of less than about 6.6. In some embodiments, the composition has a pD of less than about 6.5. In some embodiments, the composition has a pD of less than about 6.4. In some embodiments, the composition has a pD of less than about 6.3. In some embodiments, the composition has a pD of less than about 6.2. In some embodiments, the composition has a pD of less than about 6.1. In some embodiments, the composition has a pD of less than about 6. In some embodiments, the composition has a pD of less than about 5.9. In some embodiments, the composition has a pD of less than about 5.8. In some embodiments, the composition has a pD of less than about 5.7. In some embodiments, the composition has a pD of less than about 5.6. In some embodiments, the composition has a pD of less than about 5.5. In some embodiments, the composition has a pD of less than about 5.4. In some embodiments, the composition has a pD of less than about 5.3. In some embodiments, the composition has a pD of less than about 5.2. In some embodiments, the composition has a pD of less than about 5.1. In some embodiments, the composition has a pD of less than about 5. In some embodiments, the composition has a pD of less than about 4.9. In some embodiments, the composition has a pD of less than about 4.8. In some embodiments, the composition has a pD of less than about 4.7. In some embodiments, the composition has a pD of less than about 4.6. In some embodiments, the composition has a pD of less than about 4.5. In some embodiments, the composition has a pD of less than about 4.4. In some embodiments, the composition has a pD of less than about 4.3.

In some embodiments, the composition comprising deuterated water has a lowered buffering capacity than an equivalent composition comprising H₂O. As described elsewhere herein, in some embodiments, the lowered buffering capacity allows the composition comprising deuterated water to normalize to physiological pH at a faster rate than a composition comprising H₂O. In some embodiments, the lowered buffering capacity allows the composition to induce less tear reflex than an equivalent composition comprising H₂O.

In some instances, the composition comprising deuterated water stabilizes muscarinic antagonist (e.g., atropine). In some embodiments, this is due to a lower concentration of the reactive species (e.g., -OD) in the D₂O aqueous system compared to the concentration of the reactive species (e.g., -OH) in an equivalent H₂O aqueous system. In some cases, base catalyzed hydrolysis leads to the presence of tropine degradant from atropine. In some cases, with a lower concentration of the reactive species that causes tropine degradant formation, atropine solution is more stable in a D₂O aqueous system than compared to an equivalent H₂O aqueous system. In some embodiments, the ophthalmic composition formulated with deuterated water allows for a more stable ophthalmic composition relative to the ophthalmic composition formulated with H₂O.

In some embodiments, the composition comprises less than 20% of major degradant based on the concentration of the ophthalmic drug after extended period of time under storage condition. In some embodiments, the composition comprises less than 15% of major degradant based on the concentration of the ophthalmic drug after extended period of time under storage condition.

In some embodiments, the composition comprises less than 10% of major degradant based on the concentration of the ophthalmic drug after extended period of time under storage condition. In some embodiments, the composition comprises less than 5% of major degradant based on the concentration of the ophthalmic drug after extended period of time under storage condition. In some embodiments, the composition comprises less than 2.0% of major degradant based on the concentration of the ophthalmic drug after extended period of time under storage condition. In some embodiments, the composition comprises less than 1.5% of major degradant based on the concentration of the ophthalmic drug after extended period of time under storage condition. In some embodiments, the composition comprises less than 1.0% of major degradant based on the concentration of the ophthalmic drug after extended period of time under storage condition. In some embodiments, the composition comprises less than 0.5% of major degradant based on the concentration of the ophthalmic drug after extended period of time under storage condition. In some embodiments, the composition comprises less than 0.4% of major degradant based on the concentration of the ophthalmic drug after extended period of time under storage condition. In some embodiments, the composition comprises less than 0.3% of major degradant based on the concentration of the ophthalmic drug after extended period of time under storage condition. In some embodiments, the composition comprises less than 0.2% of major degradant based on the concentration of the ophthalmic drug after extended period of time under storage condition. In some embodiments, the composition comprises less than 0.1% of major degradant based on the concentration of the ophthalmic drug after extended period of time under storage condition. In some embodiments, the major degradant is tropic acid.

In some embodiments, the primary degradant is an early eluting related substance at RRT of 0.87-0.89 according to the UPLC method described herein (Table 10). In some instances, the early eluting related substance is referred to as RRT 0.87-0.89. In some embodiments, the primary degradant is RRT 0.87-0.89.

### Ophthalmic Muscarinic Antagonist Concentration

According to the invention, the compositions described herein have a concentration of ophthalmic drug between about 0.001% to about 0.050%. In some embodiments, he compositions described herein have a concentration of ophthalmic drug between about 0.005% to about 0.050%, between about 0.010% to about 0.050%, between about 0.015% to about 0.050%, between about 0.020% to about 0.050%, between about 0.025% to about 0.050%, between about 0.030% to about 0.050%, between about 0.035% to about 0.050%, between about 0.040% to about 0.050%, or between about 0.045% to about 0.050% of the ophthalmic drug, by weight of the composition. The ophthalmic drug is a muscarinic antagonist. The muscarinic antagonist is atropine or atropine sulfate In some embodiments, the muscarinic antagonist is atropine. In some embodiments, the muscarinic antagonist is atropine sulfate. As described herein, the ophthalmic drug includes optically pure stereoisomers, optically enriched stereoisomers, and a racemic mixture of stereoisomers. For example, some ophthalmic compositions disclosed herein includes atropine or atropine sulfate in which the atropine is a racemic mixture of D- and L-isomers; and some ophthalmic compositions disclosed herein includes atropine or atropine sulfate in which the atropine is an optically enriched in favor of the more ophthalmically active L-isomer.

In some embodiments, the compositions described herein have a concentration of ophthalmic drug between about 0.001% to about 0.045%, between about 0.005% to about 0.045%, between about 0.010% to about 0.045%, between about 0.015% to about 0.045%, between about 0.020% to about 0.045%, between about 0.025% to about 0.045%, between about 0.030% to about 0.045%, between about 0.035% to about 0.045%, or between about 0.040% to about 0.045% of the ophthalmic drug by weight of the composition. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

In some embodiments, the compositions described herein have a concentration of ophthalmic drug between about 0.001% to about 0.040%, between about 0.005% to about 0.040%, between about 0.010% to about 0.040%, between about 0.015% to about 0.040%, between about 0.020% to about 0.040%, between about 0.025% to about 0.040%, between about 0.030% to about 0.040%, between about 0.035% to about 0.040% of the active ingredient. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

In some embodiments, the compositions described herein have a concentration of ophthalmic drug between about 0.001% to about 0.035%, between about 0.005% to about 0.035%, between about 0.010% to about 0.035%, between about 0.015% to about 0.035%, between about 0.020% to about 0.035%, between about 0.025% to about 0.035%, or between about 0.030% to about 0.035% of the ophthalmic drug by weight of the composition. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

In some embodiments, the compositions described herein have a concentration of ophthalmic drug between about 0.001% to about 0.030%, between about 0.005% to about 0.030%, between about 0.010% to about 0.030%, between about 0.015% to about 0.030%, between about 0.020% to about 0.030%, or between about 0.025% to about 0.030% of the active ingredient by weight of the composition. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

In some embodiments, the compositions described herein have a concentration of ophthalmic drug between about 0.001% to about 0.025%, between about 0.005% to about 0.025%, between about 0.010% to about 0.025%, between about 0.015% to about 0.025%, or between about 0.020% to about 0.025% of the ophthalmic drug by weight of the composition. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

In some embodiments, the compositions described herein have a concentration of ophthalmic drug between about 0.001% to about 0.020%, between about 0.005% to about 0.020%, between about 0.010% to about 0.020%, or between about 0.015% to about 0.020% of the active ingredient by weight of the composition. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

In some embodiments, the compositions described herein have a concentration of ophthalmic drug between about 0.001% to about 0.015%, between about 0.005% to about 0.015%, or between about 0.010% to about 0.015% of the ophthalmic drug by weight of the composition. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

In some embodiments, the compositions described herein have a concentration of ophthalmic drug between about 0.001% to about 0.010%, between about 0.005% to about 0.010%, or between about 0.008% to about 0.010% of the ophthalmic drug by weight of the composition. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

In some embodiments, the compositions described herein have a concentration of ophthalmic drug about 0.001%, 0.005%, 0.010%, 0.015%, 0.020%, 0.025%, 0.030%, 0.035%, 0.040%, 0.045%, or 0.050% of the ophthalmic drug by weight of the composition. The ophthalmic drug is a muscarinic antagonist. The muscarinic antagonist is atropine or atropine sulfate. In some embodiments, the muscarinic antagonist is atropine. In some embodiments, the muscarinic antagonist is atropine sulfate.

Without wishing to be bound by any particular theory, it is contemplated herein that the low concentration of the ophthalmic drug (i.e., atropine or atropine sulfate) in the disclosed ophthalmic composition provides sufficient and consistent therapeutic benefits to an individual in need thereof, while reducing or avoiding the ocular side effects including glare from pupillary dilation and blurred vision due to loss of accommodation that are associated with ophthalmic formulations containing higher concentrations of said ophthalmic drug.

### Ophthalmic Agents

Also described herein, are pharmaceutical compositions comprising one or more ophthalmic agents as claimed formulated in the presence of deuterated water. As used herein, deuterated water refers to D₂O, DHO, heavy water, and/or deuterium oxide. In some instances, the one or more ophthalmic agents are not muscarinic antagonist described above. In some cases, the ophthalmic compositions are formulated as an aqueous solution, gel, or as an ointment.

In some instances, the ophthalmic agents used in the ophthalmic compositions are susceptible to degradation through hydrolysis. In some instances, the ophthalmic agents used in the ophthalmic compositions are susceptible to degradation through base-catalyzed hydrolysis.

In some instances, ophthalmic agents include anti-angiogenic ophthalmic agents, mydriatics, antimydriatic agents, ophthalmic anesthetics, ophthalmic anti-infectives, ophthalmic anti-inflammatory agents, ophthalmic antihistamines and decongestants, ophthalmic diagnostic agents, ophthalmic glaucoma agents, ophthalmic lubricants and irrigation agents, ophthalmic steroids, ophthalmic steroids with anti-infectives, or ophthalmic surgical agents.

In some instances, an ophthalmic composition formulated in the presence of deuterated water include anti-angiogenic ophthalmic agents, mydriatics, antimydriatic agents, ophthalmic anesthetics, ophthalmic anti-infectives, ophthalmic anti-inflammatory agents, ophthalmic antihistamines and decongestants, ophthalmic diagnostic agents, ophthalmic glaucoma agents, ophthalmic lubricants and irrigation agents, ophthalmic steroids, ophthalmic steroids with anti-infectives, ophthalmic surgical agents, or combinations thereof.

Anti-angiogenic ophthalmic agents are vascular endothelial growth factor (VEGF) antagonists that prevent generation of new blood vessels by a process termed neovascularization. In some instances, anti-angiogenic ophthalmic agents are used to inhibit neovascularization in age related macular degeneration. In some instances, anti-angiogenic ophthalmic agents are used to treat diabetic macular edema, diabetic retinopathy, or macular edema. In some instances, macular edema is a swelling or thickening of the eye's macula, or the region of the eye responsible for central vision. In some instances, diabetic retinopathy refers to damages to the blood vessels in the retina. Examplary anti-angiogenic ophthalmic agents include, but are not limited to, aflibercept (also known as VEGF Trap) (e.g., Eylea), ranibizumab (e.g., Lucentis), or pegaptanib (e.g., Macugen).

In some instances, an ophthalmic composition formulated in the presence of deuterated water includes anti-angiogenic ophthalmic agents such as for example aflibercept (also known as VEGF Trap), ranibizumab, or pegaptanib. In some instances, an ophthalmic composition formulated in the presence of deuterated water includes aflibercept (also known as VEGF Trap), ranibizumab, pegaptanib, or combinations thereof.

Mydriatic agents are agents that dilate the pupil of the eye. In some instances, mydriatics are used to treat eye dryness, redness, or itching, uveitis, organophosphate poisoning, or inflammatory eye conditions such as iritis and cyclitis. Examplary mydriatic agents include, but are not limited to, cyclopentolate (e.g., Cyclogyl, Ak-Pentolate, Cylate, Ocu-Pentolate, or Pentolair), phenylephrine (e.g., AK-Dilate, AK-Nefrin, Altafrin, Isopto Frin, Mydfrin, Neo-synephrine Ophthalmic, Neofrin, Ocu-Phrin, Prefrin, or Refresh Redness Relief), homatropine (e.g., Homatropaire, Isopto Homatropine), scopolamine (e.g., Isopto Hyoscine), cyclopentolate/phenylephrine (e.g., Cyclomydril), phenylephrine/scopolamine (e.g., Murocoll 2), tropicamide (e.g., Mydral, Ocu-Tropic, or Tropicacyl), ketorolac/phenylephrine (e.g., Omidria), or hydroxyamphetamine/tropicamide (e.g., Paremyd).

In some instances, an ophthalmic composition formulated in the presence of deuterated water includes mydriatic agents such as for example cyclopentolate, phenylephrine, homatropine, scopolamine, cyclopentolate/phenylephrine, phenylephrine/scopolamine, tropicamide, ketorolac/phenylephrine, or hydroxyamphetamine/tropicamide. In some instances, an ophthalmic composition formulated in the presence of deuterated water includes cyclopentolate, phenylephrine, homatropine, scopolamine, cyclopentolate/phenylephrine, phenylephrine/scopolamine, tropicamide, ketorolac/phenylephrine, hydroxyamphetamine/tropicamide, or combinations thereof. In some instances, an ophthalmic composition formulated in the presence of deuterated water does not include atropine, atropine sulfate, noratropine, atropine-N-oxide, tropine, tropic acid, or atropine methonitrate. In some instances, an ophthalmic composition formulated in the presence of deuterated water does not include atropine. In some instances, an ophthalmic composition formulated in the presence of deuterated water does not include atropine sulfate.

Antimydriatic agents are agents that decrease the size of the pupil. Examplary antimydriatic agents include, but are not limited to, cysteamine (e.g., Cystaran), ocriplasmin (e.g., Jetrea), mitomycin (e.g., Mitosol), or dapiprazole (e.g., Rev-Eyes).

In some instances, an ophthalmic composition formulated in the presence of deuterated water includes antimydriatic agents such as for example cysteamine, ocriplasmin, mitomycin, or dapiprazole. In some instances, an ophthalmic composition formulated in the presence of deuterated water includes cysteamine, ocriplasmin, mitomycin, dapiprazole, or combinations thereof.

Ophthalmic anesthetics are local anesthetics that block pain signals at the nerve endings in the eyes. Examplary ophthalmic anesthetics include, but are not limited to, lidocaine (e.g., Akten), proparacaine (e.g., Alcaine, Ocu-Caine, Ophthetic, or Parcaine), tetracaine (e.g., Altacaine, Opticaine, or TetraVisc), or benoxinate (or oxybuprocaine) (e.g., Novesine, Novesin).

In some instances, an ophthalmic composition formulated in the presence of deuterated water includes ophthalmic anesthetics such as for example lidocaine, proparacaine, tetracaine, or benoxinate. In some instances, an ophthalmic composition formulated in the presence of deuterated water includes lidocaine, proparacaine, tetracaine, benoxinate, or combinations thereof.

Ophthalmic anti-infectives are ophthalmic formulations that comprise antibiotics and/or antiviral agents. In some instances, ophthalmic anti-infectives are used to treat blepharitis, blepharoconjunctivitis, CMV retinitis, conjunctivitis, corneal ulcer, eye dryness or redness, Herpes Simplex dendritic keratitis, Herpetic keratitis, hordeolum, keratitis, keratoconjunctivitis, neonatol conjunctivitis, or trachoma, or are used during surgery. Examplary ophthalmic anti-infectives include, but are not limited to, azithromycin (e.g., Azasite), bacitracin (e.g., AK-Tracin, Ocu-Tracin), besifloxacin (e.g., Besivance), boric acid (e.g., Collyrium Fresh), chloramphenicol (e.g., AK-Chlor, Chloromycetin ophthalmic, Chloroptic, Ocu-Chlor), ciprofloxacin (e.g., Ciloxan), erythromycin (e.g., Eyemycin, Ilotycin, Roymicin), ganciclovir (e.g., Vitrasert, Zirgan), gatifloxacin (e.g., Zymar, Zymaxid), gentamicin (e.g., Garamycin ophthalmic, Genoptic, Gentacidin, Gentak, Gentasol, Ocu-Mycin), idoxuridine (e.g., Herplex), levofloxacin (e.g., Iquix, Quixin), moxifloxacin (e.g., Vigamox, Moxeza), natamycin (e.g., Natacyn), norfloxacin (e.g., Chibroxin), ofloxacin (e.g., Ocuflox), bacitracin/polymyxin b (e.g., Polysporin ophthalmic, AK-Poly-Bac, Polycin-B, Polytracin ophthalmic), tobramycin (e.g., Tobrex, AK-Tob, Tomycine), polymyxin b/trimethoprim (e.g., Polytrim), povidone iodine (e.g., Betadine ophthalmic solution), trifluridine (e.g., Viroptic), gramicidin/neomycin/polymyxin b (e.g., AK-Spore, AK-Spore ointment, Neocidin ophthalmic solution), sulfacetamide sodium (e.g., AK-Sulf, Bleph-10, Cetamide, Isopto Cetamide), sulfisoxazole (e.g., Gantrisin ophthalmic), bacitracin/neomycin/polymyxin b (e.g., Neocidin, Neocin, Ocu-Spore-B, Ocutricin), oxytetracycline/polymyxin b (e.g., Terak, Tetramycin with Polymyxin B sulfate), phenylephrine/sulfacetamide sodium (e.g., Vasosulf), or vidarabine (e.g., Vira-A).

In some instances, an ophthalmic composition formulated in the presence of deuterated water includes ophthalmic anti-infectives such as for example azithromycin, bacitracin, besifloxacin, boric acid, chloramphenicol, ciprofloxacin, erythromycin, ganciclovir, gatifloxacin, gentamicin, idoxuridine, levofloxacin, moxifloxacin, natamycin, norfloxacin, ofloxacin, bacitracin/polymyxin b, tobramycin, polymyxin b/trimethoprim, povidone iodine, trifluridine, gramicidin/neomycin/polymyxin b, sulfacetamide sodium, sulfisoxazole, bacitracin/neomycin/polymyxin b, oxytetracycline/polymyxin b, phenylephrine/sulfacetamide sodium, or vidarabine. In some instances, an ophthalmic composition formulated in the presence of deuterated water includes azithromycin, bacitracin, besifloxacin, boric acid, chloramphenicol, ciprofloxacin, erythromycin, ganciclovir, gatifloxacin, gentamicin, idoxuridine, levofloxacin, moxifloxacin, natamycin, norfloxacin, ofloxacin, bacitracin/polymyxin b, tobramycin, polymyxin b/trimethoprim, povidone iodine, trifluridine, gramicidin/neomycin/polymyxin b, sulfacetamide sodium, sulfisoxazole, bacitracin/neomycin/polymyxin b, oxytetracycline/polymyxin b, phenylephrine/sulfacetamide sodium, vidarabine, or combinations thereof.

Ophthalmic anti-inflammatory agents are agents that reduce pain and/or inflammation of the eye. In some instances, ophthalmic anti-inflammatory agents are used to treat conjunctivitis, corneal ulcer, keratoconjunctivitis, keratoconjunctivitis sicca, postoperative increased intraocular pressure, postoperative ocular inflammation, or seasonal allergic conjunctivitis. In some instances, ophthalmic anti-inflammatory agents are used to inhibit intraoperative miosis. In some instances, ophthalmic anti-inflammatory agents are used during corneal refractive surgery. Examplary ophthalmic anti-inflammatory agents include, but are not limited to, bromfenac (e.g., Bromday, Xibrom), nepafenac (e.g., Nevanac), ketorolac (e.g., Acular, Acular LS, Acular PF, Acuvail), cyclosporine (e.g., Restasis), flurbiprofen (e.g., Ocufen), suprofen (e.g., Profenal), or diclofenac (e.g., Voltaren ophthalmic).

In some instances, an ophthalmic composition formulated in the presence of deuterated water includes ophthalmic anti-inflammatory agents such as for example bromfenac, nepafenac, ketorolac, cyclosporine, flurbiprofen, suprofen, or diclofenac. In some instances, an ophthalmic composition formulated in the presence of deuterated water includes bromfenac, nepafenac, ketorolac, cyclosporine, flurbiprofen, suprofen, diclofenac, or combinations thereof.

Ophthalmic antihistamines are antihistamines that block the histamine receptors that cause for example runny eyes, redness, itching, and the like. Ophthalmic decongestants are sympathomimetic agents that relieve redness of the eye. Examplary ophthalmic antihistamines and decongestants include, but are not limited to, alcaftadine (e.g., Lastacaft), azelastine (e.g., Optivar), bepotastine (e.g., Bepreve), cromolyn (e.g., Opticrom, Crolom), emedastine (e.g., Emadine), epinastine (e.g., Elestat), ketotifen (e.g., Alaway, Zaditor, Claritin Eye, Zyrtec Itchy Eye Drops), levocabastine (e.g., Livostin), lodoxamide (e.g., Alomide), nedocromil (e.g., Alocril), naphazoline (e.g., AK-Con, Albalon, All Clear, Allerest eye drops, Allersol, Clear Eyes, Ocu-Zoline, VasoClear, Vasocon), naphazoline/pheniramine (e.g., Visine-A, Opcon-A, Eye Allergy Relief), naphazoline/zinc sulfate (e.g., Clear Eyes ACR, VasoClear A), olopatadine (e.g., Patanol, Pataday, Pazeo), oxymetazoline (e.g., OcuClear), pemirolast (e.g., Alamast), phenylephrine (e.g., AK-Dilate, AK-Nefrin, Altafrin, Isopto Frin, Mydfrin, Neofrin, Ocu-Phrin, Prefrin, Refresh redness Relief), phenylephrine/zinc sulfate (e.g., Zincfrin), tetrahydrozoline (e.g., Visine original, Altazine, Geneyes, Opti-Clear, Optigene 3), or tetrahydrozoline/zinc sulfate (e.g., Visine totality multi-symptom relief).

In some instances, an ophthalmic composition formulated in the presence of deuterated water includes ophthalmic antihistamines and decongestants such as for example alcaftadine, azelastine, bepotastine, cromolyn, emedastine, epinastine, ketotifen, levocabastine, lodoxamide, nedocromil, naphazoline, naphazoline/pheniramine, naphazoline/zinc sulfate, olopatadine, oxymetazoline, pemirolast, phenylephrine, phenylephrine/zinc sulfate, tetrahydrozoline, or tetrahydrozoline/zinc sulfate. In some instances, an ophthalmic composition formulated in the presence of deuterated water includes alcaftadine, azelastine, bepotastine, cromolyn, emedastine, epinastine, ketotifen, levocabastine, lodoxamide, nedocromil, naphazoline, naphazoline/pheniramine, naphazoline/zinc sulfate, olopatadine, oxymetazoline, pemirolast, phenylephrine, phenylephrine/zinc sulfate, tetrahydrozoline, tetrahydrozoline/zinc sulfate, or combinations thereof.

Ophthalmic diagnostic agents are fluorescent molecules used for diagnostic fluorescein angiography or angioscopy of the retina and iris vasculature. Examplary ophthalmic diagnostic agents include, but are not limited to, fluorescein (e.g., AK-Fluor, BioGlo, Ful-Glo), fluorescein/proparacaine (e.g., Flucaine, Fluoracaine), benoxinate/fluorescein (e.g., Flurox), indocyanine green (e.g., IC-Green), or trypan blue (e.g., MembraneBlue, VisinBlue).

In some instances, an ophthalmic composition formulated in the presence of deuterated water includes ophthalmic diagnostic agents such as for example fluorescein, fluorescein/proparacaine, benoxinate/fluorescein, indocyanine green, or trypan blue. In some instances, an ophthalmic composition formulated in the presence of deuterated water includes fluorescein, fluorescein/proparacaine, benoxinate/fluorescein, indocyanine green, trypan blue, or combinations thereof.

Ophthalmic glaucoma agents are agents that reduce eye pressure in glaucoma. In some instances, ophthalmic glaucoma agents are also used to treat intraocular hypertension, postoperative increased intraocular pressure, or production of miosis. Examplary ophthalmic glaucoma agents include, but are not limited to, acetylcholine (e.g., Miochol-E), apraclonidine (e.g., Iopidine), betaxolol (e.g., Betoptic, Betoptic S), bimatoprost (e.g., Lumigan),brimonidine (e.g., Alphagan, Alphagan P), brinzolamide (e.g., Azopt), brimonidine/brinzolamide (e.g., Simbrinza), carbachol (e.g., Carbastat, Carboptic, Isopto Carbachol, Miostat), carteolol (e.g., Ocupress), demecarium bromide (e.g., Humorsol Ocumeter), dipivefrin (e.g., Propine), dorzolamide (e.g., Trusopt), dorzolamide/timolol (e.g., Cosopt, Cosopt PF, Combigan), echothiophate iodide (e.g., phospholine iodide), epinephrine (e.g., Epifrin, Epinal, Eppy/N, Glaucon), epinephrine/pilocarpine (e.g., E-Pilo-1, Epilo-2, P1E1, P2E1, P3E1, P4E1, P6E1), latanoprost (e.g., Xalatan), levobunolol (e.g., AK-Beta, Betagan), levobetaxolol (e.g., Betaxon), metipranolol (e.g., OptiPranolol), physostigmine (e.g., Eserine sulfate ophthalmic), pilocarpine (e.g., Isopto Carpine, Ocu-Carpine, Pilopine HS, Pilostat), tafluprost (e.g., Zioptan), timolol (e.g., Betimol, Timoptic Ocudose, Istalol, Timoptic, Timoptic-XE), travoprost (e.g., Travatan, Travatan Z, Izba), or unoprostone (e.g., Rescula).

In some instances, an ophthalmic composition formulated in the presence of deuterated water includes ophthalmic glaucoma agents such as for example acetylcholine, apraclonidine, betaxolol, bimatoprost, brimonidine, brinzolamide, brimonidine/brinzolamide, carbachol, carteolol, demecarium bromide, dipivefrin, dorzolamide, dorzolamide/timolol, echothiophate iodide, epinephrine, epinephrine/pilocarpine, latanoprost, levobunolol, levobetaxolol, metipranolol, physostigmine, pilocarpine, tafluprost, timolol, travoprost, or unoprostone. In some instances, an ophthalmic composition formulated in the presence of deuterated water includes acetylcholine, apraclonidine, betaxolol, bimatoprost, brimonidine, brinzolamide, brimonidine/brinzolamide, carbachol, carteolol, demecarium bromide, dipivefrin, dorzolamide, dorzolamide/timolol, echothiophate iodide, epinephrine, epinephrine/pilocarpine, latanoprost, levobunolol, levobetaxolol, metipranolol, physostigmine, pilocarpine, tafluprost, timolol, travoprost, unoprostone, or combinations thereof.

In some instances, ophthalmic lubricants and irrigation agents are used to treat dry and/or irritated eyes. Examplary ophthalmic lubricants and irrigation agents include, but are not limited to, artificial tear from Hypotears, System Balance, FreshKote, GenTeal, TheraTears, Lacrisert, Tears Again, Lacri-Lube S.O.P, Systane, Oasis Tears, Artificial Tears, Celluvisc, Clear Eyes CLR, Comfort Tears, Dry Eye Relief, Isopto Tears, Liquitears, Lubricant Eye drops, Lubrifresh PM, Moisture Drops, Murocel, Opti-Free Rewetting Drops, Optive, Puralube Tears, Refresh, Soothe, Sterilube, Tears Naturale, Tears Renew, Ultra Fresh, or Visine Tears. In some instances, artificial tear preparations include carboxymethyl cellulose, polyvinyl alcohol, hydroxypropyl methylcellulose, hydroxypropyl cellulose, and hyaluronic acid.

In some instances, an ophthalmic composition formulated in the presence of deuterated water includes ophthalmic lubricants and irrigation agents such as for example artificial tear. In some instances, an ophthalmic composition formulated in the presence of deuterated water includes artificial tear.

In some instances, ophthalmic steroids are used to treat conjunctivitis, cyclitis, diabetic macular edema, eye dryness/redness/itches, eyelash hypotrichosis, iritis, keratitis, macular edema, postoperative ocular inflammation, rosacea, seasonal allergic conjunctivitis, steroid responsive inflammatory conditions, temporal arteritis, uveitis, or vitrectomy. Examplary ophthalmic steroids include, but are not limited to, dexamethasone (e.g., Ozurdex, AK-Dex, Decadron Ocumeter, Dexasol, Maxidex, Ocu-Dex), difluprednate (e.g., Durezol), fluocinolone (e.g., Retisert, Iluvien), fluorometholone (e.g., FML Forte Liquifilm, Flarex, Fluor-Op, FML, FML S.O.P.), loteprednol (e.g., Alrex, Lotemax), medrysone (e.g., HMS), prednisolone (e.g., AK-Pred, Econopred, Econopred Plus, Inflamase Forte, Inflamase Mild, Omnipred, Pred Forte, Prednisol), rimexolone (e.g., Vexol), or triamcinolone (e.g., Triesence, Trivaris).

In some instances, an ophthalmic composition formulated in the presence of deuterated water includes ophthalmic steroids such as for example dexamethasone, difluprednate, fluocinolone, fluorometholone, loteprednol, medrysone, prednisolone, rimexolone, or triamcinolone. In some instances, an ophthalmic composition formulated in the presence of deuterated water includes dexamethasone, difluprednate, fluocinolone, fluorometholone, loteprednol, medrysone, prednisolone, rimexolone, triamcinolone, or combinations thereof.

Examplary ophthalmic steroids with anti-infectives include, but are not limited to, fluorometholone/sulfacetamide sodium (e.g., FML-S Liquifilm), dexamethasone/neomycin (e.g., Neo-Decadron, AK-Neo-Dex, Neo-Decadron Ocumeter, Neo-Dex, Neo-Dexair), dexamethasone/tobramycin (e.g., TobraDex, Tobradex ST), dexamethasone/neomycin/polymyxin b (e.g., Neo-Poly-Dex, Maxitrol, AK-Trol, Dexacidin, Dexacine, Dexasporin, Methadex, Ocu-Trol), loteprednol/tobramycin (e.g., Zylet), prednisolone/sulfacetamide sodium (e.g., Blephamide, Blephamide S.O.P., AK-Cide, Cetapred, Isopto Cetapred, Metimyd, Ocu-Lone C, Vasocidin), bacitracin/hydrocortisone/neomycin/polymyxin b (e.g., Cortisporin Ophthalmic ointment, Cortomycin eye ointment, Neo-Poly-Bac, Neotricin HC, Triple Antibiotic HC ophthalmic ointment), hydrocortisone/neomycin/polymyxin b (e.g., Cortisporin ophthalmic suspension, Cortomycin suspension), chloramphenicol/hydrocortisone/polymyxin b (e.g., Ophthocort), neomycin/polymyxin b/prednisolone (e.g., Poly Pred), or gentamicin/prednisolone (e.g., Pred-G, Pred-G S.O.P.).

In some instances, an ophthalmic composition formulated in the presence of deuterated water includes ophthalmic steroids with anti-infectives such as for example fluorometholone/sulfacetamide sodium, dexamethasone/neomycin, dexamethasone/tobramycin, dexamethasone/neomycin/polymyxin b, loteprednol/tobramycin, prednisolone/sulfacetamide sodium, bacitracin/hydrocortisone/neomycin/polymyxin b, hydrocortisone/neomycin/polymyxin b, chloramphenicol/hydrocortisone/polymyxin b, neomycin/polymyxin b/prednisolone, or gentamicin/prednisolone. In some instances, an ophthalmic composition formulated in the presence of deuterated water includes fluorometholone/sulfacetamide sodium, dexamethasone/neomycin, dexamethasone/tobramycin, dexamethasone/neomycin/polymyxin b, loteprednol/tobramycin, prednisolone/sulfacetamide sodium, bacitracin/hydrocortisone/neomycin/polymyxin b, hydrocortisone/neomycin/polymyxin b, chloramphenicol/hydrocortisone/polymyxin b, neomycin/polymyxin b/prednisolone, gentamicin/prednisolone, or combinations thereof.

Examplary ophthalmic surgical agents include, but are not limited to, ketorolac/phenylephrine (e.g., Omidria).

In some instances, an ophthalmic composition formulated in the presence of deuterated water includes ophthalmic surgical agents such as for example ketorolac/phenylephrine. In some instances, an ophthalmic composition formulated in the presence of deuterated water includes ketorolac/phenylephrine.

In some instances, an ophthalmic composition formulated in the presence of deuterated water includes diphenhydramine, dimenhydrinate, dicyclomine, flavoxate, oxybutynin, tiotropium, hyoscine, scopolamine (L-hyoscine), hydroxyzine, ipratropium, pirenzapine, solifenacin, darifenacin, benzatropine, mebeverine, procyclidine, aclidinium bromide, trihexyphenidyl/benzhexol, tolterodine, or a combination thereof.

In some instances, an ophthalmic composition formulated in the presence of deuterated water includes aflibercept (also known as VEGF Trap), ranibizumab, pegaptanib, cyclopentolate, phenylephrine, homatropine, scopolamine, cyclopentolate/phenylephrine, phenylephrine/scopolamine, tropicamide, ketorolac/phenylephrine, hydroxyamphetamine/tropicamide, cysteamine, ocriplasmin, mitomycin, dapiprazole, lidocaine, proparacaine, tetracaine, benoxinate, azithromycin, bacitracin, besifloxacin, boric acid, chloramphenicol, ciprofloxacin, erythromycin, ganciclovir, gatifloxacin, gentamicin, idoxuridine, levofloxacin, moxifloxacin, natamycin, norfloxacin, ofloxacin, bacitracin/polymyxin b, tobramycin, polymyxin b/trimethoprim, povidone iodine, trifluridine, gramicidin/neomycin/polymyxin b, sulfacetamide sodium, sulfisoxazole, bacitracin/neomycin/polymyxin b, oxytetracycline/polymyxin b, phenylephrine/sulfacetamide sodium, vidarabine, bromfenac, nepafenac, ketorolac, cyclosporine, flurbiprofen, suprofen, diclofenac, alcaftadine, azelastine, bepotastine, cromolyn, emedastine, epinastine, ketotifen, levocabastine, lodoxamide, nedocromil, naphazoline, naphazoline/pheniramine, naphazoline/zinc sulfate, olopatadine, oxymetazoline, pemirolast, phenylephrine, phenylephrine/zinc sulfate, tetrahydrozoline, tetrahydrozoline/zinc sulfate, fluorescein, fluorescein/proparacaine, benoxinate/fluorescein, indocyanine green, trypan blue, acetylcholine, apraclonidine, betaxolol, bimatoprost, brimonidine, brinzolamide, brimonidine/brinzolamide, carbachol, carteolol, demecarium bromide, dipivefrin, dorzolamide, dorzolamide/timolol, echothiophate iodide, epinephrine, epinephrine/pilocarpine, latanoprost, levobunolol, levobetaxolol, metipranolol, physostigmine, pilocarpine, tafluprost, timolol, travoprost, unoprostone, artificial tear, dexamethasone, difluprednate, fluocinolone, fluorometholone, loteprednol, medrysone, prednisolone, rimexolone, triamcinolone, fluorometholone/sulfacetamide sodium, dexamethasone/neomycin, dexamethasone/tobramycin, dexamethasone/neomycin/polymyxin b, loteprednol/tobramycin, prednisolone/sulfacetamide sodium, bacitracin/hydrocortisone/neomycin/polymyxin b, hydrocortisone/neomycin/polymyxin b, chloramphenicol/hydrocortisone/polymyxin b, neomycin/polymyxin b/prednisolone, gentamicin/prednisolone, ketorolac/phenylephrine, diphenhydramine, dimenhydrinate, dicyclomine, flavoxate, oxybutynin, tiotropium, hyoscine, scopolamine (L-hyoscine), hydroxyzine, ipratropium, pirenzapine, solifenacin, darifenacin, benzatropine, mebeverine, procyclidine, aclidinium bromide, trihexyphenidyl/benzhexol, tolterodine, or any combinations thereof.

### Ophthalmic Composition Comprising One or More Ophthalmic Agents

Also described herein is an ophthalmic composition comprising one or more ophthalmic agents for the treatment of an ophthalmic disorder or condition in which the ophthalmic composition is formulated with deuterated water. In some instances, the ophthalmic composition is stable at different temperatures, at different relative humidity, and with a potency of at least 80% relative to the ophthalmic agent. In additional instances, the ophthalmic composition has a lowered buffering capacity. In such instances, the lowered buffering capacity of the ophthalmic composition when administered into the eye allows the ophthalmic composition to reach physiological pH at a faster rate than compared to an equivalent ophthalmic formulation or solution formulated in H₂O.

In some instances, described herein is an ophthalmic composition that does not have a dose-to-dose variation. In some instances, described herein is an ophthalmic composition that is stable at different temperatures, at different relative humidity, and with a potency of at least 80% relative to the ophthalmic agent.

In other instances, described herein include formulating the ophthalmic composition as an ophthalmic gel or an ophthalmic ointment. For example, some ophthalmic gel or an ophthalmic ointment described herein allows desirable dose-to-dose uniformity, increased stability, reduced or limited systemic exposure, or combinations thereof.

### Ophthalmic Solution Composition or Formulation

Disclosed herein, in certain instances, is an ophthalmic composition comprising an ophthalmic agent formulated as an aqueous solution. In some instances, the ophthalmic composition comprises an ophthalmic agent and deuterated water. In some cases, the ophthalmic agent is not a muscarinic antagonist. As used herein, deuterated water refers to D₂O, DHO, heavy water, and/or deuterium oxide.

In some instances, the composition comprises at least about 80% of the ophthalmic agent for an extended period of time under storage condition. In some instances, the composition comprises at least about 81% of the ophthalmic agent for an extended period of time under storage condition. In some instances, the composition comprises at least about 82% of the ophthalmic agent for an extended period of time under storage condition. In some instances, the composition comprises at least about 83% of the ophthalmic agent for an extended period of time under storage condition. In some instances, the composition comprises at least about 84% of the ophthalmic agent for an extended period of time under storage condition. In some instances, the composition comprises at least about 85% of the ophthalmic agent for an extended period of time under storage condition. In some instances, the composition comprises at least about 86% of the ophthalmic agent for an extended period of time under storage condition. In some instances, the composition comprises at least about 87% of the ophthalmic agent for an extended period of time under storage condition. In some instances, the composition comprises at least about 88% of the ophthalmic agent for an extended period of time under storage condition. In some instances, the composition comprises at least about 89% of the ophthalmic agent for an extended period of time under storage condition. In some instances, the composition comprises at least about 90% of the ophthalmic agent for an extended period of time under storage condition. In some instances, the composition comprises at least about 91% of the ophthalmic agent for an extended period of time under storage condition. In some instances, the composition comprises at least about 92% of the ophthalmic agent for an extended period of time under storage condition. In some instances, the composition comprises at least about 93% of the ophthalmic agent for an extended period of time under storage condition. In some instances, the composition comprises at least about 94% of the ophthalmic agent for an extended period of time under storage condition. In some instances, the composition comprises at least about 95% of the ophthalmic agent for an extended period of time under storage condition. In some instances, the composition comprises at least about 96% of the ophthalmic agent for an extended period of time under storage condition. In some instances, the composition comprises at least about 97% of the ophthalmic agent for an extended period of time under storage condition. In some instances, the composition comprises at least about 98% of the ophthalmic agent for an extended period of time under storage condition. In some instances, the composition comprises at least about 99% of the ophthalmic agent for an extended period of time under storage condition. In some instances, the composition comprises at least about 99.5% of the ophthalmic agent for an extended period of time under storage condition. In some instances, the composition comprises at least about 99.9% of the ophthalmic agent for an extended period of time under storage condition.

In some instances, the composition has a potency of at least about 80% after extended period of time under storage condition. In some instances, the composition has a potency of at least about 81% after extended period of time under storage condition. In some instances, the composition has a potency of at least about 82% after extended period of time under storage condition. In some instances, the composition has a potency of at least about 83% after extended period of time under storage condition. In some instances, the composition has a potency of at least about 84% after extended period of time under storage condition. In some instances, the composition has a potency of at least about 85% after extended period of time under storage condition. In some instances, the composition has a potency of at least about 86% after extended period of time under storage condition. In some instances, the composition has a potency of at least about 87% after extended period of time under storage condition. In some instances, the composition has a potency of at least about 88% after extended period of time under storage condition. In some instances, the composition has a potency of at least about 89% after extended period of time under storage condition. In some instances, the composition has a potency of at least 90% after extended period of time under storage condition. In some instances, the composition has a potency of at least 91% after extended period of time under storage condition. In some instances, the composition has a potency of at least 92% after extended period of time under storage condition. In some instances, the composition has a potency of at least 93% after extended period of time under storage condition. In some instances, the composition has a potency of at least 94% after extended period of time under storage condition. In some instances, the composition has a potency of at least 95% after extended period of time under storage condition. In some instances, the composition has a potency of at least 96% after extended period of time under storage condition. In some instances, the composition has a potency of at least 97% after extended period of time under storage condition. In some instances, the composition has a potency of at least 98% after extended period of time under storage condition. In some instances, the composition has a potency of at least 99% after extended period of time under storage condition.

In some instances, the extended period of time is at least 1 week. In some instances, the extended period of time is at least 2 weeks. In some instances, the extended period of time is at least 3 weeks. In some instances, the extended period of time is at least 1 month. In some instances, the extended period of time is at least 2 months. In some instances, the extended period of time is at least 3 months. In some instances, the extended period of time is at least 4 months. In some instances, the extended period of time is at least 5 months. In some instances, the extended period of time is at least 6 months. In some instances, the extended period of time is at least 7 months. In some instances, the extended period of time is at least 8 months. In some instances, the extended period of time is at least 9 months. In some instances, the extended period of time is at least 10 months. In some instances, the extended period of time is at least 11 months. In some instances, the extended period of time is at least 12 months (i.e. 1 year). In some instances, the extended period of time is at least 18 months (i.e. 1.5 years). In some instances, the extended period of time is at least 24 months (i.e. 2 years). In some instances, the extended period of time is at least 36 months (i.e. 3 years). In some instances, the extended period of time is at least 3 years. In some instances, the extended period of time is at least 5 years, 6 years, 7 years, 8 years, 9 years, 10 years, 15 years, 30 years, or more.

In some instances, the temperature of the storage condition is between about 2°C and about 70°C. In some instances, the temperature of the storage condition is between about 2°C and about 65°C, about 8°C and about 65°C, about 10°C and about 65°C, about 25°C and about 65°C, about 30°C and about 60°C, about 35°C and about 55°C, or about 40°C and about 50°C. In some instances, the temperature of the storage condition is between about 2°C and about 10°C. In some instances, the temperature of the storage condition is between about 20°C and about 26°C. In some instances, the temperature of the storage condition is about 25°C. In some instances, the temperature of the storage condition is about 40°C. In some instances, the temperature of the storage condition is about 60°C.

In some instances, the relative humidity of the storage condition is between about 50% and about 80%, or between about 60% and about 75%. In some instances, the relative humidity of the storage condition is about 60%. In some instances, the relative humidity of the storage condition is about 75%.

In some instances, the composition comprises less than 60% of H₂O. In some instances, the composition comprises less than 55% of H₂O. In some instances, the composition comprises less than 50% of H₂O. In some instances, the composition comprises less than 45% of H₂O. In some instances, the composition comprises less than 40% of H₂O. In some instances, the composition comprises less than 35% of H₂O. In some instances, the composition comprises less than 30% of H₂O. In some instances, the composition comprises less than 25% of H₂O. In some instances, the composition comprises less than 20% of H₂O. In some instances, the composition comprises less than 15% of H₂O. In some instances, the composition comprises less than 10% of H₂O. In some instances, the composition comprises less than 9% of H₂O. In some instances, the composition comprises less than 8% of H₂O. In some instances, the composition comprises less than 7% of H₂O. In some instances, the composition comprises less than 6% of H₂O.

In some instances, the composition comprises from less than 5% of H₂O to less than 0.1% of H₂O. In some instances, the composition comprises less than 5% of H₂O. In some instances, the composition comprises less than 4.5% of H₂O. In some instances, the composition comprises less than 4% of H₂O. In some instances, the composition comprises less than 3.5% of H₂O. In some instances, the composition comprises less than 3% of H₂O. In some instances, the composition comprises less than 2.5% of H₂O. In some instances, the composition comprises less than 2% of H₂O. In some instances, the composition comprises less than 1.5% of H₂O. In some instances, the composition comprises less than 1% of H₂O. In some instances, the composition comprises less than 0.5% of H₂O. In some instances, the composition comprises less than 0.4% of H₂O. In some instances, the composition comprises less than 0.3% of H₂O. In some instances, the composition comprises less than 0.2% of H₂O. In some instances, the composition comprises less than 0.1% of H₂O. In some instances, the composition comprises 0% of H₂O.

According to the invention, the composition has a pD of between about 4.2 to about 7.9. In some instance, the composition has a pD of between about 4.5 and about 7.8, about 5 and about 7.5, or about 5.5 and about 7. In some instances, the composition has a pD of less than about 7.9. In some instances, the composition has a pD of less than about 7.8. In some instances, the composition has a pD of less than about 7.7. In some instances, the composition has a pD of less than about 7.6. In some instances, the composition has a pD of less than about 7.5. In some instances, the composition has a pD of less than about 7.4. In some instances, the composition has a pD of less than about 7.3. In some instances, the composition has a pD of less than about 7.2. In some instances, the composition has a pD of less than about 7.1. In some instances, the composition has a pD of less than about 7. In some instances, the composition has a pD of less than about 6.9. In some instances, the composition has a pD of less than about 6.8. In some instances, the composition has a pD of less than about 6.7. In some instances, the composition has a pD of less than about 6.6. In some instances, the composition has a pD of less than about 6.5. In some instances, the composition has a pD of less than about 6.4. In some instances, the composition has a pD of less than about 6.3. In some instances, the composition has a pD of less than about 6.2. In some instances, the composition has a pD of less than about 6.1. In some instances, the composition has a pD of less than about 6. In some instances, the composition has a pD of less than about 5.9. In some instances, the composition has a pD of less than about 5.8. In some instances, the composition has a pD of less than about 5.7. In some instances, the composition has a pD of less than about 5.6. In some instances, the composition has a pD of less than about 5.5. In some instances, the composition has a pD of less than about 5.4. In some instances, the composition has a pD of less than about 5.3. In some instances, the composition has a pD of less than about 5.2. In some instances, the composition has a pD of less than about 5.1. In some instances, the composition has a pD of less than about 5. In some instances, the composition has a pD of less than about 4.9. In some instances, the composition has a pD of less than about 4.8. In some instances, the composition has a pD of less than about 4.7. In some instances, the composition has a pD of less than about 4.6. In some instances, the composition has a pD of less than about 4.5. In some instances, the composition has a pD of less than about 4.4. In some instances, the composition has a pD of less than about 4.3.

In some instances, the composition comprising deuterated water has a lowered buffering capacity than an equivalent composition comprising H₂O. As described elsewhere herein, in some instances, the lowered buffering capacity allows the composition comprising deuterated water to normalize to physiological pH at a faster rate than a composition comprising H₂O. In some instances, the lowered buffering capacity allows the composition to induce less tear reflex than an equivalent composition comprising H₂O.

In some instances, the composition comprising deuterated water stabilizes the ophthalmic agent. In some instances, this is due to a lower concentration of the reactive species (e.g., -OD) in the D₂O aqueous system compared to the concentration of the reactive species (e.g., -OH) in an equivalent H₂O aqueous system. In some cases, base catalysis leads to the presence of degradant from the ophthalmic agent. In some cases, with a lower concentration of the reactive species that causes degradant formation, the ophthalmic solution is more stable in a D₂O aqueous system than compared to an equivalent H₂O aqueous system. In some instances, the ophthalmic composition formulated with deuterated water allows for a more stable ophthalmic composition relative to the ophthalmic composition formulated with H₂O.

In some instances, the composition comprises less than 20% of major degradant based on the concentration of the ophthalmic agent after extended period of time under storage condition. In some instances, the composition comprises less than 15% of major degradant based on the concentration of the ophthalmic agent after extended period of time under storage condition. In some instances, the composition comprises less than 10% of major degradant based on the concentration of the ophthalmic agent after extended period of time under storage condition. In some instances, the composition comprises less than 5% of major degradant based on the concentration of the ophthalmic agent after extended period of time under storage condition. In some instances, the composition comprises less than 2.5% of major degradant based on the concentration of the ophthalmic agent after extended period of time under storage condition. In some instances, the composition comprises less than 2.0% of major degradant based on the concentration of the ophthalmic agent after extended period of time under storage condition. In some instances, the composition comprises less than 1.5% of major degradant based on the concentration of the ophthalmic agent after extended period of time under storage condition. In some instances, the composition comprises less than 1.0% of major degradant based on the concentration of the ophthalmic agent after extended period of time under storage condition. In some instances, the composition comprises less than 0.5% of major degradant based on the concentration of the ophthalmic agent after extended period of time under storage condition. In some instances, the composition comprises less than 0.4% of major degradant based on the concentration of the ophthalmic agent after extended period of time under storage condition. In some instances, the composition comprises less than 0.3% of major degradant based on the concentration of the ophthalmic agent after extended period of time under storage condition. In some instances, the composition comprises less than 0.2% of major degradant based on the concentration of the ophthalmic agent after extended period of time under storage condition. In some instances, the composition comprises less than 0.1% of major degradant based on the concentration of the ophthalmic agent after extended period of time under storage condition.

In some instances, the composition does not extend singlet oxygen lifetime upon irradiation with UV. In some instances, one or more of the ophthalmic agents described herein does not extend singlet oxygen lifetime upon irradiation with UV. In some instances, one or more of the ophthalmic agents described herein is a radical scavenger, which quenches photogenerated singlet oxygen species within the composition. In some instances, one or more of the ophthalmic agents selected from: aflibercept, ranibizumab, pegaptanib, cyclopentolate, phenylephrine, homatropine, scopolamine, cyclopentolate/phenylephrine, phenylephrine/scopolamine, tropicamide, ketorolac/phenylephrine, hydroxyamphetamine/tropicamide, cysteamine, ocriplasmin, mitomycin, dapiprazole, lidocaine, proparacaine, tetracaine, benoxinate, azithromycin, bacitracin, besifloxacin, boric acid, chloramphenicol, ciprofloxacin, erythromycin, ganciclovir, gatifloxacin, gentamicin, idoxuridine, levofloxacin, moxifloxacin, natamycin, norfloxacin, ofloxacin, bacitracin/polymyxin b, tobramycin, polymyxin b/trimethoprim, povidone iodine, trifluridine, gramicidin/neomycin/polymyxin b, sulfacetamide sodium, sulfisoxazole, bacitracin/neomycin/polymyxin b, oxytetracycline/polymyxin b, phenylephrine/sulfacetamide sodium, vidarabine, bromfenac, nepafenac, ketorolac, cyclosporine, flurbiprofen, suprofen, diclofenac, alcaftadine, azelastine, bepotastine, cromolyn, emedastine, epinastine, ketotifen, levocabastine, lodoxamide, nedocromil, naphazoline, naphazoline/pheniramine, naphazoline/zinc sulfate, olopatadine, oxymetazoline, pemirolast, phenylephrine, phenylephrine/zinc sulfate, tetrahydrozoline, tetrahydrozoline/zinc sulfate, fluorescein, fluorescein/proparacaine, benoxinate/fluorescein, indocyanine green, trypan blue, acetylcholine, apraclonidine, betaxolol, bimatoprost,brimonidine, brinzolamide, brimonidine/brinzolamide, carbachol, carteolol, demecarium bromide, dipivefrin, dorzolamide, dorzolamide/timolol, echothiophate iodide, epinephrine, epinephrine/pilocarpine, latanoprost, levobunolol, levobetaxolol, metipranolol, physostigmine, pilocarpine, tafluprost, timolol, travoprost, unoprostone, artificial tear, dexamethasone, difluprednate, fluocinolone, fluorometholone, loteprednol, medrysone, prednisolone, rimexolone, triamcinolone, fluorometholone/sulfacetamide sodium, dexamethasone/neomycin, dexamethasone/tobramycin, dexamethasone/neomycin/polymyxin b, loteprednol/tobramycin, prednisolone/sulfacetamide sodium, bacitracin/hydrocortisone/neomycin/polymyxin b, hydrocortisone/neomycin/polymyxin b, chloramphenicol/hydrocortisone/polymyxin b, neomycin/polymyxin b/prednisolone, gentamicin/prednisolone, ketorolac/phenylephrine, diphenhydramine, dimenhydrinate, dicyclomine, flavoxate, oxybutynin, tiotropium, hyoscine, scopolamine (L-hyoscine), hydroxyzine, ipratropium, pirenzapine, solifenacin, darifenacin, benzatropine, mebeverine, procyclidine, aclidinium bromide, trihexyphenidyl/benzhexol, and tolterodine, does not extend singlet oxygen lifetime upon irradiation with UV or quenches photogenerated singlet oxygen species within the composition. In some cases, the ophthalmic agent is not an alpha-amino-carboxylic acid or an alpha-hydroxy-carboxylic acid. In some cases, the ophthalmic agent is not benactyzine hydrochloride. In some cases, the ophthalmic composition is not saturated with oxygen. In additional cases, the ophthalmic composition does not comprise a photosensitizer. According to the invention, the ophthalmic composition comprises atropine and atropine sulfate.

### Ophthalmic Agent Concentration

According to the invention, the ophthalmic composition comprises from about 0.001 wt% to about 0.05 wt% of atropine or atropine sulfate by weight of the composition. In some instances, the compositions described herein have a concentration of atropine or atropine sulfate between about 0.030% to about 0.050%, between about 0.035% to about 0.050%, between about 0.040% to about 0.050%, or between about 0.045% to about 0.050% of the ophthalmic agent, by weight of the composition.

As described herein, the ophthalmic agent includes optically pure stereoisomers, optically enriched stereoisomers, and a racemic mixture of stereoisomers. For example, some ophthalmic compositions disclosed herein include racemic mixture of D- and L-isomers; and some ophthalmic compositions disclosed herein include optically enriched in favor of an ophthalmically active L-isomer.

### Aqueous Solution Stability

In some embodiments, the composition described herein comprises a buffer. In some embodiments, a buffer is selected from borates, borate-polyol complexes, phosphate buffering agents, citrate buffering agents, acetate buffering agents, carbonate buffering agents, organic buffering agents, amino acid buffering agents, or combinations thereof. In some embodiments, the composition described herein comprises buffer comprising deuterated water. In some embodiments, a deuterated buffer is selected from borates, borate-polyol complexes, phosphate buffering agents, citrate buffering agents, acetate buffering agents, carbonate buffering agents, organic buffering agents, amino acid buffering agents, or combinations thereof, formulated in deuterated water.

In some instances, borates include boric acid, salts of boric acid, other pharmaceutically acceptable borates, and combinations thereof. In some cases, borates include boric acid, sodium borate, potassium borate, calcium borate, magnesium borate, manganese borate, and other such borate salts.

As used herein, the term polyol includes any compound having at least one hydroxyl group on each of two adjacent carbon atoms that are not in trans configuration relative to each other. In some embodiments, the polyols is linear or cyclic, substituted or unsubstituted, or mixtures thereof, so long as the resultant complex is water soluble and pharmaceutically acceptable. In some instances, examples of polyol include: sugars, sugar alcohols, sugar acids and uronic acids. In some cases, polyols include, but are not limited to: mannitol, glycerin, xylitol and sorbitol.

In some embodiments, phosphate buffering agents include phosphoric acid; alkali metal phosphates such as disodium hydrogen phosphate, sodium dihydrogen phosphate, trisodium phosphate, dipotassium hydrogen phosphate, potassium dihydrogen phosphate, and tripotassium phosphate; alkaline earth metal phosphates such as calcium phosphate, calcium hydrogen phosphate, calcium dihydrogen phosphate, monomagnesium phosphate, dimagnesium phosphate (magnesium hydrogen phosphate), and trimagnesium phosphate; ammonium phosphates such as diammonium hydrogen phosphate and ammonium dihydrogen phosphate; or a combination thereof. In some instances, the phosphate buffering agent is an anhydride. In some instances, the phosphate buffering agent is a hydrate.

In some embodiments, borate-polyol complexes include those described in U.S. Pat. No. 6,503,497. In some instances, the borate-polyol complexes comprise borates in an amount of from about 0.01 to about 2.0% w/v, and one or more polyols in an amount of from about 0.01% to about 5.0% w/v.

In some cases, citrate buffering agents include citric acid and sodium citrate.

In some instances, acetate buffering agents include acetic acid, potassium acetate, and sodium acetate.

In some instances, carbonate buffering agents include sodium bicarbonate and sodium carbonate.

In some cases, organic buffering agents include Good's Buffer, such as for example 2-(N-morpholino)ethanesulfonic acid (MES), N-(2-Acetamido)iminodiacetic acid, *N-*(Carbamoylmethyl)iminodiacetic acid (ADA), piperazine-N,N'-bis(2-ethanesulfonic acid (PIPES), N-(2-acetamido)-2-aminoethanesulfonic acid (ACES), β-Hydroxy-4-morpholinepropanesulfonic acid, 3-Morpholino-2-hydroxypropanesulfonic acid (MOPSO), cholamine chloride, 3-(N-morpholino)propansulfonic acid (MOPS), N,N-bis(2-hydroxyethyl)-2-aminoethanesulfonic acid (BES), 2-[(2-Hydroxy-1,1-bis(hydroxymethyl)ethyl)amino]ethanesulfonic acid (TES), 4-(2-hydroxyethyl)-1-piperazineethanesulfonic acid (HEPES), 3-(N,N-Bis[2-hydroxyethyl]amino)-2-hydroxypropanesulfonic acid (DIPSO), acetamidoglycine, 3-{[1,3-Dihydroxy-2-(hydroxymethyl)-2-propanyl]amino}-2-hydroxy-1-propanesulfonic acid (TAPSO), piperazine-1,4,-bis (2-hydroxypropanesulphonic acid) (POPSO), 4-(2-hydroxyethyl)piperazine-1-(2-hydroxypropanesulfonic acid) hydrate (HEPPSO), 3-[4-(2-hydroxyethyl)-1-piperazinyl]propanesulfonic acid (HEPPS), tricine, glycinamide, bicine or N-tris(hydroxymethyl)methyl-3-aminopropanesulfonic acid sodium (TAPS); glycine; and diethanolamine (DEA).

In some cases, amino acid buffering agents include taurine, aspartic acid and its salts (e.g., potassium salts, etc), E-aminocaproic acid, and the like.

In some instances, the composition described herein further comprises a tonicity adjusting agent. Tonicity adjusting agent is an agent introduced into a preparation such as an ophthalmic composition to reduce local irritation by preventing osmotic shock at the site of application. In some instances, buffer solution and/or a pD adjusting agent that broadly maintains the ophthalmic solution at a particular ion concentration and pD are considered as tonicity adjusting agents. In some cases, tonicity adjusting agents include various salts, such as halide salts of a monovalent cation. In some cases, tonicity adjusting agents include mannitol, sorbitol, dextrose, sucrose, urea, and glycerin. In some instances, suitable tonicity adjustors comprise sodium chloride, sodium nitrate, sodium sulfate, sodium bisulfate, potassium chloride, calcium chloride, magnesium chloride, zinc chloride, potassium acetate, sodium acetate, sodium bicarbonate, sodium carbonate, sodium thiosulfate, magnesium sulfate, disodium hydrogen phosphate, sodium dihydrogen phosphate, potassium dihydrogen phosphate, dextrose, mannitol, sorbitol, dextrose, sucrose, urea, propylene glycol, glycerin, or a combination thereof.

In some instances, the concentration of the tonicity adjusting agent in a composition described herein is between about 0.5% and about 2.0%. In some instances, the concentration of the tonicity adjusting agent in a composition described herein is between about 0.7% and about 1.8%, about 0.8% and about 1.5%, or about 1% and about 1.3%. In some instances, the concentration of the tonicity adjusting agent is about 0.6%, 0.7%, 0.8%, 0.9%, 1.0%, 1.1%, 1.2%, 1.3%, 1.4%, 1.5%, 1.6%, 1.7%, 1.8%, or 1.9%. In some cases, the percentage is a weight percentage.

In some cases, the composition described herein further comprises a pD adjusting agent. In some embodiments, the pD adjusting agent used is an acid or a base. In some embodiments, the base is oxides, hydroxides, carbonates, bicarbonates and the likes. In some instances, the oxides are metal oxides such as calcium oxide, magnesium oxide and the likes; hydroxides are of alkali metals and alkaline earth metals such as sodium hydroxide, potassium hydroxide, calcium hydroxide and the likes or their deuterated equivalents, and carbonates are sodium carbonate, sodium bicarbonates, potassium bicarbonates and the likes. In some instances, the acid is mineral acid and organic acids such as hydrochloric acid, nitric acid, phosphoric acid, acetic acid, citric acid, fumaric acid, malic acid tartaric acid and the likes or their deuterated equivalents. In some instances, the pD adjusting agent includes, but is not limited to, acetate, bicarbonate, ammonium chloride, citrate, phosphate, pharmaceutically acceptable salts thereof and combinations or mixtures thereof. In some embodiments, the pD adjusting agent comprises DCl and NaOD.

The composition has a pD of from about 4.2 to about 7.9. In some instances, the composition has a pD of between about 4.5 and about 7.8, about 5 and about 7.5, or about 5.5 and about 7. In some embodiments, the composition has a pD of less than about 7.5. In some embodiments, the composition has a pD of less than about 7.4. In some embodiments, the composition has a pD of less than about 7.3. In some embodiments, the composition has a pD of less than about 7.2. In some embodiments, the composition has a pD of less than about 7.1. In some embodiments, the composition has a pD of less than about 7. In some embodiments, the composition has a pD of less than about 6.9. In some embodiments, the composition has a pD of less than about 6.8. In some embodiments, the composition has a pD of less than about 6.7. In some embodiments, the composition has a pD of less than about 6.6. In some embodiments, the composition has a pD of less than about 6.5. In some embodiments, the composition has a pD of less than about 6.4. In some embodiments, the composition has a pD of less than about 6.3. In some embodiments, the composition has a pD of less than about 6.2. In some embodiments, the composition has a pD of less than about 6.1. In some embodiments, the composition has a pD of less than about 6. In some embodiments, the composition has a pD of less than about 5.9. In some embodiments, the composition has a pD of less than about 5.8. In some embodiments, the composition has a pD of less than about 5.7. In some embodiments, the composition has a pD of less than about 5.6. In some embodiments, the composition has a pD of less than about 5.5. In some embodiments, the composition has a pD of less than about 5.4. In some embodiments, the composition has a pD of less than about 5.3. In some embodiments, the composition has a pD of less than about 5.2. In some embodiments, the composition has a pD of less than about 5.1. In some embodiments, the composition has a pD of less than about 5. In some embodiments, the composition has a pD of less than about 4.9. In some embodiments, the composition has a pD of less than about 4.8. In some embodiments, the composition has a pD of less than about 4.7. In some embodiments, the composition has a pD of less than about 4.6. In some embodiments, the composition has a pD of less than about 4.5. In some embodiments, the composition has a pD of less than about 4.4. In some embodiments, the composition has a pD of less than about 4.3. In some embodiments, the pD is the pD of the composition after extended period of time under storage condition.

In some instances, the composition has an initial pD of between about 4.5 and about 7.8, about 5 and about 7.5, or about 5.5 and about 7. In some embodiments, the composition has an initial pD of about 7.5. In some embodiments, the composition has an initial pD of about 7.4. In some embodiments, the composition has an initial pD of about 7.3. In some embodiments, the composition has an initial pD of about 7.2. In some embodiments, the composition has an initial pD of about 7.1. In some embodiments, the composition has an initial pD of about 7. In some embodiments, the composition has an initial pD of about 6.9. In some embodiments, the composition has an initial pD of about 6.8. In some embodiments, the composition has an initial pD of about 6.7. In some embodiments, the composition has an initial pD of about 6.6. In some embodiments, the composition has an initial pD of about 6.5. In some embodiments, the composition has an initial pD of about 6.4. In some embodiments, the composition has an initial pD of about 6.3. In some embodiments, the composition has an initial pD of about 6.2. In some embodiments, the composition has an initial pD of about 6.1. In some embodiments, the composition has an initial pD of about 6. In some embodiments, the composition has an initial pD of about 5.9. In some embodiments, the composition has an initial pD of about 5.8. In some embodiments, the composition has an initial pD of about 5.7. In some embodiments, the composition has an initial pD of about 5.6. In some embodiments, the composition has an initial pD of about 5.5. In some embodiments, the composition has an initial pD of about 5.4. In some embodiments, the composition has an initial pD of about 5.3. In some embodiments, the composition has an initial pD of about 5.2. In some embodiments, the composition has an initial pD of about 5.1. In some embodiments, the composition has an initial pD of about 5. In some embodiments, the composition has an initial pD of about 4.9. In some embodiments, the composition has an initial pD of about 4.8. In some embodiments, the composition has an initial pD of about 4.7. In some embodiments, the composition has an initial pD of about 4.6. In some embodiments, the composition has an initial pD of about 4.5. In some embodiments, the composition has an initial pD of about 4.4. In some embodiments, the composition has an initial pD of about 4.3. In some embodiments, the composition has an initial pD of about 4.2.

In some embodiments, the pD of the composition described herein is associated with the stability of the composition. In some embodiments, a stable composition comprises a pD of between about 4.5 and about 7.8, about 5 and about 7.5, or about 5.5 and about 7. In some embodiments, a stable composition comprises a pD of less than about 7.5. In some embodiments, a stable composition comprises a pD of less than about 7.4. In some embodiments, a stable composition comprises a pD of less than about 7.3. In some embodiments, a stable composition comprises a pD of less than about 7.2. In some embodiments, a stable composition comprises a pD of less than about 7.1. In some embodiments, a stable composition comprises a pD of less than about 7. In some embodiments, a stable composition comprises a pD of less than about 6.9. In some embodiments, a stable composition comprises a pD of less than about 6.8. In some embodiments, a stable composition comprises a pD of less than about 6.7. In some embodiments, a stable composition comprises a pD of less than about 6.6. In some embodiments, a stable composition comprises a pD of less than about 6.5. In some embodiments, a stable composition comprises a pD of less than about 6.4. In some embodiments, a stable composition comprises a pD of less than about 6.3. In some embodiments, a stable composition comprises a pD of less than about 6.2. In some embodiments, a stable composition comprises a pD of less than about 6.1. In some embodiments, a stable composition comprises a pD of less than about 6. In some embodiments, a stable composition comprises a pD of less than about 5.9. In some embodiments, a stable composition comprises a pD of less than about 5.8. In some embodiments, a stable composition comprises a pD of less than about 5.7. In some embodiments, a stable composition comprises a pD of less than about 5.6. In some embodiments, a stable composition comprises a pD of less than about 5.5. In some embodiments, a stable composition comprises a pD of less than about 5.4. In some embodiments, a stable composition comprises a pD of less than about 5.3. In some embodiments, a stable composition comprises a pD of less than about 5.2. In some embodiments, a stable composition comprises a pD of less than about 5.1. In some embodiments, a stable composition comprises a pD of less than about 5. In some embodiments, a stable composition comprises a pD of less than about 4.9. In some embodiments, a stable composition comprises a pD of less than about 4.8. In some embodiments, a stable composition comprises a pD of less than about 4.7. In some embodiments, a stable composition comprises a pD of less than about 4.6. In some embodiments, a stable composition comprises a pD of less than about 4.5. In some embodiments, a stable composition comprises a pD of less than about 4.4. In some embodiments, a stable composition comprises a pD of less than about 4.3.

As described elsewhere herein, in some instances, the D₂O aqueous system stabilizes a muscarinic antagonist (e.g., atropine). In some embodiments, this is due to a lower concentration of the reactive species (e.g., -OD) in the D₂O aqueous system compared to the concentration of the reactive species (e.g., -OH) in an equivalent H₂O aqueous system. In some instances, the concentration of the reactive species (e.g., -OD) in the D₂O aqueous system is about one third less than the concentration of the reactive species (e.g., -OH) in the equivalent H₂O aqueous system. In some cases, this is due to a lower or smaller dissociation constant of D₂O than H₂O. For example, the K,(H₂O) is 1x10⁻¹⁴, whereas the Kₐ(D₂O) is 1x10⁻¹⁵. As such, D₂O is a weaker acid than H₂O. In some cases, base catalyzed hydrolysis leads to the presence of tropine degradant from atropine. In some cases, with a lower concentration of the reactive species that causes tropine degradant formation, atropine solution is more stable in a D₂O aqueous system than compared to an equivalent H₂O aqueous system. In some embodiments, the ophthalmic composition formulated with deuterated water allows for a more stable ophthalmic composition relative to the ophthalmic composition formulated with H₂O.

In some instances, the D₂O aqueous system stabilizes an ophthalmic composition comprising an ophthalmic agent. In such instances, this is due to a lower concentration of the reactive species (e.g., -OD) in the D₂O aqueous system compared to the concentration of the reactive species (e.g., - OH) in an equivalent H₂O aqueous system. In some instances, the concentration of the reactive species (e.g., -OD) in the D₂O aqueous system is about one third less than the concentration of the reactive species (e.g., -OH) in the equivalent H₂O aqueous system. In some cases, this is due to a lower or smaller dissociation constant of D₂O than H₂O. For example, the Kₐ(H₂O) is 1x10⁻¹⁴, whereas the Kₐ(D₂O) is 1x10⁻¹⁵. As such, D₂O is a weaker acid than H₂O. In some cases, base catalyzed hydrolysis leads to the presence of a degradant from the ophthalmic agent. In some cases, with a lower concentration of the reactive species that causes degradant formation, the ophthalmic solution is more stable in a D₂O aqueous system than compared to an equivalent H₂O aqueous system. In some instances, the ophthalmic composition formulated with deuterated water allows for a more stable ophthalmic composition relative to the ophthalmic composition formulated with H₂O.

In some embodiments, the presence of deuterated water shifts the pKa of the buffer. In some embodiments, the presence of deuterated water allows for the ophthalmic composition to simulate the stability of a lower pH system. In some instances, the buffer capacity of the ophthalmic composition is lowered, thereby allowing a faster shift in pH. In some instances, the lowered buffering capacity of the ophthalmic composition when administered into the eye allows the ophthalmic composition to reach physiological pH at a faster rate than compared to an ophthalmic composition formulated in H₂O. In some instances, the ophthalmic composition formulated with deuterated water allows for a lower tear production, or less tear reflex in the eye, in comparison with an ophthalmic composition formulated with H₂O.

In some instances, the composition described herein further comprises a disinfecting agent. In some cases, disinfecting agents include polymeric biguanides, polymeric quaternary ammonium compounds, chlorites, bisbiguanides, chlorite compounds (e.g. potassium chlorite, sodium chlorite, calcium chlorite, magnesium chlorite, or mixtures thereof), and a combination thereof.

In some instances, the composition described herein further comprises a preservative. In some cases, a preservative is added at a concentration to a composition described herein to prevent the growth of or to destroy a microorganism introduced into the composition. In some instances, microorganisms refer to bacteria (e.g. *Proteus mirabilis, Serratia marcesens),* virus (e.g. Herpes simplex virus, herpes zoster virus), fungus (e.g. fungi from the genus Fusarium), yeast (e.g. *Candida albicans),* parasites (e.g. *Plasmodium* spp., *Gnathostoma* spp.), protozoan (e.g. *Giardia lamblia),* nematodes (e.g. *Onchocercus volvulus),* worm (e.g. *Dirofilaria immitis),* and/or amoeba (e.g. Acanthameoba).

In some instances, the concentration of the preservative is between about 0.0001% and about 1%, about 0.001% and about 0.8%, about 0.004% and about 0.5%, about 0.008 % and about 0.1%, and about 0.01% and about 0.08%. In some cases, the concentration of the preservatives is about 0.001%, 0.002%, 0.003%, 0.004%, 0.005%, 0.006%, 0.008%, 0.009%, 0.009%, 0.01%, 0.015%, 0.02%, 0.025%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9% or 1.0%.

In some embodiments, the preservative is selected from benzalkonium chloride, cetrimonium, sodium perborate, stabilized oxychloro complex, SofZia (Alcon), polyquaternium-1, chlorobutanol, edetate disodium, and polyhexamethylene biguanide.

In some embodiments, the composition described herein is substantially preservative-free. In some cases, the composition described herein comprises less than about 1%, less than about 0.5%, less than about 0.4%, less than about 0.3%, less than about 0.2%, less than about 0.1%, less than about 0.01%, less than about 0.001%, or less than about 0.0001% of a preservative. In some cases, the composition described herein is preservative-free.

In some embodiments, the composition described herein is stored in a reservoir of a fluid-dispensing device. As described elsewhere herein, the reservoir comprises a plastic material and/or a glass material. In some embodiments, the plastic material comprises high-density polyethylene (HDPE), low-density polyethylene (LDPE), polyethylene terephthalate (PET), polyvinyl chloride (PVC), polypropylene (PP), polystyrene (PS), fluorine treated HDPE, post-consumer resin (PCR), K-resin (SBC), or bioplastic. In some embodiments, the material of the plastic reservoir comprises LDPE.

In some embodiments, the composition described herein is stored in a plastic reservoir. In some embodiments, the composition stored in a plastic reservoir has a pD of between about 4.5 and about 7.9, or about 4.9 and about 7.5. In some embodiments, the composition stored in a plastic reservoir has a pD of less than about 7.4. In some embodiments, the composition stored in a plastic reservoir has a pD of less than about 7.3. In some embodiments, the composition stored in a plastic reservoir has a pD of less than about 7.2. In some embodiments, the composition stored in a plastic reservoir has a pD of less than about 7.1. In some embodiments, the composition stored in a plastic reservoir has a pD of less than about 7. In some embodiments, the composition stored in a plastic reservoir has a pD of less than about 6.9. In some embodiments, the composition stored in a plastic reservoir has a pD of less than about 6.8. In some embodiments, the composition stored in a plastic reservoir has a pD of less than about 6.7. In some embodiments, the composition stored in a plastic reservoir has a pD of less than about 6.6. In some embodiments, the composition stored in a plastic reservoir has a pD of less than about 6.5. In some embodiments, the composition stored in a plastic reservoir has a pD of less than about 6.4. In some embodiments, the composition stored in a plastic reservoir has a pD of less than about 6.3. In some embodiments, the composition stored in a plastic reservoir has a pD of less than about 6.2. In some embodiments, the composition stored in a plastic reservoir has a pD of less than about 6.1. In some embodiments, the composition stored in a plastic reservoir has a pD of less than about 6. In some embodiments, the composition stored in a plastic reservoir has a pD of less than about 5.9. In some embodiments, the composition stored in a plastic reservoir has a pD of less than about 5.8. In some embodiments, the composition stored in a plastic reservoir has a pD of less than about 5.7. In some embodiments, the composition stored in a plastic reservoir has a pD of less than about 5.6. In some embodiments, the composition stored in a plastic reservoir has a pD of less than about 5.5. In some embodiments, the composition stored in a plastic reservoir has a pD of less than about 5.4. In some embodiments, the composition stored in a plastic reservoir has a pD of less than about 5.3. In some embodiments, the composition stored in a plastic reservoir has a pD of less than about 5.2. In some embodiments, the composition stored in a plastic reservoir has a pD of less than about 5.1. In some embodiments, the composition stored in a plastic reservoir has a pD of less than about 5. In some embodiments, the composition stored in a plastic reservoir has a pD of less than about 4.9. In some embodiments, the composition stored in a plastic reservoir has a pD of less than about 4.8. In some embodiments, the composition stored in a plastic reservoir has a pD of less than about 4.7. In some embodiments, the composition stored in a plastic reservoir has a pD of less than about 4.6. In some embodiments, the composition stored in a plastic reservoir has a pD of less than about 4.5. In some embodiments, the composition stored in a plastic reservoir has a pD of less than about 4.4. In some embodiments, the composition stored in a plastic reservoir has a pD of less than about 4.3.

In some embodiments, the composition stored in a plastic reservoir has a potency of at least 80% after extended period of time under storage condition. In some embodiments, the composition stored in a plastic reservoir has a potency of at least 85% after extended period of time under storage condition. In some embodiments, the composition stored in a plastic reservoir has a potency of at least 90% after extended period of time under storage condition. In some embodiments, the composition stored in a plastic reservoir has a potency of at least 93% after extended period of time under storage condition. In some embodiments, the composition stored in a plastic reservoir has a potency of at least 95% after extended period of time under storage condition. In some embodiments, the composition stored in a plastic reservoir has a potency of at least 97% after extended period of time under storage condition. In some embodiments, the composition stored in a plastic reservoir has a potency of at least 98% after extended period of time under storage condition. In some embodiments, the composition stored in a plastic reservoir has a potency of at least 99% after extended period of time under storage condition. In some instances, the storage condition comprises a temperature of about 25°C, about 40°C, or about 60°C. In some instances, the extended period of time is at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months.

In some embodiments, the composition stored in a plastic reservoir has a potency of at least 80% at a temperature of about 25°C, about 40°C, or about 60°C. In some embodiments, the composition stored in a plastic reservoir has a potency of at least 85% at a temperature of about 25°C, about 40°C, or about 60°C. In some embodiments, the composition stored in a plastic reservoir has a potency of at least 90% at a temperature of about 25°C, about 40°C, or about 60°C. In some embodiments, the composition stored in a plastic reservoir has a potency of at least 93% at a temperature of about 25°C, about 40°C, or about 60°C. In some embodiments, the composition stored in a plastic reservoir has a potency of at least 95% at a temperature of about 25°C, about 40°C, or about 60°C. In some embodiments, the composition stored in a plastic reservoir has a potency of at least 97% at a temperature of about 25°C, about 40°C, or about 60°C. In some embodiments, the composition stored in a plastic reservoir has a potency of at least 98% at a temperature of about 25°C, about 40°C, or about 60°C. In some embodiments, the composition stored in a plastic reservoir has a potency of at least 99% at a temperature of about 25°C, about 40°C, or about 60°C.

In some embodiments, the composition stored in a plastic reservoir has a potency of at least 80% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir has a potency of at least 85% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir has a potency of at least 90% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir has a potency of at least 93% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir has a potency of at least 95% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir has a potency of at least 97% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir has a potency of at least 98% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir has a potency of at least 99% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months.

In some embodiments, the composition stored in a plastic reservoir comprises less than 20% of primary degradant based on the concentration of the muscarinic antagonist or ophthalmic agent after extended period of time under storage condition. In some embodiments, the composition stored in a plastic reservoir comprises less than 15% of primary degradant based on the concentration of the muscarinic antagonist or ophthalmic agent after extended period of time under storage condition. In some embodiments, the composition stored in a plastic reservoir comprises less than 10% of primary degradant based on the concentration of the muscarinic antagonist or ophthalmic agent after extended period of time under storage condition. In some embodiments, the composition stored in a plastic reservoir comprises less than 5% of primary degradant based on the concentration of the muscarinic antagonist or ophthalmic agent after extended period of time under storage condition.

In some embodiments, the composition stored in a plastic reservoir comprises from less than 2.5% of primary degradant to less than 0.1% of primary degradant based on the concentration of the muscarinic antagonist or ophthalmic agent after extended period of time under storage condition. In some embodiments, the composition stored in a plastic reservoir comprises less than 2.5% of primary degradant based on the concentration of the muscarinic antagonist or ophthalmic agent after extended period of time under storage condition. In some embodiments, the composition stored in a plastic reservoir comprises less than 2.0% of primary degradant based on the concentration of the muscarinic antagonist or ophthalmic agent after extended period of time under storage condition. In some embodiments, the composition stored in a plastic reservoir comprises less than 1.5% of primary degradant based on the concentration of the muscarinic antagonist or ophthalmic agent after extended period of time under storage condition. In some embodiments, the composition stored in a plastic reservoir comprises less than 1.0% of primary degradant based on the concentration of the muscarinic antagonist or ophthalmic agent after extended period of time under storage condition. In some embodiments, the composition stored in a plastic reservoir comprises less than 0.5% of primary degradant based on the concentration of the muscarinic antagonist or ophthalmic agent after extended period of time under storage condition. In some embodiments, the composition stored in a plastic reservoir comprises less than 0.4% of primary degradant based on the concentration of the muscarinic antagonist or ophthalmic agent after extended period of time under storage condition. In some embodiments, the composition stored in a plastic reservoir comprises less than 0.3% of primary degradant based on the concentration of the muscarinic antagonist or ophthalmic agent after extended period of time under storage condition. In some embodiments, the composition stored in a plastic reservoir comprises less than 0.2% of primary degradant based on the concentration of the muscarinic antagonist or ophthalmic agent after extended period of time under storage condition. In some embodiments, the composition stored in a plastic reservoir comprises less than 0.1% of primary degradant based on the concentration of the muscarinic antagonist or ophthalmic agent after extended period of time under storage condition. In some instances, the storage condition comprises a temperature of about 25°C, about 40°C, or about 60°C. In some instances, the extended period of time is at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months.

In some embodiments, the composition stored in a plastic reservoir comprises less than 20% of primary degradant based on the concentration of the muscarinic antagonist or ophthalmic agent at a temperature of about 25°C, about 40°C, or about 60°C. In some embodiments, the composition stored in a plastic reservoir comprises less than 15% of primary degradant based on the concentration of the muscarinic antagonist or ophthalmic agent at a temperature of about 25°C, about 40°C, or about 60°C. In some embodiments, the composition stored in a plastic reservoir comprises less than 10% of primary degradant based on the concentration of the muscarinic antagonist or ophthalmic agent at a temperature of about 25°C, about 40°C, or about 60°C. In some embodiments, the composition stored in a plastic reservoir comprises less than 5% of primary degradant based on the concentration of the muscarinic antagonist or ophthalmic agent at a temperature of about 25°C, about 40°C, or about 60°C.

In some embodiments, the composition stored in a plastic reservoir comprises from less than 2.5% of primary degradant to less than 0.1% of primary degradant based on the concentration of the muscarinic antagonist or ophthalmic agent at a temperature of about 25°C, about 40°C, or about 60°C. In some embodiments, the composition stored in a plastic reservoir comprises less than 2.5% of primary degradant based on the concentration of the muscarinic antagonist or ophthalmic agent at a temperature of about 25°C, about 40°C, or about 60°C. In some embodiments, the composition stored in a plastic reservoir comprises less than 2.0% of primary degradant based on the concentration of the muscarinic antagonist or ophthalmic agent at a temperature of about 25°C, about 40°C, or about 60°C. In some embodiments, the composition stored in a plastic reservoir comprises less than 1.5% of primary degradant based on the concentration of the muscarinic antagonist or ophthalmic agent at a temperature of about 25°C, about 40°C, or about 60°C. In some embodiments, the composition stored in a plastic reservoir comprises less than 1.0% of primary degradant based on the concentration of the muscarinic antagonist or ophthalmic agent at a temperature of about 25°C, about 40°C, or about 60°C. In some embodiments, the composition stored in a plastic reservoir comprises less than 0.5% of primary degradant based on the concentration of the muscarinic antagonist or ophthalmic agent at a temperature of about 25°C, about 40°C, or about 60°C. In some embodiments, the composition stored in a plastic reservoir comprises less than 0.4% of primary degradant based on the concentration of the muscarinic antagonist or ophthalmic agent at a temperature of about 25°C, about 40°C, or about 60°C. In some embodiments, the composition stored in a plastic reservoir comprises less than 0.3% of primary degradant based on the concentration of the muscarinic antagonist or ophthalmic agent at a temperature of about 25°C, about 40°C, or about 60°C. In some embodiments, the composition stored in a plastic reservoir comprises less than 0.2% of primary degradant based on the concentration of the muscarinic antagonist or ophthalmic agent at a temperature of about 25°C, about 40°C, or about 60°C. In some embodiments, the composition stored in a plastic reservoir comprises less than 0.1% of primary degradant based on the concentration of the muscarinic antagonist or ophthalmic agent at a temperature of about 25°C, about 40°C, or about 60°C.

In some embodiments, the composition stored in a plastic reservoir comprises less than 20% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 15% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 10% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 5% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months.

In some embodiments, the composition stored in a plastic reservoir comprises from less than 2.5% of primary degradant to less than 0.1% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 2.5% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 2.0% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 1.5% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 1.0% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 0.5% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 0.4% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 0.3% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 0.2% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months. In some embodiments, the composition stored in a plastic reservoir comprises less than 0.1% of primary degradant based on the concentration of the muscarinic antagonist or ophthalmic agent for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months.

In some embodiments, the composition stored in a plastic reservoir comprises one of: less than about 60 colony forming units (CFU), less than about 50 colony forming units, less than about 40 colony forming units, or less than about 30 colony forming units of microbial agents per gram of formulation. In some cases, the composition stored in a plastic reservoir is substantially free of microorganism. In some cases, the composition is substantially preservative-free.

In some cases, the composition stored in a plastic reservoir is substantially free of endotoxins. In some cases, the composition is substantially preservative-free.

In some embodiments, the composition described herein is stored in a glass reservoir. In some embodiments, the glass reservoir is a glass vial, such as for example, a type I, type II or type III glass vial. In some embodiments, the glass reservoir is a type I glass vial. In some embodiments, the type I glass vial is a borosilicate glass vial.

In some embodiments, the composition stored in a glass reservoir has a pD of higher than about 7. In some embodiments, the composition stored in a glass reservoir has a pD of higher than about 7.5.

In some embodiments, the composition stored in a glass reservoir has a potency of less than 60% at a temperature of about 25°C, about 40°C, or about 60°C. In some embodiments, the composition stored in a glass reservoir has a potency of less than 60% for a period of at least 1 week, at least 2 weeks, at least 3 weeks, at least 1 month, at least 2 months, at least 3 months, at least 4 months, at least 5 months, at least 6 months, at least 8 months, at least 10 months, at least 12 months, at least 18 months, or at least 24 months.

In some embodiments, the composition stored in a glass reservoir comprises one of: less than about 60 colony forming units (CFU), less than about 50 colony forming units, less than about 40 colony forming units, or less than about 30 colony forming units of microbial agents per gram of formulation. In some cases, the composition stored in a glass reservoir is substantially free of microorganism. In some cases, the composition is substantially preservative-free.

In some cases, the composition stored in a glass reservoir is substantially free of endotoxins. In some cases, the composition is substantially preservative-free.

In some embodiments, the composition stored in a glass reservoir is less stable than a composition stored in a plastic reservoir.

In some embodiments, the composition is stored under in the dark. In some instances, the composition is stored in the presence of light. In some instances, the light is indoor light, room light, or sun light. In some instances, the composition is stable while stored in the presence of light.

In some embodiments, the composition described herein is formulated as an aqueous solution. In some embodiments, the aqueous solution is a stable aqueous solution. In some instances, the aqueous solution is stored in a plastic reservoir as described above. In some instances, the aqueous solution is not stored in a glass reservoir. In some instances, the aqueous solution is stored in the dark. In some instances, the aqueous solution is stored in the presence of light. In some instances, the aqueous solution is stable in the presence of light.

In a specific embodiment, the ophthalmically acceptable formulations alternatively comprise a cyclodextrin. Cyclodextrins are cyclic oligosaccharides containing 6, 7, or 8 glucopyranose units, referred to as α-cyclodextrin, β-cyclodextrin, or γ-cyclodextrin respectively. Cyclodextrins have a hydrophilic exterior, which enhances water-soluble, and a hydrophobic interior which forms a cavity. In an aqueous environment, hydrophobic portions of other molecules often enter the hydrophobic cavity of cyclodextrin to form inclusion compounds. Additionally, cyclodextrins are also capable of other types of nonbonding interactions with molecules that are not inside the hydrophobic cavity. Cyclodextrins have three free hydroxyl groups for each glucopyranose unit, or 18 hydroxyl groups on α-cyclodextrin, 21 hydroxyl groups on β-cyclodextrin, and 24 hydroxyl groups on γ-cyclodextrin. In some embodiments, one or more of these hydroxyl groups are reacted with any of a number of reagents to form a large variety of cyclodextrin derivatives, including hydroxypropyl ethers, sulfonates, and sulfoalkylethers. Shown below is the structure of β-cyclodextrin and the hydroxypropyl-β-cyclodextrin (HPβCD). 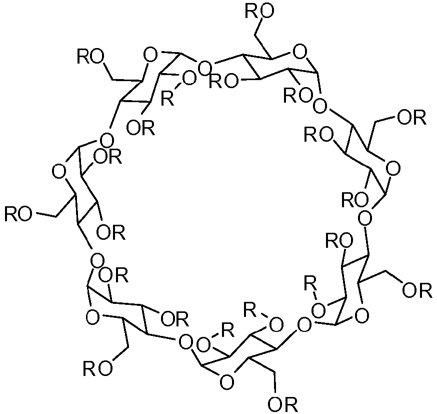
R = H
β-cyclodextrin
R = CH₂CH(OH)CH₃
hydroxypropyl β-cyclodextrin

In some embodiments, the use of cyclodextrins in the pharmaceutical compositions described herein improves the solubility of the drug. Inclusion compounds are involved in many cases of enhanced solubility; however other interactions between cyclodextrins and insoluble compounds also improves solubility. Hydroxypropyl-β-cyclodextrin (HPβCD) is commercially available as a pyrogen free product. It is a nonhygroscopic white powder that readily dissolves in water. HPβCD is thermally stable and does not degrade at neutral pH. Thus, cyclodextrins improve the solubility of a therapeutic agent in a composition or formulation. Accordingly, in some embodiments, cyclodextrins are included to increase the solubility of the ophthalmically acceptable muscarinic antagonists or ophthalmic agents within the formulations described herein. In other embodiments, cyclodextrins in addition serve as controlled release excipients within the formulations described herein.

By way of example only, cyclodextrin derivatives for use include α-cyclodextrin, β-cyclodextrin, γ-cyclodextrin, hydroxyethyl-β-cyclodextrin, hydroxypropyl-γ-cyclodextrin, sulfated β-cyclodextrin, sulfated α-cyclodextrin, sulfobutyl ether β-cyclodextrin.

The concentration of the cyclodextrin used in the compositions and methods disclosed herein varies according to the physiochemical properties, pharmacokinetic properties, side effect or adverse events, formulation considerations, or other factors associated with the therapeutically muscarinic antagonist or ophthalmic agent or with the properties of other excipients in the composition. Thus, in certain circumstances, the concentration or amount of cyclodextrin used in accordance with the compositions and methods disclosed herein will vary, depending on the need. When used, the amount of cyclodextrins needed to increase solubility of the muscarinic antagonist or ophthalmic agent and/or function as a controlled release excipient in any of the formulations described herein is selected using the principles, examples, and teachings described herein.

Other stabilizers that are useful in the ophthalmically acceptable formulations disclosed herein include, for example, fatty acids, fatty alcohols, alcohols, long chain fatty acid esters, long chain ethers, hydrophilic derivatives of fatty acids, polyvinyl pyrrolidones, polyvinyl ethers, polyvinyl alcohols, hydrocarbons, hydrophobic polymers, moisture-absorbing polymers, and combinations thereof. In some embodiments, amide analogues of stabilizers are also used. In further embodiments, the chosen stabilizer changes the hydrophobicity of the formulation, improves the mixing of various components in the formulation, controls the moisture level in the formula, or controls the mobility of the phase.

In other embodiments, stabilizers are present in sufficient amounts to inhibit the degradation of the muscarinic antagonist or ophthalmic agent. Examples of such stabilizing agents, include, but are not limited to: glycerol, methionine, monothioglycerol, EDTA, ascorbic acid, polysorbate 80, polysorbate 20, arginine, heparin, dextran sulfate, cyclodextrins, pentosan polysulfate and other heparinoids, divalent cations such as magnesium and zinc, or combinations thereof.

Additional useful stabilization agents for ophthalmically acceptable formulations include one or more anti-aggregation additives to enhance stability of ophthalmic formulations by reducing the rate of protein aggregation. The anti-aggregation additive selected depends upon the nature of the conditions to which the muscarinic antagonist or ophthalmic agents, for example a muscarinic antagonist (e.g. atropine or its pharmaceutically acceptable salts), are exposed. For example, certain formulations undergoing agitation and thermal stress require a different anti-aggregation additive than a formulation undergoing lyophilization and reconstitution. Useful anti-aggregation additives include, by way of example only, urea, guanidinium chloride, simple amino acids such as glycine or arginine, sugars, polyalcohols, polysorbates, polymers such as polyethylene glycol and dextrans, alkyl saccharides, such as alkyl glycoside, and surfactants.

Other useful formulations optionally include one or more ophthalmically acceptable antioxidants to enhance chemical stability where required. Suitable antioxidants include, by way of example only, ascorbic acid, methionine, sodium thiosulfate and sodium metabisulfite. In one embodiment, antioxidants are selected from metal chelating agents, thiol containing compounds and other general stabilizing agents.

Still other useful compositions include one or more ophthalmically acceptable surfactants to enhance physical stability or for other purposes. Suitable nonionic surfactants include, but are not limited to, polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40.

In some embodiments, the ophthalmically acceptable pharmaceutical formulations described herein are stable with respect to compound degradation (e.g. less than 30% degradation, less than 25% degradation, less than 20% degradation, less than 15% degradation, less than 10% degradation, less than 8% degradation, less than 5% degradation, less than 3% degradation, less than 2% degradation, or less than 5% degradation) over a period of any of at least about 1 day, at least about 2 days, at least about 3 days, at least about 4 days, at least about 5 days, at least about 6 days, at least about 1 week, at least about 2 weeks, at least about 3 weeks, at least about 4 weeks, at least about 5 weeks, at least about 6 weeks, at least about 7 weeks, at least about 8 weeks, at least about 3 months, at least about 4 months, at least about 5 months, or at least about 6 months under storage conditions (e.g. room temperature). In other embodiments, the formulations described herein are stable with respect to compound degradation over a period of at least about 1 week. Also described herein are formulations that are stable with respect to compound degradation over a period of at least about 1 month.

In other embodiments, an additional surfactant (co-surfactant) and/or buffering agent is combined with one or more of the pharmaceutically acceptable vehicles previously described herein so that the surfactant and/or buffering agent maintains the product at an optimal pD for stability. Suitable co-surfactants include, but are not limited to: a) natural and synthetic lipophilic agents, e.g., phospholipids, cholesterol, and cholesterol fatty acid esters and derivatives thereof; b) nonionic surfactants, which include for example, polyoxyethylene fatty alcohol esters, sorbitan fatty acid esters (Spans), polyoxyethylene sorbitan fatty acid esters (e.g., polyoxyethylene (20) sorbitan monooleate (Tween 80), polyoxyethylene (20) sorbitan monostearate (Tween 60), polyoxyethylene (20) sorbitan monolaurate (Tween 20) and other Tweens, sorbitan esters, glycerol esters, e.g., Myrj and glycerol triacetate (triacetin), polyethylene glycols, cetyl alcohol, cetostearyl alcohol, stearyl alcohol, polysorbate 80, poloxamers, poloxamines, polyoxyethylene castor oil derivatives (e.g., Cremophor^{®} RH40, Cremphor A25, Cremphor A20, Cremophor^{®} EL) and other Cremophors, sulfosuccinates, alkyl sulphates (SLS); PEG glyceryl fatty acid esters such as PEG-8 glyceryl caprylate/caprate (Labrasol), PEG-4 glyceryl caprylate/caprate (Labrafac Hydro WL 1219), PEG-32 glyceryl laurate (Gelucire 444/14), PEG-6 glyceryl mono oleate (Labrafil M 1944 CS), PEG-6 glyceryl linoleate (Labrafil M 2125 CS); propylene glycol mono- and di-fatty acid esters, such as propylene glycol laurate, propylene glycol caprylate/caprate; Brij^{®} 700, ascorbyl-6-palmitate, stearylamine, sodium lauryl sulfate, polyoxethyleneglycerol triiricinoleate, and any combinations or mixtures thereof; c) anionic surfactants include, but are not limited to, calcium carboxymethylcellulose, sodium carboxymethylcellulose, sodium sulfosuccinate, dioctyl, sodium alginate, alkyl polyoxyethylene sulfates, sodium lauryl sulfate, triethanolamine stearate, potassium laurate, bile salts, and any combinations or mixtures thereof; and d) cationic surfactants such as cetyltrimethylammonium bromide, and lauryldimethylbenzyl-ammonium chloride.

In a further embodiment, when one or more co-surfactants are utilized in the ophthalmically acceptable formulations of the present disclosure, they are combined, e.g., with a pharmaceutically acceptable vehicle and is present in the final formulation, e.g., in an amount ranging from about 0.1% to about 20%, from about 0.5% to about 10%.

In one embodiment, the surfactant has an HLB value of 0 to 20. In additional embodiments, the surfactant has an HLB value of 0 to 3, of 4 to 6, of 7 to 9, of 8 to 18, of 13 to 15, of 10 to 18.

### Fluid-Dispensing Device

The invention as described herein includes an ophthalmic product, which comprises a fluid-dispensing device comprising an internal filter or membrane, a reservoir and a dispensing tip fitted onto the reservoir, and the composition described herein, wherein the composition is dispensable from the dispensing tip into an eye of an individual in need thereof. In some embodiments, the composition in the reservoir is substantially preservative-free. In other embodiments, the composition in the reservoir comprises a preservative, but is filtered prior to dispensing from the dispensing tip, and the dispensed composition is preservative-free.

The ophthalmic composition comprises a muscarinic antagonist. The ophthalmic product comprises a fluid-dispensing device comprising a reservoir and a dispensing tip fitted onto the reservoir; and an ophthalmic composition comprising from about 0.001 wt% to about 0.05 wt% of a muscarinic antagonist and deuterated water, at a pD of from about 4.2 to about 7.9, in the reservoir; wherein the ophthalmic composition is dispensable from the dispensing tip into an eye of an individual in need thereof, and wherein the dispensed ophthalmic composition is preservative-free.

In some instances, the ophthalmic composition comprises an ophthalmic agent. In some cases, the ophthalmic product comprises a fluid-dispensing device comprising a reservoir and a dispensing tip fitted onto the reservoir; and an ophthalmic composition comprising an ophthalmic agent and deuterated water, at a pD of from about 4 to about 8, in the reservoir; wherein the ophthalmic agent is not a muscarinic antagonist and does not extend singlet oxygen lifetime, wherein the ophthalmic composition is dispensed from the dispensing tip into an eye of an individual in need thereof, and wherein the dispensed ophthalmic composition is substantially preservative-free.

As used herein, the term "substantially preservative-free" refers to the composition as having one of: less than about 1%, less than about 0.5%, less than about 0.4%, less than about 0.3%, less than about 0.2%, less than about 0.1%, less than about 0.01%, or less than about 0.001% of a preservative. In some instances, the term refers to the composition as having 0% of a preservative, or preservative-free.

In some embodiments, the reservoir comprises of a polymeric material, for example, polyvinyl chloride (PVC) plastics or non-PVC plastics. In some instances, the material of the reservoir comprises high-density polyethylene (HDPE), low-density polyethylene (LDPE), polyethylene terephthalate (PET), polyvinyl chloride (PVC), polypropylene (PP), polystyrene (PS), fluorine treated HDPE, post-consumer resin (PCR), K-resin (SBC), or bioplastic. In some embodiments, the material of the reservoir comprises ethylene vinyl acetate (EVA) and block copolymers such as Kraton^{®}. In some cases, the material of the reservoir comprises high-density polyethylene (HDPE). In some cases, the material of the reservoir comprises low-density polyethylene (LDPE). In some cases, the material of the reservoir comprises polyethylene terephthalate (PET). In some cases, the material of the reservoir comprises polypropylene (PP). In some cases, the material of the reservoir comprises polystyrene (PS). In some cases, the material of the reservoir comprises ethylene vinyl acetate (EVA).

In some instances, the reservoir further comprises a plasticizer. Exemplary plasticizer includes families of phthalate esters such as di-2-ethylhexylphthalate (DEHP), mono-(2-ethylhexyl) phthalate (MEHP), and triethylhexyltrimellitate (TEHTM); citrate esters such as acetyltri-n-hexyl citrate, acetyltri-n-(hexyl/octyl/decyl) citrate, acetyltri-n-(octyl/decyl) citrate, and n-butyryltri-n-hexyl citrate; and non-phthalate plasticizers such as TEHTM, di(isononyl) cyclohexane-1,2-dicarboxylate (DINCH), or n-butyryltri-n-hexyl citrate.

In some embodiments, the reservoir is at least partially elastically deformable so as to dispense the ophthalmic composition by pressing on the reservoir.

In some embodiments, the reservoir comprises glass.

In some embodiments, the reservoir stores multiple unit doses of the composition described herein.

In some embodiments, the fluid-dispensing device described herein is a multi-dose fluid-dispensing device.

In some embodiments, the fluid-dispensing device described herein enables storage of a preservative-free or substantially preservative-free composition. In some cases, the fluid-dispensing device is a multi-dose preservative-free device.

In some instances, a fluid-dispensing device from Aptar Pharma (AptarGroup) is utilized for delivery of a composition described herein. In some cases, the composition is preservative-free.

In some cases, a fluid-dispensing device from Nemera La Verpillière S.A.S. is utilized for delivery of a composition described herein. In some cases, a fluid-dispensing device as described in U.S. Patent no. 8,986,266 and/or 8,863,998 is utilized for delivery of a composition described herein. In some cases, the composition is preservative-free.

In some cases, a fluid-dispensing device from CIS Pharma is utilized for delivery of a composition described herein. In some cases, the composition is preservative-free.

In some embodiments, the fluid-dispensing device described herein optionally comprises an atomizer, a pump, or a mister. In such cases, a mechanical system such as a pump, a mister, or an atomizer is incorporated into the fluid-dispensing device to facilitate delivery of the composition described herein and optionally to facilitate dose uniformity (e.g., between each administration, minimize excessive drug volume, and/or enhance droplet uniformity). In additional cases, a mechanical system such as a pump, a mister, or an atomizer is incorporated into the fluid-dispensing device to enhance and/or optimize the amount of drug delivered to the eye.

In some instances, an atomizer and/or pump system from Aero Pump GMBH (Adelphi Healthcare Packaging) is utilized with the fluid-dispensing device and the composition described herein. In some instances, a multiple-dosage fluid-dispensing device from Aero Pump GMBH is utilized for delivery of the composition described herein. In some cases, a fluid-dispensing device as described in U.S. Patent Publication 2016/279663 and/or 2015/076174 (Aero Pump GMBH) is utilized with the fluid-dispensing device and the composition described herein.

In some embodiments, a fluid-dispensing device from Eyenovia, Inc. is utilized for delivery of the composition described herein. In some cases, a fluid-dispensing device comprising one or more of a delivery system and/or component described in U.S. Patents and Patent Publications 9,539,604, 9,087,145, 9,463,486, or 2012/143152 are utilized for delivery of the composition described herein.

In some cases, a fluid-dispensing device comprising one or more of a delivery system and/or component from Kedalion Therapeutics is utilized for delivery of the composition described herein.

In some cases, a fluid-dispensing device comprising one or more of a delivery system and/or component from Aptar Pharma (e.g., a pump dispensing system) is utilized for delivery of the composition described herein.

The fluid-dispensing device comprises an internal filter or membrane. In some instances, the internal filter or membrane is located within the fluid-dispensing device at a position capable of removing a preservative from the ophthalmic composition prior to dispensing the ophthalmic composition into the eye of the individual. In some instances, the preservative is selected from benzalkonium chloride, cetrimonium, sodium perborate, stabilized oxychloro complex, SofZia, polyquaternium-1, chlorobutanol, edetate disodium, polyhexamethylene biguanide, or combinations thereof. In some instances, the internal filter or membrane is located within the fluid-dispensing device at a position capable of removing a preservative selected from benzalkonium chloride, cetrimonium, sodium perborate, stabilized oxychloro complex, SofZia, polyquaternium-1, chlorobutanol, edetate disodium, polyhexamethylene biguanide, or combinations thereof, from the ophthalmic composition prior to dispensing the ophthalmic composition into the eye of the individual. In some instances, the internal filter or membrane is located within the fluid-dispensing device at a position capable of removing a preservative selected from benzalkonium chloride (BAK, BAC, or BKC) from the ophthalmic composition prior to dispensing the ophthalmic composition into the eye of the individual. In some cases, the internal filter or membrane is located at the junction connecting the dispensing tip to the reservoir. In other cases, the internal filter or membrane is located within the dispensing tip.

In some instances, the internal filter or membrane is located within the fluid-dispensing device at a position capable of removing a microorganism and/or an endotoxin from the ophthalmic composition prior to dispensing the ophthalmic composition into the eye of the individual. In some cases, the internal filter or membrane is located at the junction connecting the dispensing tip to the reservoir. In other cases, the internal filter or membrane is located within the dispensing tip. In some cases, the ophthalmic composition is a preservative-free composition.

In some cases, the internal filter or membrane comprises cellulose acetate, cellulose nitrate, nylon, polyether sulfone (PES), polypropylene (PP), polyvinyl difluoride (PVDF), silicone, polycarbonate, or a combination thereof.

In some embodiments, a filter system from TearClear is utilized with a fluid-dispensing device and composition described herein. In some cases, a filter system from TearClear removes a preservative from the composition described herein in-situ, e.g., the filter system is within the fluid-dispensing device which removes a preservative from the composition as the composition is passed from the filter and dispensed into the eye of an individual.

According to the invention, the dispensed composition is preservative-free.

In some instances, the droplet volume dispensed from the fluid-dispensing device described herein is from about 0.1 µL to about 50µL. In some instances, the droplet volume is one of: about 0.1 µL to about 40µL, about 0.5 µL to about 30µL, about 1 µL to about 30µL, about 5 µL to about 20µL, about 10 µL to about 20µL, about 5 µL to about 40µL, about 5 µL to about 30µL, about 6 µL to about 8µL, about 6 µL to about 7µL, about 7 µL to about 8µL, about 10 µL to about 40µL, or about 10 µL to about 30µL. In some cases, the droplet volume dispensed from the fluid-dispensing device described herein is about 0.1 µL, about 0.2 µL, about 0.3 µL, about 0.4 µL, about 0.5 µL, about 1 µL, about 5 µL, about 6 µL, about 7 µL, about 8 µL, about 9 µL, about 10 µL, about 20 µL, about 30 µL, about 40 µL, or about 50 µL.

In some embodiments, the linear size or diameter of the droplet when spherical is about 1 up to less than 100 microns. In some cases, the linear size or diameter of the droplet is about 20 to 100 microns, about 1 to 20 microns, 1-15 microns, 1-10 microns, 8-20 microns, 8-15 microns, 8-12 microns, or 1-5 microns. In the context of an aerosol or mist, the size of the droplet is, for example, 1-5 microns, 1-10 microns, less than 10 microns, greater than 10 microns, or up to 100 microns.

In some cases, the diameter of the droplet is calculated using the equation V=4πr³ where the diameter=2r.

In some instances, the fluid-dispensing device is suitable for dispensing the composition described herein having a viscosity described herein. In some cases, the composition has a viscosity of up to 500 mPa·s, up to 600 mPa·s, up to 1000 mPa·s, up to 10,000 mPa·s, or up to 50,000 mPa·s.

In some instances, the fluid-dispensing device described herein facilitates at least 60%, 70%, 80%, 85%, 90%, 95%, or 99% of the ejected mass of a droplet deposited on the eye of an individual. In some cases, the fluid-dispensing device described herein facilitates at least 70% of the ejected mass of a droplet to be deposited on the eye of an individual. In some cases, the fluid-dispensing device described herein facilitates at least 80% of the ejected mass of a droplet to be deposited on the eye of an individual. In some cases, the fluid-dispensing device described herein facilitates at least 90% of the ejected mass of a droplet to be deposited on the eye of an individual. In some cases, the fluid-dispensing device described herein facilitates at least 95% of the ejected mass of a droplet to be deposited on the eye of an individual. In some cases, the fluid-dispensing device described herein facilitates at least 99% of the ejected mass of a droplet to be deposited on the eye of an individual.

### pD

In some embodiments, the pD of a composition described herein is adjusted (e.g., by use of a buffer and/or a pD adjusting agent) to an ophthalmically compatible pD range of from about 4.2 to about 7.9, about 4.5 to about 7.5, or about 5 to about 7. In some embodiments, the ophthalmic composition has a pD of from about 5.0 to about 7.0. In some embodiments, the ophthalmic composition has a pD of from about 5.5 to about 7.0. In some embodiments, the ophthalmic composition has a pD of from about 6.0 to about 7.0.

In some embodiments, useful formulations include one or more pD adjusting agents or buffering agents. Suitable pD adjusting agents or buffers include, but are not limited to, acetate, bicarbonate, ammonium chloride, citrate, phosphate, deuterated forms of acetate, bicarbonate, ammonium chloride, citrate, phosphate, pharmaceutically acceptable salts thereof and combinations or mixtures thereof. In some embodiments, the pD adjusting agents or buffers include deuterated hydrochloric acid (DCl), deuterated sodium hydroxide (NaOD), deuterated acetic acid (CD₃COOD), or deuterated citric acid (C₆D₈O₇).

In one embodiment, when one or more buffers are utilized in the formulations of the present disclosure, they are combined, e.g., with a pharmaceutically acceptable vehicle and are present in the final formulation, e.g., in an amount ranging from about 0.1% to about 20%, from about 0.5% to about 10%. In certain embodiments of the present disclosure, the amount of buffer included in the gel formulations are an amount such that the pD of the gel formulation does not interfere with the body's natural buffering system.

In one embodiment, diluents are also used to stabilize compounds because they provide a more stable environment. In some instances, salts dissolved in buffered solutions (which also provides pD control or maintenance) are utilized as diluents in the art, including, but not limited to, a phosphate buffered saline solution.

In some embodiments, the pD is calculated according to the formula disclosed in Glasoe et al., "Use of glass electrodes to measure acidities in deuterium oxide," J. Physical Chem. 64(1): 188-190 (1960). In some embodiment, the pD is calculated as pD = pH* + 0.4, in which pH* is the measured or observed pH of the ophthalmic composition formulated in a solution comprising deuterated water (e.g., D₂O).

In some embodiments, the ophthalmic aqueous or gel composition described herein has a pD of between between about 4.5 and about 8, between about 4.9 and about 7.9, between about 5.4 and about 7.9, between about 5.9 and about 7.9, between about 6.4 and about 7.9, or between about 7.4 and about 7.9. In some embodiments, the ophthalmic aqueous or gel composition described herein has a pD of between about 4.5-7.5, between about 5.0 and about 7.5, between about 5.5 and about 7.5, between about 6.0 and about 7.5, or between about 7.0 and about 7.5. In some embodiments, the ophthalmic aqueous or gel composition described herein has a pD of between about 4.5-7.0, between about 5.0 and about 7.0, between about 5.5 and about 7.0, between about 6.0 and about 7.0, or between about 6.5 and about 7.0. In some embodiments, the ophthalmic aqueous or gel composition described herein has a pD of between about 4.9-7.4, between about 5.4 and about 7.4, between about 5.9 and about 7.4, between about 6.4 and about 7.4, or between about 6.9 and about 7.4. In some embodiments, the ophthalmic aqueous or gel composition described herein has a pD of between about 4.5-6.5, between about 5.0 and about 6.5, between about 5.5 and about 6.5, or between about 6.0 and about 6.5. In some embodiments, the ophthalmic aqueous or gel composition described herein has a pD of between about 4.9-6.9, between about 5.4 and about 6.9, between about 5.9 and about 6.9, or between about 6.4 and about 6.9. In some embodiments, the ophthalmic aqueous or gel composition described herein has a pD of between about 4.5-6.0, between about 5.0 and about 6.0, or between about 5.5 and about 6.0. In some embodiments, the ophthalmic aqueous or gel composition described herein has a pD of between about 4.9-6.4, between about 5.4 and about 6.4, or between about 5.9 and about 6.4. In some embodiments, the ophthalmic aqueous or gel composition described herein has a pD of between about 4.5-5.5, or between about 5.0 and about 5.5. In some embodiments, the ophthalmic aqueous or gel composition described herein has a pD of between about 4.9-5.9, or between about 5.4 and about 5.9. In some embodiments, the ophthalmic aqueous or gel composition described herein has a pD of between about 4.5-5.0. In some embodiments, the ophthalmic aqueous or gel composition described herein has a pD of between about 4.9-5.4.

In some embodiments, the ophthalmic composition is an ophthalmic aqueous composition. In some instances, the ophthalmic aqueous composition has a pD of between about 4.5 and about 7.8, about 5 and about 7.5, or about 5.5 and about 7. In some embodiments, the ophthalmic aqueous composition has a pD of about 7.5. In some embodiments, the ophthalmic aqueous composition has a pD of about 7.4. In some embodiments, the ophthalmic aqueous composition has a pD of about 7.3. In some embodiments, the ophthalmic aqueous composition has a pD of about 7.2. In some embodiments, the ophthalmic aqueous composition has a pD of about 7.1. In some embodiments, the ophthalmic aqueous composition has a pD of about 7. In some embodiments, the ophthalmic aqueous composition has a pD of about 6.9. In some embodiments, the ophthalmic aqueous composition has a pD of about 6.8. In some embodiments, the ophthalmic aqueous composition has a pD of about 6.7. In some embodiments, the ophthalmic aqueous composition has a pD of about 6.6. In some embodiments, the ophthalmic aqueous composition has a pD of about 6.5. In some embodiments, the ophthalmic aqueous composition has a pD of about 6.4. In some embodiments, the ophthalmic aqueous composition has a pD of about 6.3. In some embodiments, the ophthalmic aqueous composition has a pD of about 6.2. In some embodiments, the ophthalmic aqueous composition has a pD of about 6.1. In some embodiments, the ophthalmic aqueous composition has a pD of about 6. In some embodiments, the ophthalmic aqueous composition has a pD of about 5.9. In some embodiments, the ophthalmic aqueous composition has a pD of about 5.8. In some embodiments, the ophthalmic aqueous composition has a pD of about 5.7. In some embodiments, the ophthalmic aqueous composition has a pD of about 5.6. In some embodiments, the ophthalmic aqueous composition has a pD of about 5.5. In some embodiments, the ophthalmic aqueous composition has a pD of about 5.4. In some embodiments, the ophthalmic aqueous composition has a pD of about 5.3. In some embodiments, the ophthalmic aqueous composition has a pD of about 5.2. In some embodiments, the ophthalmic aqueous composition has a pD of about 5.1. In some embodiments, the ophthalmic aqueous composition has a pD of about 5. In some embodiments, the ophthalmic aqueous composition has a pD of about 4.9. In some embodiments, the ophthalmic aqueous composition has a pD of about 4.8. In some embodiments, the ophthalmic aqueous composition has a pD of about 4.7. In some embodiments, the ophthalmic aqueous composition has a pD of about 4.6. In some embodiments, the ophthalmic aqueous composition has a pD of about 4.5. In some embodiments, the ophthalmic aqueous composition has a pD of about 4.4. In some embodiments, the ophthalmic aqueous composition has a pD of about 4.3. In some embodiments, the ophthalmic aqueous composition has a pD of about 4.2. In some embodiments, the pD is an initial pD of the ophthalmic aqueous composition. In some embodiments, the pD is the pD of the ophthalmic aqueous composition after extended period of time under storage condition.

In some instances, the ophthalmic aqueous composition has an initial pD of between about 4.5 and about 7.8, about 5 and about 7.5, or about 5.5 and about 7. In some embodiments, the ophthalmic aqueous composition has an initial pD of about 7.5. In some embodiments, the ophthalmic aqueous composition has an initial pD of about 7.4. In some embodiments, the ophthalmic aqueous composition has an initial pD of about 7.3. In some embodiments, the ophthalmic aqueous composition has an initial pD of about 7.2. In some embodiments, the ophthalmic aqueous composition has an initial pD of about 7.1. In some embodiments, the ophthalmic aqueous composition has an initial pD of about 7. In some embodiments, the ophthalmic aqueous composition has an initial pD of about 6.9. In some embodiments, the ophthalmic aqueous composition has an initial pD of about 6.8. In some embodiments, the ophthalmic aqueous composition has an initial pD of about 6.7. In some embodiments, the ophthalmic aqueous composition has an initial pD of about 6.6. In some embodiments, the ophthalmic aqueous composition has an initial pD of about 6.5. In some embodiments, the ophthalmic aqueous composition has an initial pD of about 6.4. In some embodiments, the ophthalmic aqueous composition has an initial pD of about 6.3. In some embodiments, the ophthalmic aqueous composition has an initial pD of about 6.2. In some embodiments, the ophthalmic aqueous composition has an initial pD of about 6.1. In some embodiments, the ophthalmic aqueous composition has an initial pD of about 6. In some embodiments, the ophthalmic aqueous composition has an initial pD of about 5.9. In some embodiments, the ophthalmic aqueous composition has an initial pD of about 5.8. In some embodiments, the ophthalmic aqueous composition has an initial pD of about 5.7. In some embodiments, the ophthalmic aqueous composition has an initial pD of about 5.6. In some embodiments, the ophthalmic aqueous composition has an initial pD of about 5.5. In some embodiments, the ophthalmic aqueous composition has an initial pD of about 5.4. In some embodiments, the ophthalmic aqueous composition has an initial pD of about 5.3. In some embodiments, the ophthalmic aqueous composition has an initial pD of about 5.2. In some embodiments, the ophthalmic aqueous composition has an initial pD of about 5.1. In some embodiments, the ophthalmic aqueous composition has an initial pD of about 5. In some embodiments, the ophthalmic aqueous composition has an initial pD of about 4.9. In some embodiments, the ophthalmic aqueous composition has an initial pD of about 4.8. In some embodiments, the ophthalmic aqueous composition has an initial pD of about 4.7. In some embodiments, the ophthalmic aqueous composition has an initial pD of about 4.6. In some embodiments, the ophthalmic aqueous composition has an initial pD of about 4.5. In some embodiments, the ophthalmic aqueous composition has an initial pD of about 4.4. In some embodiments, the ophthalmic aqueous composition has an initial pD of about 4.3. In some embodiments, the ophthalmic aqueous composition has an initial pD of about 4.2.

In some instances, the ophthalmic aqueous composition has a pD of between about 4.5 and about 7.8, about 5 and about 7.5, or about 5.5 and about 7. In some embodiments, the ophthalmic aqueous composition has a pD of less than about 7.5. In some embodiments, the ophthalmic aqueous composition has a pD of less than about 7.4. In some embodiments, the ophthalmic aqueous composition has a pD of less than about 7.3. In some embodiments, the ophthalmic aqueous composition has a pD of less than about 7.2. In some embodiments, the ophthalmic aqueous composition has a pD of less than about 7.1. In some embodiments, the ophthalmic aqueous composition has a pD of less than about 7. In some embodiments, the ophthalmic aqueous composition has a pD of less than about 6.9. In some embodiments, the ophthalmic aqueous composition has a pD of less than about 6.8. In some embodiments, the ophthalmic aqueous composition has a pD of less than about 6.7. In some embodiments, the ophthalmic aqueous composition has a pD of less than about 6.6. In some embodiments, the ophthalmic aqueous composition has a pD of less than about 6.5. In some embodiments, the ophthalmic aqueous composition has a pD of less than about 6.4. In some embodiments, the ophthalmic aqueous composition has a pD of less than about 6.3. In some embodiments, the ophthalmic aqueous composition has a pD of less than about 6.2. In some embodiments, the ophthalmic aqueous composition has a pD of less than about 6.1. In some embodiments, the ophthalmic aqueous composition has a pD of less than about 6. In some embodiments, the ophthalmic aqueous composition has a pD of less than about 5.9. In some embodiments, the ophthalmic aqueous composition has a pD of less than about 5.8. In some embodiments, the ophthalmic aqueous composition has a pD of less than about 5.7. In some embodiments, the ophthalmic aqueous composition has a pD of less than about 5.6. In some embodiments, the ophthalmic aqueous composition has a pD of less than about 5.5. In some embodiments, the ophthalmic aqueous composition has a pD of less than about 5.4. In some embodiments, the ophthalmic aqueous composition has a pD of less than about 5.3. In some embodiments, the ophthalmic aqueous composition has a pD of less than about 5.2. In some embodiments, the ophthalmic aqueous composition has a pD of less than about 5.1. In some embodiments, the ophthalmic aqueous composition has a pD of less than about 5. In some embodiments, the ophthalmic aqueous composition has a pD of less than about 4.9. In some embodiments, the ophthalmic aqueous composition has a pD of less than about 4.8. In some embodiments, the ophthalmic aqueous composition has a pD of less than about 4.7. In some embodiments, the ophthalmic aqueous composition has a pD of less than about 4.6. In some embodiments, the ophthalmic aqueous composition has a pD of less than about 4.5. In some embodiments, the ophthalmic aqueous composition has a pD of less than about 4.4. In some embodiments, the ophthalmic aqueous composition has a pD of less than about 4.3.In some embodiments, the pD is the pD of the ophthalmic aqueous composition after extended period of time under storage condition.

In some embodiments, the pD of the ophthalmic aqueous composition described herein is associated with the stability of the ophthalmic aqueous composition. In some embodiments, a stable composition comprises a pD of between about 4.5 and about 7.8, about 5 and about 7.5, or about 5.5 and about 7. In some embodiments, a stable composition comprises a pD of less than about 7.5. In some embodiments, a stable composition comprises a pD of less than about 7.4. In some embodiments, a stable composition comprises a pD of less than about 7.3. In some embodiments, a stable composition comprises a pD of less than about 7.2. In some embodiments, a stable composition comprises a pD of less than about 7.1. In some embodiments, a stable composition comprises a pD of less than about 7. In some embodiments, a stable composition comprises a pD of less than about 6.9. In some embodiments, a stable composition comprises a pD of less than about 6.8. In some embodiments, a stable composition comprises a pD of less than about 6.7. In some embodiments, a stable composition comprises a pD of less than about 6.6. In some embodiments, a stable composition comprises a pD of less than about 6.5. In some embodiments, a stable composition comprises a pD of less than about 6.4. In some embodiments, a stable composition comprises a pD of less than about 6.3. In some embodiments, a stable composition comprises a pD of less than about 6.2. In some embodiments, a stable composition comprises a pD of less than about 6.1. In some embodiments, a stable composition comprises a pD of less than about 6. In some embodiments, a stable composition comprises a pD of less than about 5.9. In some embodiments, a stable composition comprises a pD of less than about 5.8. In some embodiments, a stable composition comprises a pD of less than about 5.7. In some embodiments, a stable composition comprises a pD of less than about 5.6. In some embodiments, a stable composition comprises a pD of less than about 5.5. In some embodiments, a stable composition comprises a pD of less than about 5.4. In some embodiments, a stable composition comprises a pD of less than about 5.3. In some embodiments, a stable composition comprises a pD of less than about 5.2. In some embodiments, a stable composition comprises a pD of less than about 5.1. In some embodiments, a stable composition comprises a pD of less than about 5. In some embodiments, a stable composition comprises a pD of less than about 4.9. In some embodiments, a stable composition comprises a pD of less than about 4.8. In some embodiments, a stable composition comprises a pD of less than about 4.7. In some embodiments, a stable composition comprises a pD of less than about 4.6. In some embodiments, a stable composition comprises a pD of less than about 4.5. In some embodiments, a stable composition comprises a pD of less than about 4.4. In some embodiments, a stable composition comprises a pD of less than about 4.3.

In some embodiments, the D₂O aqueous system stabilizes a muscarinic antagonist (e.g., atropine). In some embodiments, this is due to a lower concentration of the reactive species (e.g., - OD) in the D₂O aqueous system compared to the concentration of the reactive species (e.g., -OH) in an equivalent H₂O aqueous system. In some instances, the concentration of the reactive species (e.g., - OD) in the D₂O aqueous system is about one third less than the concentration of the reactive species (e.g., -OH) in the equivalent H₂O aqueous system. In some cases, this is due to a lower or smaller dissociation constant of D₂O than H₂O. For example, the Kₐ(H₂O) is 1x10⁻¹⁴, whereas the Kₐ(D₂O) is 1x10⁻¹⁵. As such, D₂O is a weaker acid than H₂O. In some cases, base catalyzed hydrolysis leads to the presence of tropine degradant from atropine. In some cases, with a lower concentration of the reactive species that causes tropine degradant formation, atropine solution is more stable in a D₂O aqueous system than compared to an equivalent H₂O aqueous system. In some embodiments, the ophthalmic composition formulated with deuterated water allows for a more stable ophthalmic composition relative to the ophthalmic composition formulated with H₂O.

In some embodiments, the presence of deuterated water shifts the pKa of the buffer. In some embodiments, the presence of deuterated water allows for the ophthalmic composition to simulate the stability of a lower pH system. In some instances, the buffer capacity of the ophthalmic composition is lowered, thereby allowing a faster shift in pH. In some instances, the lowered buffering capacity of the ophthalmic composition when administered into the eye allows the ophthalmic composition to reach physiological pH at a faster rate than compared to an ophthalmic composition formulated in H₂O. In some instances, the ophthalmic composition formulated with deuterated water allows for a lower tear production, or less tear reflex in the eye, in comparison with an ophthalmic composition formulated with H₂O.

In some embodiments, the ophthalmic gel, or the also-described ophthalmic ointment, composition described herein has a pD of about 4, about 4.1, about 4.2, about 4.3, about 4.4, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, or about 7.9.

In some embodiments, the pD of the ophthalmic aqueous or gel composition, or the also-described ophthalmic ointment composition, described herein is suitable for sterilization (e.g., by filtration or aseptic mixing or heat treatment and/or autoclaving (e.g., terminal sterilization)) of ophthalmic formulations described herein. As used in in the present disclosure, the term "aqueous composition" includes compositions that are based on D₂O.

In some embodiments, the pharmaceutical formulations described herein are stable with respect to pD over a period of any of at least about 1 day, at least about 2 days, at least about 3 days, at least about 4 days, at least about 5 days, at least about 6 days, at least about 1 week, at least about 2 weeks, at least about 3 weeks, at least about 4 weeks, at least about 5 weeks, at least about 6 weeks, at least about 7 weeks, at least about 8 weeks, at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 18 months, at least about 24 months, at least about 3 years, at least about 4 years, at least about 5 years, at least about 6 years, at least about 7 years, at least about 8 years, at least about 9 years, at least about 10 years, or more. In other embodiments, the formulations described herein are stable with respect to pD over a period of at least about 1 week. In other embodiments, the formulations described herein are stable with respect to pD over a period of at least about 2 weeks. In other embodiments, the formulations described herein are stable with respect to pD over a period of at least about 3 weeks. In other embodiments, the formulations described herein are stable with respect to pD over a period of at least about 1 month. Also described herein are formulations that are stable with respect to pD over a period of at least about 2 months, at least about 3 months, at least about 4 months, at least about 5 months, at least about 6 months, at least about 12 months, at least about 18 months, at least about 2 years, or more.

### Aqueous Solution Dose-To-Dose Uniformity

Typical ophthalmic aqueous solutions are packaged in eye drop bottles and administered as drops. For example, a single administration (i.e. a single dose) of an ophthalmic aqueous solution includes a single drop, two drops, three drops or more into the eyes of the patient. In some embodiments, one dose of the ophthalmic aqueous solution described herein is one drop of the aqueous solution composition from the eye drop bottle.

In some cases, described herein include ophthalmic aqueous compositions which provide a dose-to-dose uniform concentrations. In some instances, the dose-to-dose uniform concentration does not present significant variations of drug content from one dose to another. In some instances, the dose-to-dose uniform concentration does provide consistent drug content from one dose to another.

In some embodiments, the composition has a dose-to-dose muscarinic antagonist or ophthalmic agent concentration variation of less than 50%. In some embodiments, the composition has a dose-to-dose muscarinic antagonist or ophthalmic agent concentration variation of less than 40%. In some embodiments, the composition has a dose-to-dose muscarinic antagonist or ophthalmic agent concentration variation of less than 30%. In some embodiments, the composition has a dose-to-dose muscarinic antagonist or ophthalmic agent concentration variation of less than 20%. In some embodiments, the composition has a dose-to-dose muscarinic antagonist or ophthalmic agent concentration variation of less than 10%. In some embodiments, the composition has a dose-to-dose muscarinic antagonist or ophthalmic agent concentration variation of less than 5%.

In some embodiments, the dose-to-dose muscarinic antagonist or ophthalmic agent concentration variation is based on 10 consecutive doses. In some embodiments, the dose-to-dose muscarinic antagonist or ophthalmic agent concentration variation is based on 8 consecutive doses. In some embodiments, the dose-to-dose muscarinic antagonist or ophthalmic agent concentration variation is based on 5 consecutive doses. In some embodiments, the dose-to-dose muscarinic antagonist or ophthalmic agent concentration variation is based on 3 consecutive doses. In some embodiments, the dose-to-dose muscarinic antagonist or ophthalmic agent concentration variation is based on 2 consecutive doses.

A nonsettling formulation should not require shaking to disperse drug uniformly. A "no-shake" formulation is potentially advantageous over formulations that require shaking for the simple reason that patients' shaking behavior is a major source of variability in the amount of drug dosed. It has been reported that patients often times do not or forget to shake their ophthalmic compositions that requires shaking before administering a dose, despite the instructions to shake that were clearly marked on the label. On the other hand, even for those patients who do shake the product, it is normally not possible to determine whether the shaking is adequate in intensity and/or duration to render the product uniform. In some embodiments, the ophthalmic gel compositions and ophthalmic ointment compositions described herein are "no-shake" formulations that maintained the dose-to-dose uniformity described herein.

To evaluate the dose-to-dose uniformity, drop bottles or tubes containing the ophthalmic aqueous compositions, the ophthalmic gel compositions, or ophthalmic ointment compositions are stored upright for a minimum of 12 hours prior to the start of the test. To simulate the recommended dosing of these products, predetermined number of drops or strips are dispensed from each commercial bottles or tubes at predetermined time intervals for an extended period of time or until no product was left in the bottle or tube. All drops and strips are dispensed into tared glass vials, capped, and stored at room temperature until analysis. Concentrations of a muscarinic antagonist such as atropine in the expressed drops were determined using a reverse-phase HPLC method.

### Aqueous Solution Viscosity

In some embodiments, the composition has a Brookfield RVDV viscosity of from about 10 to about 50,000 cps at about 20°C and sheer rate of 1s⁻¹. The unit "cps" or "centipoise" corresponds to the unit mPa·s. In some embodiments, the composition has a Brookfield RVDV viscosity of from about 100 to about 40,000 cps at about 20°C and sheer rate of 1s⁻¹. In some embodiments, the composition has a Brookfield RVDV viscosity of from about 500 to about 30,000 cps at about 20°C and sheer rate of 1s⁻¹. In some embodiments, the composition has a Brookfield RVDV viscosity of from about 1000 to about 20,000 cps at about 20°C and sheer rate of 1s⁻¹. In some embodiments, the composition has a Brookfield RVDV viscosity of from about 2000 to about 10,000 cps at about 20°C and sheer rate of 1s⁻¹. In some embodiments, the composition has a Brookfield RVDV viscosity of from about 4000 to about 8000 cps at about 20°C and sheer rate of 1s⁻¹.

In some embodiments, the ophthalmic aqueous formulation contains a viscosity enhancing agent sufficient to provide a viscosity of between about 500 and 50,000 centipoise, between about 750 and 50,000 centipoise; between about 1000 and 50,000 centipoise; between about 1000 and 40,000 centipoise; between about 2000 and 30,000 centipoise; between about 3000 and 20,000 centipoise; between about 4000 and 10,000 centipoise, or between about 5000 and 8000 centipoise.

In some embodiments, the compositions described herein are low viscosity compositions at body temperature. In some embodiments, low viscosity compositions contain from about 1% to about 10% of a viscosity enhancing agent (e.g., gelling components such as polyoxyethylene-polyoxypropylene copolymers). In some embodiments, low viscosity compositions contain from about 2% to about 10% of a viscosity enhancing agent (e.g., gelling components such as polyoxyethylene-polyoxypropylene copolymers). In some embodiments, low viscosity compositions contain from about 5% to about 10% of a viscosity enhancing agent (e.g., gelling components such as polyoxyethylene-polyoxypropylene copolymers). In some embodiments, low viscosity compositions are substantially free of a viscosity enhancing agent (e.g., gelling components such as polyoxyethylene-polyoxypropylene copolymers). In some embodiments, a low viscosity muscarinic antagonist/ophthalmic agent composition described herein provides an apparent viscosity of from about 100 cP to about 10,000 cP. In some embodiments, a low viscosity muscarinic antagonist/ophthalmic agent composition described herein provides an apparent viscosity of from about 500 cP to about 10,000 cP. In some embodiments, a low viscosity muscarinic antagonist/ophthalmic agent composition described herein provides an apparent viscosity of from about 1000 cP to about 10,000 cP.

### Osmolarity

In some embodiments, a composition disclosed herein is formulated in order to not disrupt the ionic balance of the eye. In some embodiments, a composition disclosed herein has an ionic balance that is the same as or substantially the same as the eye. In some embodiments, a composition disclosed herein does not does not disrupt the ionic balance of the eye.

As used herein, "practical osmolarity/osmolality" or "deliverable osmolarity/osmolality" means the osmolarity/osmolality of a composition as determined by measuring the osmolarity/osmolality of the muscarinic antagonist or ophthalmic agent and all excipients except the gelling and/or the thickening agent (e.g., polyoxyethylene-polyoxypropylene copolymers, carboxymethylcellulose or the like). The practical osmolarity of a composition disclosed herein is measured by a suitable method, e.g., a freezing point depression method as described in Viegas et. al., Int. J. Pharm., 1998, 160, 157-162. In some instances, the practical osmolarity of a composition disclosed herein is measured by vapor pressure osmometry (e.g., vapor pressure depression method) that allows for determination of the osmolarity of a composition at higher temperatures. In some instances, vapor pressure depression method allows for determination of the osmolarity of a composition comprising a gelling agent (e.g., a thermoreversible polymer) at a higher temperature wherein the gelling agent is in the form of a gel.

In some embodiments, the osmolarity at a target site of action (e.g., the eye) is about the same as the delivered osmolarity of a composition described herein. In some embodiments, a composition described herein has a deliverable osmolarity of about 150 mOsm/L to about 500 mOsm/L, about 250 mOsm/L to about 500 mOsm/L, about 250 mOsm/L to about 350 mOsm/L, about 280 mOsm/L to about 370 mOsm/L or about 250 mOsm/L to about 320 mOsm/L.

The practical osmolality of an ophthalmic composition disclosed herein is from about 100 mOsm/kg to about 1000 mOsm/kg, from about 200 mOsm/kg to about 800 mOsm/kg, from about 250 mOsm/kg to about 500 mOsm/kg, or from about 250 mOsm/kg to about 320 mOsm/kg, or from about 250 mOsm/kg to about 350 mOsm/kg or from about 280 mOsm/kg to about 320 mOsm/kg. In some embodiments, a composition described herein has a practical osmolarity of about 100 mOsm/L to about 1000 mOsm/L, about 200 mOsm/L to about 800 mOsm/L, about 250 mOsm/L to about 500 mOsm/L, about 250 mOsm/L to about 350 mOsm/L, about 250 mOsm/L to about 320 mOsm/L, or about 280 mOsm/L to about 320 mOsm/L.

In some embodiments, suitable tonicity adjusting agents include, but are not limited to any pharmaceutically acceptable sugar, salt or any combinations or mixtures thereof, such as, but not limited to dextrose, glycerin, mannitol, sorbitol, sodium chloride, and other electrolytes. In some instances, the tonicity adjusting agent is selected from sodium chloride, sodium nitrate, sodium sulfate, sodium bisulfate, potassium chloride, calcium chloride, magnesium chloride, zinc chloride, potassium acetate, sodium acetate, sodium bicarbonate, sodium carbonate, sodium thiosulfate, magnesium sulfate, disodium hydrogen phosphate, sodium dihydrogen phosphate, potassium dihydrogen phosphate, dextrose, mannitol, sorbitol, dextrose, sucrose, urea, propylene glycol, glycerin, or a combination thereof.

In some embodiment, the ophthalmic compositions described herein include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

### Sterility

In some embodiments, the compositions are sterilized. Included within the embodiments disclosed herein are means and processes for sterilization of a pharmaceutical composition disclosed herein for use in humans. The goal is to provide a safe pharmaceutical product, relatively free of infection causing micro-organisms. The U. S. Food and Drug Administration has provided regulatory guidance in the publication "Guidance for Industry: Sterile Drug Products Produced by Aseptic Processing" available at: http://www.fda.gov/cder/guidance/5882fnl.htm.

As used herein, sterilization means a process used to destroy or remove microorganisms that are present in a product or packaging. Any suitable method available for sterilization of objects and compositions is used. Available methods for the inactivation of microorganisms include, but are not limited to, the application of extreme heat, lethal chemicals, or gamma radiation. In some embodiments, a process for the preparation of an ophthalmic formulation comprises subjecting the formulation to a sterilization method selected from heat sterilization, chemical sterilization, radiation sterilization or filtration sterilization. The method used depends largely upon the nature of the device or composition to be sterilized. Detailed descriptions of many methods of sterilization are given in Chapter 40 of Remington: The Science and Practice of Pharmacy published by Lippincott, Williams & Wilkins.

### Filtration

Filtration sterilization is a method used to remove but not destroy microorganisms from solutions. Membrane filters are used to filter heat-sensitive solutions. Such filters are thin, strong, homogenous polymers of mixed cellulosic esters (MCE), polyvinylidene fluoride (PVF; also known as PVDF), or polytetrafluoroethylene (PTFE) and have pore sizes ranging from 0.1 to 0.22 µm. Solutions of various characteristics are optionally filtered using different filter membranes. For example, PVF and PTFE membranes are well suited to filtering organic solvents while aqueous solutions are filtered through PVF or MCE membranes. Filter apparatus are available for use on many scales ranging from the single point-of-use disposable filter attached to a syringe up to commercial scale filters for use in manufacturing plants. The membrane filters are sterilized by autoclave or chemical sterilization. Validation of membrane filtration systems is performed following standardized protocols (Microbiological Evaluation of Filters for Sterilizing Liquids, Vol 4, No. 3. Washington, D.C: Health Industry Manufacturers Association, 1981) and involve challenging the membrane filter with a known quantity (ca. 10^{7/}cm²) of unusually small microorganisms, such as Brevundimonas diminuta (ATCC 19146).

Pharmaceutical compositions are optionally sterilized by passing through membrane filters. Formulations comprising nanoparticles (U.S. Pat No. 6,139,870) or multilamellar vesicles (Richard et al., International Journal of Pharmaceutics (2006), 312(1-2):144-50) are amenable to sterilization by filtration through 0.22 µm filters without destroying their organized structure.

In some embodiments, the methods disclosed herein comprise sterilizing the formulation (or components thereof) by means of filtration sterilization. In ophthalmic gel compositions that includes thermosetting polymers, filtration is carried out below (e.g. about 5°C) the gel temperature (Tgel) of a formulation described herein and with viscosity that allows for filtration in a reasonable time using a peristaltic pump (e.g. below a theoretical value of 100cP ).

Accordingly, provided herein are methods for sterilization of ophthalmic formulations that prevent degradation of polymeric components (e.g., thermosetting and/or other viscosity enhancing agents) and/or the muscarinic antagonist or ophthalmic agent during the process of sterilization. In some embodiments, degradation of the muscarinic antagonist such as atropine or atropine sulfate or the ophthalmic agent is reduced or eliminated through the use of specific pD ranges for buffer components and specific proportions of viscosity enhancing agents in the formulations. In some embodiments, the choice of an appropriate viscosity enhancing agents or thermosetting polymer allows for sterilization of formulations described herein by filtration. In some embodiments, the use of an appropriate thermosetting polymer or other viscosity enhancing agents in combination with a specific pD range for the formulation allows for high temperature sterilization of formulations described with substantially no degradation of the therapeutic agent or the polymeric excipients. An advantage of the methods of sterilization provided herein is that, in certain instances, the formulations are subjected to terminal sterilization via autoclaving without any loss of the muscarinic antagonist or ophthalmic agent and/or excipients and/or viscosity enhancing agents during the sterilization step and are rendered substantially free of microbes and/or pyrogens.

### Radiation Sterilization

One advantage of radiation sterilization is the ability to sterilize many types of products without heat degradation or other damage. The radiation commonly employed is beta radiation or alternatively, gamma radiation from a ⁶⁰Co source. The penetrating ability of gamma radiation allows its use in the sterilization of many product types, including solutions, compositions and heterogeneous mixtures. The germicidal effects of irradiation arise from the interaction of gamma radiation with biological macromolecules. This interaction generates charged species and free-radicals. Subsequent chemical reactions, such as rearrangements and cross-linking processes, result in the loss of normal function for these biological macromolecules. The formulations described herein are also optionally sterilized using beta irradiation.

### Sterilization by Heat

Many methods are available for sterilization by the application of high heat. One method is through the use of a saturated steam autoclave. In this method, saturated steam at a temperature of at least 121 °C is allowed to contact the object to be sterilized. The transfer of heat is either directly to the microorganism, in the case of an object to be sterilized, or indirectly to the microorganism by heating the bulk of an aqueous solution to be sterilized. This method is widely practiced as it allows flexibility, safety and economy in the sterilization process.

### Microorganisms

In some embodiments, the compositions are substantially free of microorganisms. Acceptable bioburden or sterility levels are based on applicable standards that define therapeutically acceptable compositions, including but not limited to United States Pharmacopeia Chapters <1111> et seq. For example, acceptable sterility (e.g., bioburden) levels include about 10 colony forming units (cfu) per gram of formulation, about 50 cfu per gram of formulation, about 100 cfu per gram of formulation, about 500 cfu per gram of formulation or about 1000 cfu per gram of formulation. In some embodiments, acceptable bioburden levels or sterility for formulations include less than 10 cfu/mL, less than 50 cfu/mL, less than 500 cfu/mL or less than 1000 cfu/mL microbial agents. In addition, acceptable bioburden levels or sterility include the exclusion of specified objectionable microbiological agents. By way of example, specified objectionable microbiological agents include but are not limited to *Escherichia coli (E. coli), Salmonella sp., Pseudomonas aeruginosa (P. aeruginosa)* and/or other specific microbial agents.

An important component of the sterility assurance quality control, quality assurance and validation process is the method of sterility testing. Sterility testing, by way of example only, is performed by two methods. The first is direct inoculation wherein a sample of the composition to be tested is added to growth medium and incubated for a period of time up to 21 days. Turbidity of the growth medium indicates contamination. Drawbacks to this method include the small sampling size of bulk materials which reduces sensitivity, and detection of microorganism growth based on a visual observation. An alternative method is membrane filtration sterility testing. In this method, a volume of product is passed through a small membrane filter paper. The filter paper is then placed into media to promote the growth of microorganisms. This method has the advantage of greater sensitivity as the entire bulk product is sampled. The commercially available Millipore Steritest sterility testing system is optionally used for determinations by membrane filtration sterility testing. For the filtration testing of creams or ointments Steritest filter system No. TLHVSL210 are used. For the filtration testing of emulsions or viscous products Steritest filter system No. TLAREM210 or TDAREM210 are used. For the filtration testing of pre-filled syringes Steritest filter system No. TTHASY210 are used. For the filtration testing of material dispensed as an aerosol or foam Steritest filter system No. TTHVA210 are used. For the filtration testing of soluble powders in ampoules or vials Steritest filter system No. TTHADA210 or TTHADV210 are used.

Testing for *E. coli and Salmonella* includes the use of lactose broths incubated at 30 - 35 °C for 24-72 hours, incubation in MacConkey and/or EMB agars for 18-24 hours, and/or the use of Rappaport medium. Testing for the detection of *P. aeruginosa* includes the use of NAC agar. United States Pharmacopeia Chapter <62> further enumerates testing procedures for specified objectionable microorganisms.

In certain embodiments, the ophthalmic formulation described herein has less than about 60 colony forming units (CFU), less than about 50 colony forming units, less than about 40 colony forming units, or less than about 30 colony forming units of microbial agents per gram of formulation. In certain embodiments, the ophthalmic formulations described herein are formulated to be isotonic with the eye.

### Endotoxins

An additional aspect of the sterilization process is the removal of by-products from the killing of microorganisms (hereinafter, "Product"). The process of depyrogenation removes pyrogens from the sample. Pyrogens are endotoxins or exotoxins which induce an immune response. An example of an endotoxin is the lipopolysaccharide (LPS) molecule found in the cell wall of gram-negative bacteria. While sterilization procedures such as autoclaving or treatment with ethylene oxide kill the bacteria, the LPS residue induces a proinflammatory immune response, such as septic shock. Because the molecular size of endotoxins varies widely, the presence of endotoxins is expressed in "endotoxin units" (EU). One EU is equivalent to 100 picograms of *E. coli* LPS. In some cases, humans develop a response to as little as 5 EU/kg of body weight. The bioburden (e.g., microbial limit) and/or sterility (e.g., endotoxin level) is expressed in any units as recognized in the art. In certain embodiments, ophthalmic compositions described herein contain lower endotoxin levels (e.g. < 4 EU/kg of body weight of a subject) when compared to conventionally acceptable endotoxin levels (e.g., 5 EU/kg of body weight of a subject). In some embodiments, the ophthalmic formulation has less than about 5 EU/kg of body weight of a subject. In other embodiments, the ophthalmic formulation has less than about 4 EU/kg of body weight of a subject. In additional embodiments, the ophthalmic formulation has less than about 3 EU/kg of body weight of a subject. In additional embodiments, the ophthalmic formulation has less than about 2 EU/kg of body weight of a subject.

In some embodiments, the ophthalmic formulation has less than about 5 EU/kg of formulation. In other embodiments, the ophthalmic formulation has less than about 4 EU/kg of formulation. In additional embodiments, the ophthalmic formulation has less than about 3 EU/kg of formulation. In some embodiments, the ophthalmic formulation has less than about 5 EU/kg Product. In other embodiments, the ophthalmic formulation has less than about 1 EU/kg Product. In additional embodiments, the ophthalmic formulation has less than about 0.2 EU/kg Product. In some embodiments, the ophthalmic formulation has less than about 5 EU/g of unit or Product. In other embodiments, the ophthalmic formulation has less than about 4 EU/ g of unit or Product. In additional embodiments, the ophthalmic formulation has less than about 3 EU/g of unit or Product. In some embodiments, the ophthalmic formulation has less than about 5 EU/mg of unit or Product. In other embodiments, the ophthalmic formulation has less than about 4 EU/ mg of unit or Product. In additional embodiments, the ophthalmic formulation has less than about 3 EU/mg of unit or Product. In certain embodiments, ophthalmic formulations described herein contain from about 1 to about 5 EU/mL of formulation. In certain embodiments, ophthalmic formulations described herein contain from about 2 to about 5 EU/mL of formulation, from about 3 to about 5 EU/mL of formulation, or from about 4 to about 5 EU/mL of formulation.

In certain embodiments, ophthalmic compositions described herein contain lower endotoxin levels (e.g. < 0.5 EU/mL of formulation) when compared to conventionally acceptable endotoxin levels (e.g., 0.5 EU/mL of formulation). In some embodiments, the ophthalmic formulation has less than about 0.5 EU/mL of formulation. In other embodiments, the ophthalmic formulation has less than about 0.4 EU/mL of formulation. In additional embodiments, the ophthalmic formulation has less than about 0.2 EU/mL of formulation.

Pyrogen detection, by way of example only, is performed by several methods. Suitable tests for sterility include tests described in United States Pharmacopoeia (USP) <71> Sterility Tests (23rd edition, 1995). The rabbit pyrogen test and the Limulus amebocyte lysate test are both specified in the United States Pharmacopeia Chapters <85> and <151> (USP23/NF 18, Biological Tests, The United States Pharmacopeial Convention, Rockville, MD, 1995). Alternative pyrogen assays have been developed based upon the monocyte activation-cytokine assay. Uniform cell lines suitable for quality control applications have been developed and have demonstrated the ability to detect pyrogenicity in samples that have passed the rabbit pyrogen test and the Limulus amebocyte lysate test (Taktak et al, J. Pharm. Pharmacol. (1990), 43:578-82). In an additional embodiment, the ophthalmic formulation is subject to depyrogenation. In a further embodiment, the process for the manufacture of the ophthalmic formulation comprises testing the formulation for pyrogenicity. In certain embodiments, the formulations described herein are substantially free of pyrogens.

### Muscarinic Antagonist/Ophthalmic Agent -Mucus Penetrating Particle (MPP) Composition

Mucus-penetrating particles (MPPs) are particles that rapidly traverse mucus (e.g. human mucus). In some cases, MPPs comprise of a nanoparticle with a particle size of between about 200nm and 500nm. In some instances, the nanoparticle is further coated with a mucus penetrating agent. In some instances, a composition described herein (e.g., a muscarinic antagonist or an ophthalmic agent) is formulated with MPPs for mucus penetration. In some instances, a composition described herein is formulated with MPPs for mucus penetration. In some instances, a muscarinic antagonist composition described herein is formulated with MPPs for mucus penetration. In some instances, a muscarinic antagonist comprises atropine, atropine sulfate, noratropine, atropine-N-oxide, tropine, tropic acid, atropine methonitrate, diphenhydramine, dimenhydrinate, dicyclomine, flavoxate, oxybutynin, tiotropium, hyoscine, scopolamine (L-hyoscine), hydroxyzine, ipratropium, tropicamide, cyclopentolate, pirenzapine, homatropine, solifenacin, darifenacin, benzatropine, mebeverine, procyclidine, aclidinium bromide, trihexyphenidyl/benzhexol, or tolterodine. In some instances, a muscarinic antagonist is atropine or its pharmaceutically acceptable salt thereof. In some instances, a muscarinic antagonist is atropine sulfate. In some instances, an atropine composition described herein is formulated with MPPs for mucus penetration. In some instances, an atropine sulfate composition described herein is formulated with MPPs for mucus penetration. In a non-limiting example, the MMPs for use in the disclosed composition is obtained from Kala Pharmaceuticals, Inc. (100 Beaver Street #201, Waltham, MA 02453).

In some instances, an ophthalmic agent described herein is formulated with MPPs for mucus penetration. In some cases, the ophthalmic agent comprises aflibercept, ranibizumab, pegaptanib, cyclopentolate, phenylephrine, homatropine, scopolamine, cyclopentolate/phenylephrine, phenylephrine/scopolamine, tropicamide, ketorolac/phenylephrine, hydroxyamphetamine/tropicamide, cysteamine, ocriplasmin, mitomycin, dapiprazole, lidocaine, proparacaine, tetracaine, benoxinate, azithromycin, bacitracin, besifloxacin, boric acid, chloramphenicol, ciprofloxacin, erythromycin, ganciclovir, gatifloxacin, gentamicin, idoxuridine, levofloxacin, moxifloxacin, natamycin, norfloxacin, ofloxacin, bacitracin/polymyxin b, tobramycin, polymyxin b/trimethoprim, povidone iodine, trifluridine, gramicidin/neomycin/polymyxin b, sulfacetamide sodium, sulfisoxazole, bacitracin/neomycin/polymyxin b, oxytetracycline/polymyxin b, phenylephrine/sulfacetamide sodium, vidarabine, bromfenac, nepafenac, ketorolac, cyclosporine, flurbiprofen, suprofen, diclofenac, alcaftadine, azelastine, bepotastine, cromolyn, emedastine, epinastine, ketotifen, levocabastine, lodoxamide, nedocromil, naphazoline, naphazoline/pheniramine, naphazoline/zinc sulfate, olopatadine, oxymetazoline, pemirolast, phenylephrine, phenylephrine/zinc sulfate, tetrahydrozoline, tetrahydrozoline/zinc sulfate, fluorescein, fluorescein/proparacaine, benoxinate/fluorescein, indocyanine green, trypan blue, acetylcholine, apraclonidine, betaxolol, bimatoprost,brimonidine, brinzolamide, brimonidine/brinzolamide, carbachol, carteolol, demecarium bromide, dipivefrin, dorzolamide, dorzolamide/timolol, echothiophate iodide, epinephrine, epinephrine/pilocarpine, latanoprost, levobunolol, levobetaxolol, metipranolol, physostigmine, pilocarpine, tafluprost, timolol, travoprost, unoprostone, artificial tear, dexamethasone, difluprednate, fluocinolone, fluorometholone, loteprednol, medrysone, prednisolone, rimexolone, triamcinolone, fluorometholone/sulfacetamide sodium, dexamethasone/neomycin, dexamethasone/tobramycin, dexamethasone/neomycin/polymyxin b, loteprednol/tobramycin, prednisolone/sulfacetamide sodium, bacitracin/hydrocortisone/neomycin/polymyxin b, hydrocortisone/neomycin/polymyxin b, chloramphenicol/hydrocortisone/polymyxin b, neomycin/polymyxin b/prednisolone, gentamicin/prednisolone, ketorolac/phenylephrine, diphenhydramine, dimenhydrinate, dicyclomine, flavoxate, oxybutynin, tiotropium, hyoscine, scopolamine (L-hyoscine), hydroxyzine, ipratropium, pirenzapine, solifenacin, darifenacin, benzatropine, mebeverine, procyclidine, aclidinium bromide, trihexyphenidyl/benzhexol, tolterodine, or any combinations thereof. In a non-limiting example, the MMPs for use in the disclosed composition is obtained from Kala Pharmaceuticals, Inc. (100 Beaver Street #201, Waltham, MA 02453).

In some embodiments, the nanoparticle comprises any suitable material, such as an organic material, an inorganic material, a polymer, or combinations thereof. In some instances, the nanoparticle comprises an inorganic material, such as for example, a metal (e.g., Ag, Au, Pt, Fe, Cr, Co, Ni, Cu, Zn, and other transition metals), a semiconductor (e.g., silicon, silicon compounds and alloys, cadmium selenide, cadmium sulfide, indium arsenide, and indium phosphide), or an insulator (e.g. , ceramics such as silicon oxide). In some instances, the nanoparticle comprises organic materials such as a synthetic polymer and/or a natural polymer. Examples of synthetic polymers include nondegradable polymers such as polymethacrylate and degradable polymers such as polylactic acid, polyglycolic acid and copolymers thereof. Examples of natural polymers include hyaluronic acid, chitosan, and collagen.

In some embodiments, the nanoparticle is coated with a mucus penetrating agent. In some instances, the mucus penetrating agent comprises any suitable material, such as a hydrophobic material, a hydrophilic material, and/or an amphiphilic material. In some instances, the mucus penetrating agent is a polymer. In some instances, the polymer a synthetic polymer (i.e., a polymer not produced in nature). In other embodiments, the polymer is a natural polymer (e.g. , a protein, polysaccharide, rubber). In certain embodiments, the polymer is a surface active polymer. In certain embodiments, the polymer is a non-ionic polymer. In certain embodiments, the polymer is a non-ionic block copolymer. In some embodiments, the polymer is a diblock copolymer, a triblock copolymer, e.g., e.g., where one block is a hydrophobic polymer and another block is a hydrophilic polymer. In some embodiments, the polymer is charged or uncharged.

Additional examples of suitable polymers include, but are not limited to, polyamines, polyethers, polyamides, polyesters, polycarbamates, polyureas, polycarbonates, polystyrenes, polyimides, polysulfones, polyurethanes, polyacetylenes, polyethylenes, polyethyeneimines, polyisocyanates, polyacrylates, polymethacrylates, polyacrylonitriles, and polyarylates. Non-limiting examples of specific polymers include poly(caprolactone) (PCL), ethylene vinyl acetate polymer (EVA), poly(lactic acid) (PLA), poly(L-lactic acid) (PLLA), poly(glycolic acid) (PGA), poly(lactic acid-co-glycolic acid) (PLGA), poly(L-lactic acid-co-glycolic acid) (PLLGA), poly(D,L-lactide) (PDLA), poly(L- lactide) (PLLA), poly(D,L-lactide-co-caprolactone), poly(D,L-lactide-co-caprolactone-co-glycolide), poly(D,L-lactide-co-PEO-co-D,L-lactide), poly(D,L-lactide-co-PPO-coD,L-lactide), polyalkyl cyanoacrylate, polyurethane, poly-L-lysine (PLL), hydroxypropyl methacrylate (HPMA), poly(ethylene glycol), poly-L-glutamic acid, poly(hydroxy acids), polyanhydrides, polyorthoesters, poly(ester amides), polyamides, poly(ester ethers), polycarbonates, polyalkylenes such as polyethylene and polypropylene, polyalkylene glycols such as poly(ethylene glycol) (PEG), polyalkylene oxides (PEO), polyalkylene terephthalates such as poly(ethylene terephthalate), polyvinyl alcohols (PVA), polyvinyl ethers, polyvinyl esters such as poly(vinyl acetate), polyvinyl halides such as poly(vinyl chloride) (PVC), polyvinylpyrrolidone, polysiloxanes, polystyrene (PS), polyurethanes, derivatized celluloses such as alkyl celluloses, hydroxyalkyl celluloses, cellulose ethers, cellulose esters, nitro celluloses, hydroxypropylcellulose, carboxymethylcellulose, polymers of acrylic acids, such as poly(methyl(meth)acrylate) (PMMA), poly(ethyl(meth)acrylate), poly(butyl(meth)acrylate), poly(isobutyl(meth)acrylate), poly(hexyl(meth)acrylate), poly(isodecyl(meth)acrylate), poly(lauryl(meth)acrylate), poly(phenyl(meth)acrylate), poly(methyl acrylate), poly(isopropyl acrylate), poly(isobutyl acrylate), poly(octadecyl acrylate) (jointly referred to herein as "polyacrylic acids"), and copolymers and mixtures thereof, polydioxanone and its copolymers, polyhydroxyalkanoates, polypropylene fumarate), polyoxymethylene, poloxamers, poly(ortho)esters, poly(butyric acid), poly(valeric acid), poly(lactide-co-caprolactone), and trimethylene carbonate, polyvinylpyrrolidone.

In some cases, the muscarinic antagonist or the ophthalmic agent described herein is present in the MPP formulation at a concentration of between about 0.001 wt% and about 0.05 wt%, between about 0.005% to about 0.050%, between about 0.010% to about 0.050%, between about 0.015% to about 0.050%, between about 0.020% to about 0.050%, between about 0.025% to about 0.050%, between about 0.030% to about 0.050%, between about 0.035% to about 0.050%, between about 0.040% to about 0.050%, or between about 0.045% to about 0.050% of the muscarinic antagonist or ophthalmic agent by weight of the composition. In some instances, additional agents such as buffers, pD adjusting agents, and/or preservatives are formulated in the MPP formulation.

In some instances, muscarinic antagonist/ophthalmic agent-MPP composition is formulated using any suitable method. In some embodiments, a milling process is used to reduce the size of a solid material to form particles in the micrometer to nanometer size range. In some cases, dry and wet milling processes such as jet milling, cryo-milling, ball milling, media milling, and homogenization are known and are used in methods described herein. Generally, in a wet milling process, a suspension of the material to be used as the nanoparticle is mixed with milling media with or without excipients to reduce particle size. Dry milling is a process wherein the material to be used as the nanoparticle is mixed with milling media with or without excipients to reduce particle size. In a cryo-milling process, a suspension of the material to be used as the nanoparticle is mixed with milling media with or without excipients under cooled temperatures.

In some embodiments, any suitable grinding medium is used for milling. In some embodiments, a ceramic and/or polymeric material and/or a metal is used. Examples of suitable materials include zirconium oxide, silicon carbide, silicon oxide, silicon nitride, zirconium silicate, yttrium oxide, glass, alumina, alpha- alumina, aluminum oxide, polystyrene, poly(methyl methacrylate), titanium, or steel. In some cases, a grinding medium has any suitable size. For example, the grinding medium has an average diameter of at least about 0.1 mm, at least about 0.2 mm, at least about 0.5 mm, at least about 0.8 mm, at least about 1 mm, at least about 2 mm, or at least about 5 mm. In some cases, the grinding medium has an average diameter of less than or equal to about 5 mm, less than or equal to about 2 mm, less than or equal to about 1 mm, less than or equal to about 0.8, less than or equal to about 0.5 mm, or less than or equal to about 0.2 mm. Combinations of the above-referenced ranges are also possible (e.g. , an average diameter of at least about 0.5 millimeters and less than or equal to about 1 mm). Other ranges are also possible.

In some embodiments, any suitable solvent are used for milling. In some cases, the choice of solvent is depend on factors such as the solid material (e.g., a muscarinic antagonist such as atropine or an ophthalmic agent) being milled, the particular type of stabilizer/mucus penetrating agent being used (e.g., one that renders the particle mucus penetrating), the grinding material be used, among other factors. In some cases, suitable solvents are ones that do not substantially dissolve the solid material or the grinding material, but dissolve the stabilizer/mucus penetrating agent to a suitable degree. Non-limiting examples of solvents include, but are not limited to, water, buffered solutions, other aqueous solutions, alcohols (e.g. , ethanol, methanol, butanol), and mixtures thereof that optionally include other components such as pharmaceutical excipients, polymers, pharmaceutical agents, salts, preservative agents, viscosity modifiers, tonicity modifier, taste masking agents, antioxidants, pD modifier, and other pharmaceutical excipients. In other embodiments, an organic solvent is used. In some cases, a pharmaceutical agent (e.g. a muscarinic antagonist such as atropine or an ophthalmic agent) has any suitable solubility in these or other solvents, such as a solubility in one or more of the ranges described above for aqueous solubility or for solubility in a coating solution.

In some instances, a MPP is a MPP as described in WO2013/166385. In some instances, a MPP is a MPP as described in Lai et al., "Rapid transport of large polymeric nanoparticles in fresh undiluted human mucus," PNAS 104(5):1482-1487 (2007). In some instances, a muscarinic antagonist/ophthalmic agent-MPP composition is formulated using a method as described in WO2013/166385. In some instances, a muscarinic antagonist/ophthalmic agent-MPP composition is formulated using a method as described in Lai et al., "Rapid transport of large polymeric nanoparticles in fresh undiluted human mucus," PNAS 104(5):1482-1487 (2007). In some instances, the muscarinic antagonist is atropine or atropine sulfate.

### Ophthalmic Gel Composition

Gels have been defined in various ways. For example, the United States Pharmacopoeia defines gels as semisolid systems consisting of either suspensions made up of small inorganic particles or large organic molecules interpenetrated by a liquid. Gels include a single-phase or a two-phase system. A single-phase gel consists of organic macromolecules distributed uniformly throughout a liquid in such a manner that no apparent boundaries exist between the dispersed macromolecules and the liquid. Some single-phase gels are prepared from synthetic macromolecules (e.g., carbomer) or from natural gums, (e.g., tragacanth). In some instances, single-phase gels are generally aqueous, but will also be made using alcohols and oils. Two-phase gels consist of a network of small discrete particles.

In some instances, gels are also classified as being hydrophobic or hydrophilic. In certain instances, the base of a non-limiting example of a hydrophobic gel includes a liquid paraffin with polyethylene or fatty oils gelled with colloidal silica, or aluminum or zinc soaps. In contrast, the base of a non-limiting example of a hydrophilic gel includes water, glycerol, or propylene glycol gelled with a suitable gelling agent (e.g., tragacanth, starch, cellulose derivatives, carboxyvinylpolymers, and magnesium-aluminum silicates). In certain instances, the rheology of the compositions disclosed herein is pseudo plastic, plastic, thixotropic, or dilatant.

In some embodiments, the ophthalmic composition is an ophthalmic gel, and wherein the ophthalmically acceptable carrier comprises water and at least one viscosity-enhancing agent. In some embodiments, the viscosity-enhancing agent is selected from cellulose-based polymers, polyoxyethylene-polyoxypropylene triblock copolymers, dextran-based polymers, polyvinyl alcohol, dextrin, polyvinylpyrrolidone, polyalkylene glycols, chitosan, collagen, gelatin, hyaluronic acid, or combinations thereof.

In some embodiment, the ophthalmic gel composition described herein is a semi-solid or id in a gelled state before it is topically administered (e.g. at room temperature). For example, suitable viscosity-enhancing agents for such gels include by way of example only, gelling agents and suspending agents. In one embodiment, the enhanced viscosity formulation does not include a buffer. In other embodiments, the enhanced viscosity formulation includes a pharmaceutically acceptable buffer. Sodium chloride or other tonicity agents are optionally used to adjust tonicity, if necessary.

By way of example only, the ophthalmically acceptable viscosity agent includes hydroxypropyl methylcellulose, hydroxyethyl cellulose, polyvinylpyrrolidone, carboxymethyl cellulose, polyvinyl alcohol, sodium chondroitin sulfate, sodium hyaluronate. Other viscosity enhancing agents compatible with the targeted ocular site include, but are not limited to, acacia (gum arabic), agar, aluminum magnesium silicate, sodium alginate, sodium stearate, bladderwrack, bentonite, carbomer, carrageenan, Carbopol, xanthan, cellulose, microcrystalline cellulose (MCC), ceratonia, chitin, carboxymethylated chitosan, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthan gum, gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose (HPMC), sodium carboxymethyl-cellulose (CMC), silicon dioxide, polyvinylpyrrolidone (PVP: povidone), Splenda^{®} (dextrose, maltodextrin and sucralose) or combinations thereof. In specific embodiments, the viscosity-enhancing excipient is a combination of MCC and CMC. In another embodiment, the viscosity-enhancing agent is a combination of carboxymethylated chitosan, or chitin, and alginate. The combination of chitin and alginate with the muscarinic antagonists or ophthalmic agents disclosed herein acts as a controlled release formulation, restricting the diffusion of the muscarinic antagonists or ophthalmic agents from the formulation. Moreover, the combination of carboxymethylated chitosan and alginate is optionally used to assist in increasing the permeability of the muscarinic antagonists or ophthalmic agents in the eye.

In some embodiments is an enhanced viscosity formulation, comprising from about 0.1 mM and about 100 mM of a muscarinic antagonist or an ophthalmic agent, a pharmaceutically acceptable viscosity agent, and water for injection, the concentration of the viscosity agent in the water being sufficient to provide an enhanced viscosity formulation with a final viscosity from about 100 to about 100,000 cP. In certain embodiments, the viscosity of the gel is in the range from about 100 to about 50,000 cP, about 100 cP to about 1,000 cP, about 500 cP to about 1500 cP, about 1000 cP to about 3000 cP, about 2000 cP to about 8,000 cP, about 4,000 cP to about 50,000 cP, about 10,000 cP to about 500,000 cP, about 15,000 cP to about 1,000,000 cP (1 cP corresponds to 1 mPa·s). In other embodiments, when an even more viscous medium is desired, the biocompatible gel comprises at least about 35%, at least about 45%, at least about 55%, at least about 65%, at least about 70%, at least about 75%, or even at least about 80% or so by weight of the muscarinic antagonist or ophthalmic agent. In highly concentrated samples, the biocompatible enhanced viscosity formulation comprises at least about 25%, at least about 35%, at least about 45%, at least about 55%, at least about 65%, at least about 75%, at least about 85%, at least about 90% or at least about 95% or more by weight of the muscarinic antagonist or ophthalmic agent.

In one embodiment, the pharmaceutically acceptable enhanced viscosity ophthalmically acceptable formulation comprises at least one muscarinic antagonist or ophthalmic agent and at least one gelling agent. Suitable gelling agents for use in preparation of the gel formulation include, but are not limited to, celluloses, cellulose derivatives, cellulose ethers (e.g., carboxymethylcellulose, ethylcellulose, hydroxyethylcellulose, hydroxymethylcellulose, hydroxypropylmethylcellulose, hydroxypropylcellulose, methylcellulose), guar gum, xanthan gum, locust bean gum, alginates (e.g., alginic acid), silicates, starch, tragacanth, carboxyvinyl polymers, carrageenan, paraffin, petrolatum and any combinations or mixtures thereof. In some other embodiments, hydroxypropylmethylcellulose (Methocel^{®}) is utilized as the gelling agent. In certain embodiments, the viscosity enhancing agents described herein are also utilized as the gelling agent for the gel formulations presented herein.

In some embodiments, the ophthalmic gel composition described herein is an in situ gel formulation. In some instances, the in situ gel formation is based on increased pre-corneal residence time of the ophthalmic composition which improves ocular bioavailability, corneal mucoadhesion, lysosomal interaction and ionic gelation, improved corneal absorption, thermal gelation, or a combination thereof. In some instances, the in situ gel formulation is activated by pH, temperature, ion, UV, or solvent exchange.

In some instances, the ophthalmic gel composition comprises a muscarinic antagonist or an ophthalmic agent and one or more gelling agents. In some instances, the gelling agent includes, but is not limited to, poloxamer (e.g. Poloxamer 407), tetronics, ethyl (hydroxyethyl) cellulose, cellulose acetate phthalate (CAP), carbopol (e.g. Carbopol 1342P NF, Carbopol 980 NF), alginates (e.g. low acetyl gellan gum (Gelrite^{®})), gellan, hyaluronic acid, pluronics (e.g. Pluronic F-127), chitosan, polyvinyl alcohol (PVA), polyvinylpyrrolidone (PVP), dextran, hydroxy propyl methyl cellulose (HPMC), hydroxyethylcellulose (HEC), methylcellulose (MC), thiolated xyloglucan, polymethacrylic acid (PMMA), polyethylene glycol (PEG), pseudolatexes, xyloglucans, or combinations thereof.

In some instances, the in situ gel formation further comprises a permeation enhancer. In some instances, the permeation enhancer includes surfactants (e.g. non-ionic surfactants), benzalkonium chloride, EDTA, surface-active heteroglycosides, calcium chelators, hydroxyl propyl beta cyclodextrin (HP beta CD), bile salts, and the like.

In some embodiments, other gel formulations are useful depending upon the particular muscarinic antagonist or ophthalmic agent, other pharmaceutical agent or excipients/additives used, and as such are considered to fall within the scope of the present disclosure. For example, other commercially-available glycerin-based gels, glycerin-derived compounds, conjugated, or crosslinked gels, matrices, hydrogels, and polymers, as well as gelatins and their derivatives, alginates, and alginate-based gels, and even various native and synthetic hydrogel and hydrogel-derived compounds are all expected to be useful in the muscarinic antagonist or ophthalmic agent formulations described herein. In some embodiments, ophthalmically acceptable gels include, but are not limited to, alginate hydrogels SAF^{®}-Gel (ConvaTec, Princeton, N.J.), Duoderm^{®} Hydroactive Gel (ConvaTec), Nu-gel ^{®}(Johnson & Johnson Medical, Arlington, Tex.); Carrasyn^{®}(V) Acemannan Hydrogel (Carrington Laboratories, Inc., Irving, Tex.); glycerin gels Elta^{®} Hydrogel (Swiss-American Products, Inc., Dallas, Tex.) and K-Y^{®} Sterile (Johnson & Johnson). In further embodiments, biodegradable biocompatible gels also represent compounds present in ophthalmically acceptable formulations disclosed and described herein.

In some embodiments, the viscosity-enhancing agent is a cellulose-based polymer selected from cellulose gum, alkylcellulose, hydroxyl-alkyl cellulose, hydroxyl-alkyl alkylcellulose, carboxyalkyl cellulose, or combinations thereof. In some embodiments, the viscosity-enhancing agent is hydroxyl-alkyl alkylcellulose. In some embodiment, the viscosity-enhancing agent is hydroxypropyl methylcellulose.

In certain embodiments, the enhanced viscosity formulation is characterized by a phase transition between room temperature and body temperature (including an individual with a serious fever, e.g., up to about 42 °C). In some embodiments, the phase transition occurs at 1 °C below body temperature, at 2 °C below body temperature, at 3 °C below body temperature, at 4 °C below body temperature, at 6 °C below body temperature, at 8 °C below body temperature, or at 10 °C below body temperature. In some embodiments, the phase transition occurs at about 15 °C below body temperature, at about 20°C below body temperature or at about 25 °C below body temperature. In specific embodiments, the gelation temperature (Tgel) of a formulation described herein is about 20 °C, about 25 °C, or about 30 °C. In certain embodiments, the gelation temperature (Tgel) of a formulation described herein is about 35 °C, or about 40 °C. Included within the definition of body temperature is the body temperature of a healthy individual, or an unhealthy individual, including an individual with a fever (up to ~42 °C). In some embodiments, the pharmaceutical compositions described herein are liquids at about room temperature and are administered at or about room temperature.

Copolymers polyoxypropylene and polyoxyethylene (e.g. polyoxyethylene-polyoxypropylene triblock copolymers) form thermosetting gels when incorporated into aqueous solutions. These polymers have the ability to change from the liquid state to the gel state at temperatures close to body temperature, therefore allowing useful formulations that are applied to the targeted ocular site. The liquid state-to-gel state phase transition is dependent on the polymer concentration and the ingredients in the solution.

In some embodiments, the amount of thermosetting polymer in any formulation described herein is about 10%, about 15%, about 20%, about 25%, about 30%, about 35% or about 40% of the total weight of the formulation. In some embodiments, the amount of thermosetting polymer in any formulation described herein is about 10%, about 11%, about 12%, about 13%, about 14%, about 15%, about 16%, about 17%, about 18%, about 19%, about 20%, about 21%, about 22%, about 23%, about 24% or about 25% of the total weight of the formulation. In some embodiments, the amount of thermosetting polymer (e.g., Poloxamer 407) in any formulation described herein is about 7.5% of the total weight of the formulation. In some embodiments, the amount of thermosetting polymer (e.g., Poloxamer 407) in any formulation described herein is about 10% of the total weight of the formulation. In some embodiments, the amount of thermosetting polymer (e.g., Poloxamer 407) in any formulation described herein is about 11% of the total weight of the formulation. In some embodiments, the amount of thermosetting polymer (e.g., Poloxamer 407) in any formulation described herein is about 12% of the total weight of the formulation. In some embodiments, the amount of thermosetting polymer (e.g., Poloxamer 407) in any formulation described herein is about 13% of the total weight of the formulation. In some embodiments, the amount of thermosetting polymer (e.g., Poloxamer 407) in any formulation described herein is about 14% of the total weight of the formulation. In some embodiments, the amount of thermosetting polymer (e.g., Poloxamer 407) in any formulation described herein is about 15% of the total weight of the formulation. In some embodiments, the amount of thermosetting polymer (e.g., Poloxamer 407) in any formulation described herein is about 16% of the total weight of the formulation. In some embodiments, the amount of thermosetting polymer (e.g., Poloxamer 407) in any formulation described herein is about 17% of the total weight of the formulation. In some embodiments, the amount of thermosetting polymer (e.g., Poloxamer 407) in any formulation described herein is about 18% of the total weight of the formulation. In some embodiments, the amount of thermosetting polymer (e.g., Poloxamer 407) in any formulation described herein is about 19% of the total weight of the formulation. In some embodiments, the amount of thermosetting polymer (e.g., Poloxamer 407) in any formulation described herein is about 20% of the total weight of the formulation. In some embodiments, the amount of thermosetting polymer (e.g., Poloxamer 407) in any formulation described herein is about 21% of the total weight of the formulation. In some embodiments, the amount of thermosetting polymer (e.g., Poloxamer 407) in any formulation described herein is about 23% of the total weight of the formulation. In some embodiments, the amount of thermosetting polymer (e.g., Poloxamer 407) in any formulation described herein is about 25% of the total weight of the formulation. In some embodiments, the amount of thickening agent (e.g., a gelling agent) in any formulation described herein is about 1%, about 5%, about 10%, or about 15% of the total weight of the formulation. In some embodiments, the amount of thickening agent (e.g., a gelling agent) in any formulation described herein is about 0.5%, about 1%, about 1.5%, about 2%, about 2.5%, about 3%, about 3.5%, about 4%, about 4.5%, or about 5% of the total weight of the formulation.

In an alternative embodiment, the thermogel is a PEG-PLGA-PEG triblock copolymer (Jeong etal, Nature (1997), 388:860-2; Jeong etal, J. Control. Release (2000), 63:155-63; Jeong etal, Adv. Drug Delivery Rev. (2002), 54:37-51). The polymer exhibits sol-gel behavior over a concentration of about 5% w/w to about 40% w/w. Depending on the properties desired, the lactide/glycolide molar ratio in the PLGA copolymer ranges from about 1:1 to about 20:1. The resulting copolymers are soluble in water and form a free-flowing liquid at room temperature, but form a hydrogel at body temperature. A commercially available PEG-PLGA-PEG triblock copolymer is RESOMER RGP t50106 manufactured by Boehringer Ingelheim. This material is composed of a PLGA copolymer of 50:50 poly(DL-lactide-co-glycolide) and is 10% w/w of PEG and has a molecular weight of about 6000.

Additional biodegradable thermoplastic polyesters include AtriGel^{®} (provided by Atrix Laboratories, Inc.) and/or those disclosed, e.g., in U.S. Patent Nos. 5,324,519; 4,938,763; 5,702,716; 5,744,153; and 5,990,194; wherein the suitable biodegradable thermoplastic polyester is disclosed as a thermoplastic polymer. Examples of suitable biodegradable thermoplastic polyesters include polylactides, polyglycolides, polycaprolactones, copolymers thereof, terpolymers thereof, and any combinations thereof. In some such embodiments, the suitable biodegradable thermoplastic polyester is a polylactide, a polyglycolide, a copolymer thereof, a terpolymer thereof, or a combination thereof. In one embodiment, the biodegradable thermoplastic polyester is 50/50 poly(DL-lactide-co-glycolide) having a carboxy terminal group; is present in about 30 wt. % to about 40 wt. % of the composition; and has an average molecular weight of about 23,000 to about 45,000. Alternatively, in another embodiment, the biodegradable thermoplastic polyester is 75/25 poly (DL-lactide-co-glycolide) without a carboxy terminal group; is present in about 40 wt. % to about 50 wt. % of the composition; and has an average molecular weight of about 15,000 to about 24,000. In further or alternative embodiments, the terminal groups of the poly(DL-lactide-co-glycolide) are either hydroxyl, carboxyl, or ester depending upon the method of polymerization. Polycondensation of lactic or glycolic acid provides a polymer with terminal hydroxyl and carboxyl groups. Ring-opening polymerization of the cyclic lactide or glycolide monomers with water, lactic acid, or glycolic acid provides polymers with the same terminal groups. However, ring-opening of the cyclic monomers with a monofunctional alcohol such as methanol, ethanol, or 1-dodecanol provides a polymer with one hydroxyl group and one ester terminal groups. Ring-opening polymerization of the cyclic monomers with a diol such as 1,6-hexanediol or polyethylene glycol provides a polymer with only hydroxyl terminal groups.

Since the polymer systems of thermosetting gels dissolve more completely at reduced temperatures, methods of solubilization include adding the required amount of polymer to the amount of water to be used at reduced temperatures. Generally after wetting the polymer by shaking, the mixture is capped and placed in a cold chamber or in a thermostatic container at about 0-10 °C in order to dissolve the polymer. The mixture is stirred or shaken to bring about a more rapid dissolution of the thermosetting gel polymer. The muscarinic antagonist or ophthalmic agent and various additives such as buffers, salts, and preservatives are subsequently added and dissolved. In some instances the pharmaceutically agent is suspended if it is insoluble in water. The pD is modulated by the addition of appropriate buffering agents.

### Ophthalmic Ointment Composition

An ointment is a homogeneous, viscous, semi-solid preparation, most commonly a greasy, thick oil (e.g. oil 80% - water 20%) with a high viscosity, intended for external application to the skin or mucous membranes. Ointments have a water number that defines the maximum amount of water that it contains. They are used as emollients or for the application of active ingredients to the skin for protective, therapeutic, or prophylactic purposes and where a degree of occlusion is desired. Ointments are used topically on a variety of body surfaces. These include the skin and the mucous membranes of the eye (an eye ointment), vulva, anus, and nose

The vehicle of an ointment is known as the ointment base. The choice of a base depends upon the clinical indication for the ointment. The different types of ointment bases are: hydrocarbon bases, e.g. hard paraffin, soft paraffin, microcrystalline wax and ceresine; absorption bases, e.g. wool fat, beeswax; water soluble bases, e.g. macrogols 200, 300, 400; emulsifying bases, e.g. emulsifying wax, cetrimide; vegetable oils, e.g. olive oil, coconut oil, sesame oil, almond oil and peanut oil.

Ointments are formulated using hydrophobic, hydrophilic, or water-emulsifying bases to provide preparations that are immiscible, miscible, or emulsifiable with skin secretions. In some instances, they are also derived from hydrocarbon (fatty), absorption, water-removable, or water-soluble bases. The active agents are dispersed in the base, and later they get divided after the drug penetration into the target sites (e.g. membranes, skins, etc.).

The present disclosure recognizes that it is sometimes difficult to incorporate into the ointment a drug of low concentration with sufficient dose-to-dose uniformity for effectively treating a disorder or disease. In some instances, poly(ethylene-glycols), polyethoxylated castor oils (Cremophor^{®}EL), alcohols having 12 to 20 carbon atoms or a mixture of two or more of said components are effective excipients for dispersing and/or dissolving effective amounts of ophthalmic drugs, in particular of ascomycins and staurosporine derivatives, in an ointment base, in particular in an ointment base substantially comprising oleaginous and hydrocarbon components, and that the resulting ointments are excellently tolerated by the skin and by ocular tissue.

The present disclosure further recognizes that ophthalmic drugs, such as a muscarinic antagonist (e.g. atropine or its pharmaceutically acceptable salts) or an ophthalmic agent, incorporated in the ointment compositions described herein target the choroid and/or retina in a patient when the compositions are topically administered to the ocular surface, in particular to the sclera of said patient. In some instances, an ophthalmic ointment composition includes an ophthalmic drug, an ointment base and an agent for dispersing and/or dissolving said drug in the ointment base, selected from a poly(ethylene-glycol), a polyethoxylated castor oil, an alcohol having 12 to 20 carbon atoms and a mixture of two or more of said components.

In some instances, the ointment bases include ophthalmically acceptable oil and fat bases, such as natural wax e.g. white and yellow bees wax, carnauba wax, wool wax (wool fat), purified lanolin, anhydrous lanolin; petroleum wax e.g. hard paraffin, microcrystalline wax; hydrocarbons e.g. liquid paraffin, white and yellow soft paraffin, white petrolatum, yellow petrolatum; or combinations thereof.

The above mentioned oil and fat bases are described in more detail, for instance, in the British Pharmacopoeia, Edition 2001, or the European Pharmacopoeia, 3rd Edition.

In some instances, the ointment base is present in amounts of about 50 to about 95, preferably of 70 to 90% by weight based on the total weight of the composition.

A preferred ointment base comprises a combination of one or more of one or more natural waxes like those indicated above, preferably wool wax (wool fat), and one or more hydrocarbons like those indicated above, preferably a soft paraffin or a petrolatum, more preferably in combination with liquid paraffin.

A specific instance of the aforementioned ointment base comprises e.g. 5 to 17 parts by weight of wool fat, and 50 to 65 parts by weight of white petrolatum as well as 20 to 30 parts by weight of liquid paraffin.

In some instances, the agent for dispersing and/or dissolving the ophthalmic drug in the ointment base is selected from a poly(ethylene-glycol), a polyethoxylated castor oil, an alcohol having 12 to 20 carbon atoms and a mixture of two or more of said components. The agent is preferably used in amounts of 1 to 20 percent, more preferably 1 to 10 percent by weight of the entire semisolid ophthalmic composition.

Alcohols having 12 to 20 carbon atoms include particularly stearyl alcohol (C18H37OH), cetyl alcohol (C16H33OH) and mixtures thereof. Preferred are so-called cetostearyl alcohols, mixtures of solid alcohols substantially consisting of stearyl and cetyl alcohol and preferably comprising not less than 40 percent by weight of stearyl alcohol and a sum of stearyl alcohol and cetyl alcohol amounting to at least 90 percent by weight, and compositions comprising not less than 80 percent by weight of cetylstearyl alcohol and an emulsifier, in particular sodium cetostearyl sulfate and/or sodium lauryl sulfate, preferably in amounts not less than 7 percent by weight of emulsifier.

Polyethoxylated castor oils are reaction products of natural or hydrogenated castor oils and ethylene glycol. In some instances, such products are obtained in known manner, e.g. by reaction of a natural or hydrogenated castor oil or fractions thereof with ethylene oxide, e.g. in a molar ratio of from about 1:30 to about 1:60, with optional removal of free polyethylene glycol components from the product, e.g. in accordance with the methods disclosed in German Auslegeschriften 1,182,388 and 1,518,819. Especially suitable and preferred is a product commercially available under the trade name Cremophor^{®}EL having a molecular weight (by steam osmometry)=ca. 1630, a saponification no.=ca. 65-70, an acid no.=ca. 2, an iodine no.=ca. 28-32 and an nD 25=ca.1.471. Also suitable for use in this category is, for instance, Nikkol^{®}HCO-60, a reaction product of hydrogenated castor oil and ethylene oxide exhibiting the following characteristics: acid no.=ca. 0.3; saponification no.=ca. 47.4; hydroxy value=ca. 42.5. pH (5%)=ca. 4.6; Color APHA=ca. 40; m.p.=ca. 36.0° C.; Freezing point=ca. 32.4° C.; H2O content (%, KF)=ca. 0.03.

Poly(ethylene-glycols) are used in some instances as the agent for dispersing and/or dissolving the ophthalmic drug in the ointment base according to the present disclosure. Suitable poly(ethylene-glycol)s are typically mixtures of polymeric compounds of the general formula H-(OCH2-CH2)nOH, wherein the index n typically range from 4 to 230 and the mean molecular weight from about 200 to about 10000. Preferably n is a number from about 6 to about 22 and the mean molecular weight between about 300 and about 1000, more preferably n ranges from about 6 to about 13 and the mean molecular weight from about 300 to about 600, most preferably n has a value of about 8.5 to about 9 and the relative molecular weight is about 400. Suitable poly(ethylene-glycols) are readily available commercially, for example poly(ethylene-glycols) having a mean molecular weight of about 200, 300, 400, 600, 1000, 1500, 2000, 3000, 4000, 6000, 8000, and 10000.

The poly(ethylene-glycols), in particular the preferred types described in the foregoing paragraph, are preferably used in amounts of 1 to 10, more preferably 1 to 5 percent by weight of the entire semisolid ophthalmic composition.

A specific instance comprises an agent for dispersing and/or dissolving of the drug in the ointment base which is selected from a poly(ethylene-glycol), a polyethoxylated castor oil and preferably a mixture of said components.

### Gel/Ointment Viscosity

In some embodiments, the composition has a Brookfield RVDV viscosity of from about 10,000 to about 300,000 cps at about 20°C and sheer rate of 1s⁻¹ (1 cP corresponds to 1 mPa·s). In some embodiments, the composition has a Brookfield RVDV viscosity of from about 15,000 to about 200,000 cps at about 20°C and sheer rate of 1s⁻¹. In some embodiments, the composition has a Brookfield RVDV viscosity of from about 50,000 to about 150,000 cps at about 20°C and sheer rate of 1s⁻¹. In some embodiments, the composition has a Brookfield RVDV viscosity of from about 70,000 to about 130,000 cps at about 20°C and sheer rate of 1s⁻¹. In some embodiments, the composition has a Brookfield RVDV viscosity of from about 90,000 to about 110,000 cps at about 20°C and sheer rate of 1s⁻¹.

In some embodiments, the ophthalmic gel formulation contains a viscosity enhancing agent sufficient to provide a viscosity of between about 500 and 1,000,000 centipoise, between about 750 and 1,000,000 centipoise; between about 1000 and 1,000,000 centipoise; between about 1000 and 400,000 centipoise; between about 2000 and 100,000 centipoise; between about 3000 and 50,000 centipoise; between about 4000 and 25,000 centipoise; between about 5000 and 20,000 centipoise; or between about 6000 and 15,000 centipoise. In some embodiments, the ophthalmic gel formulation contains a viscosity enhancing agent sufficient to provide a viscosity of between about 50,0000 and 1,000,000 centipoise.

In some embodiments, the compositions described herein are low viscosity compositions at body temperature. In some embodiments, low viscosity compositions contain from about 1% to about 10% of a viscosity enhancing agent (e.g., gelling components such as polyoxyethylene-polyoxypropylene copolymers). In some embodiments, low viscosity compositions contain from about 2% to about 10% of a viscosity enhancing agent (e.g., gelling components such as polyoxyethylene-polyoxypropylene copolymers). In some embodiments, low viscosity compositions contain from about 5% to about 10% of a viscosity enhancing agent (e.g., gelling components such as polyoxyethylene-polyoxypropylene copolymers). In some embodiments, low viscosity compositions are substantially free of a viscosity enhancing agent (e.g., gelling components such as polyoxyethylene-polyoxypropylene copolymers). In some embodiments, a low viscosity muscarinic antagonist/ophthalmic agent composition described herein provides an apparent viscosity of from about 100 cP to about 10,000 cP. In some embodiments, a low viscosity muscarinic antagonist/ophthalmic agent composition described herein provides an apparent viscosity of from about 500 cP to about 10,000 cP. In some embodiments, a low viscosity muscarinic antagonist/ophthalmic agent composition described herein provides an apparent viscosity of from about 1000 cP to about 10,000 cP.

In some embodiments, the compositions described herein are viscous compositions at body temperature. In some embodiments, viscous compositions contain from about 10% to about 25% of a viscosity enhancing agent (e.g., gelling components such as polyoxyethylene-polyoxypropylene copolymers). In some embodiments, the viscous compositions contain from about 14% to about 22% of a viscosity enhancing agent (e.g., gelling components such as polyoxyethylene-polyoxypropylene copolymers). In some embodiments, the viscous compositions contain from about 15% to about 21% of a viscosity enhancing agent (e.g., gelling components such as polyoxyethylene-polyoxypropylene copolymers). In some embodiments, a viscous ophthalmic composition described herein provides an apparent viscosity of from about 100,000 cP to about 1,000,000 cP. In some embodiments, a viscous ophthalmic composition described herein provides an apparent viscosity of from about 150,000 cP to about 500,000 cP. In some embodiments, a viscous ophthalmic composition described herein provides an apparent viscosity of from about 250,000 cP to about 500,000 cP. In some of such embodiments, a viscous ophthalmic composition is a liquid at room temperature and gels at about between room temperature and body temperature (including an individual with a serious fever, e.g., up to about 42 °C). In some embodiments, a viscous ophthalmic composition is administered as monotherapy for treatment of an ophthalmic disease or condition described herein.

In some embodiments, the viscosity of the gel formulations presented herein is measured by any means described. For example, in some embodiments, an LVDV-II+CP Cone Plate Viscometer and a Cone Spindle CPE-40 is used to calculate the viscosity of the gel formulation described herein. In other embodiments, a Brookfield (spindle and cup) viscometer is used to calculate the viscosity of the gel formulation described herein. In some embodiments, the viscosity ranges referred to herein are measured at room temperature. In other embodiments, the viscosity ranges referred to herein are measured at body temperature (e.g., at the average body temperature of a healthy human).

### Gel/Ointment Dose-To-Dose Uniformity

Typical ophthalmic gels are packaged in eye drop bottles and administered as drops. For example, a single administration (i.e. a single dose) of an ophthalmic gel includes a single drop, two drops, three drops or more into the eyes of the patient. Furthermore, typical ophthalmic ointments are packaged in tubes or other squeezable containers with a dispensing nozzle through which strips of the ointment are delivered. For example, a single administration (i.e. a single dose) of an ophthalmic ointment includes a single strip, or multiple strips into the eyes of the patient. In some embodiments, one dose of the ophthalmic gel described herein is one drop of the gel composition from the eye drop bottle. In some instances, one dose of the ophthalmic ointment is one strip of the ointment composition dispensed through the nozzle of a dispersing tube.

In some cases, described herein include ophthalmic gel compositions which provide a dose-to-dose uniform concentrations. In some instances, the dose-to-dose uniform concentration does not present significant variations of drug content from one dose to another. In some instances, the dose-to-dose uniform concentration does provide consistent drug content from one dose to another.

In some cases, described herein include ophthalmic ointment compositions which provide a dose-to-dose uniform concentrations. In some instances, the dose-to-dose uniform concentration does not present significant variations of drug content from one dose to another. In some instances, the dose-to-dose uniform concentration does provide consistent drug content from one dose to another.

In some embodiments, the composition has a dose-to-dose muscarinic antagonist or ophthalmic agent concentration variation of less than 50%. In some embodiments, the composition has a dose-to-dose muscarinic antagonist or ophthalmic agent concentration variation of less than 40%. In some embodiments, the composition has a dose-to-dose muscarinic antagonist or ophthalmic agent concentration variation of less than 30%. In some embodiments, the composition has a dose-to-dose muscarinic antagonist or ophthalmic agent concentration variation of less than 20%. In some embodiments, the composition has a dose-to-dose muscarinic antagonist or ophthalmic agent concentration variation of less than 10%. In some embodiments, the composition has a dose-to-dose muscarinic antagonist or ophthalmic agent concentration variation of less than 5%.

In some embodiments, the dose-to-dose muscarinic antagonist or ophthalmic agent concentration variation is based on 10 consecutive doses. In some embodiments, the dose-to-dose muscarinic antagonist or ophthalmic agent concentration variation is based on 8 consecutive doses. In some embodiments, the dose-to-dose muscarinic antagonist or ophthalmic agent concentration variation is based on 5 consecutive doses. In some embodiments, the dose-to-dose muscarinic antagonist or ophthalmic agent concentration variation is based on 3 consecutive doses. In some embodiments, the dose-to-dose muscarinic antagonist or ophthalmic agent concentration variation is based on 2 consecutive doses.

A nonsettling formulation should not require shaking to disperse drug uniformly. A "no-shake" formulation is potentially advantageous over formulations that require shaking for the simple reason that patients' shaking behavior is a major source of variability in the amount of drug dosed. It has been reported that patients often times do not or forget to shake their ophthalmic compositions that requires shaking before administering a dose, despite the instructions to shake that were clearly marked on the label. On the other hand, even for those patients who do shake the product, it is normally not possible to determine whether the shaking is adequate in intensity and/or duration to render the product uniform. In some embodiments, the ophthalmic gel compositions, and the also-described ophthalmic ointment compositions, described herein are "no-shake" formulations that maintained the dose-to-dose uniformity described herein.

To evaluate the dose-to-dose uniformity, drop bottles or tubes containing the ophthalmic aqueous compositions, the ophthalmic gel compositions, or ophthalmic ointment compositions are stored upright for a minimum of 12 hours prior to the start of the test. To simulate the recommended dosing of these products, predetermined number of drops or strips are dispensed from each commercial bottles or tubes at predetermined time intervals for an extended period of time or until no product was left in the bottle or tube. All drops and strips are dispensed into tared glass vials, capped, and stored at room temperature until analysis. Concentrations of a muscarinic antagonist such as atropine in the expressed drops were determined using a reverse-phase HPLC method.

### Ophthalmic Composition for use in Methods of Treatment

Disclosed herein are ophthalmic compositions described *supra* for use in methods of treating one or more ophthalmic conditions or diseases by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free. Also disclosed herein are ophthalmic compositions described *supra* for use in methods of ameliorating or reducing one or more ophthalmic conditions or diseases by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free.

In some instances, the ophthalmic condition or disease includes a condition or disease associated with the eyelid, the lacrimal system, or the orbit (Fig. 1). In some instances, the lacrimal system encompasses the orbital structures for tear production and drainage. In some instances, the lacrimal system comprises the lacrimal gland responsible for tear production, excretory ducts which convey the fluid to the surface of the eye, lacrimal canaliculi, lacrimal sac, and nasolacrimal duct. In some instances, the orbit encompasses the eye and its associated appendages. In some instances, an ophthalmic composition described herein is for use in a method of treatment of a condition or disease associated with the eyelid, lacrimal system or the orbit by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free.

In some instances, the ophthalmic condition or disease includes a condition or disease associated with the conjunctiva, sclera, cornea, iris, or ciliary body (Fig. 1). Conjunctiva lines the inside of the eyelids and covers the sclera. Sclera, or the white of the eye, is an opaque, fibrous, protective outer layer of the eye. Cornea is the transparent front part of the eye that covers the iris, pupil, and anterior chamber. Iris is a thin, circular structure in the eye responsible for controlling the diameter and size of the pupil and therefore the amount of light reaching the retina. Ciliary body includes the ciliary muscle, which controls the shape of the lens and the ciliary epithelium, which produces the aqueous humor. In some instances, an ophthalmic composition described herein is for use in a method of treatment of a condition or disease associated with conjunctiva, sclera, cornea, iris, or ciliary body by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free.

In some instances, the ophthalmic condition or disease includes a condition or disease associated with the choroid or retina (Fig. 1). Choroid, also known as choroidea or choroid coat, is the vascular layer of the eye containing connective tissue and is in between the retina and the sclera. Retina is the third and inner coat of the eye and is a light-sensitive tissue layer. In some instances, an ophthalmic composition described herein is for use in a method of treatment of a condition or disease associated with choroid or retina by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free.

In some instances, the ophthalmic condition or disease includes a condition or disease associated with the lens (Fig. 1). The lens or crystalline lens is a transparent, biconvex structure in the eye that in combination with the cornea helps to refract light to be focused on the retina. In some instances, an ophthalmic composition described herein is for use in a method of treatment of a condition or disease associated with the lens by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free.

In some instances, the ophthalmic conditions or diseases include, but are not limited to, Acanthamoeba keratitis, Bell's palsy, blepharochalasis, blepharitis, chalazion, cataract, cyclitis, cytomegalovirus (CMV) retinitis, chorioretinal inflammation, conjunctivitis (e.g., allergy related conjunctivitis or conjunctivitis due to infection), neonatol conjunctivitis, corneal neovascularization, corneal ulcer, dermatitis, diabetic retinopathy, dry eye syndrome, dacryoadenitis, dacryostenosis, endophthalmitis, epiphora, episcleritis, eye impetigo, eyelash hypotrichosis, Fuchs' dystrophy (also known as Fuchs' corneal endothelial dystrophy or FCED), glaucoma, hypermetropia, iritis, keratoconjunctivitis, keratoconjunctivitis sicca, macular degeneration (e.g., Stargardt's disease), macular dystrophy, macular edema (e.g., diabetic macular edema), myopia, ocular hypertension, loiasis, ocular rosacea, onchocerciasis (or known as river blindness or Robles disease), optic neuritis and optic neuropathy, keratitis (e.g., bacterial keratitis, fungal keratitis, parasitic keratitis, or viral keratitis), pinguecular and pterygium, production of miosis, scleritis, steroid responsive inflammatory conditions, stye (or hordeolum), temporal arteritis, Thygeson's superficial punctate keratopathy (TSPK), trachoma, organophosphate poisoning, basal cell carcinoma, squamous carcinoma, sebaceous carcinoma, malignant melanoma, orbital lymphoma, uveitis, uveal melanoma, retinoblastoma, medulloepithelioma, or primary intraocular lymphoma. In some instances, viral keratitis includes ocular herpes or Herpetic keratitis, or Herpes Simplex dendritic keratitis.

In some instances, viruses that cause viral eye infections include Herpes simplex virus, Epstein Barr virus, or influenza virus.

In some instances, fungi that cause fungal eye infections include *Arthrobotrys oligospora, Aspergillus versicolar, Candida, Cladosporium, Cephaliophora irregularis, Exophiala, Fusarium* (e.g*., Fusarium solani*)*, Phoma,* or *Scedosporium* (*e.g., Scedosporium prolificans*)*.*

In some instances, bacteria that cause bacterial eye infections include *Chlamydia trachomatis, N. meningitidis, Staphylococcus aureus, S. epidermidis, S. pneumoniae, Streptococcus* spp., or *Pseudomonas aeruginosa.*

In some instances, parasites that cause eye infections include *Demodex, Leishmania,* nematode such as *Loa loa, Simulium, Toxoplasma gondii,* or *Toxocara.*

In some instances, the ophthalmic condition or disease refers to a condition or disease that requires surgery. In some instances, the administration of the one or more of the ophthalmic compositions is before, during, or after surgery, or for surgery-related complications. Exemplary surgeries include laser eye surgery, cataract surgery, glaucoma surgery, canaloplasty, refractive surgery, corneal surgery, vitrectomy, eye muscle surgery, and oculoplastic surgery. In some instances, surgery-related complications include postoperative increased intraocular pressure and postoperative ocular inflammation.

In some instances, the ophthalmic condition or disease refers to a condition or disease that requires aid of a diagnostic agent for visualization.

Disclosed herein are ophthalmic composition as described above for use in methods of arresting myopia development by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free. Also disclosed herein are ophthalmic composition as described above for use in methods of treating myopia by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free. Additionally disclosed herein are ophthalmic composition as described above for use in methods of preventing myopia development by administering to an eye of an individual in need thereof an effective amount of the ophthalmic composition from a fluid-dispensing device comprising an internal filter or membrane, a reservoir and a dispensing tip fitted onto the reservoir wherein the ophthalmic composition dispensed from the fluid-dispensing device is preservative-free.

In some instances, the ophthalmic aqueous formulations described herein are packaged in eye drop bottles and administered as drops. For example, a single administration (i.e. a single dose) of an ophthalmic aqueous formulation includes a single drop, two drops, three drops or more into the eyes of the patient. In some instances, the ophthalmic gel formulations described herein are packaged in eye drop bottles and administered as drops. For example, a single administration (i.e. a single dose) of an ophthalmic gel includes a single drop, two drops, three drops or more into the eyes of the patient. In some instances, the ophthalmic ointment formulations described herein are packaged in tubes or other squeezable containers with a dispensing nozzle through which strips of the ointment are delivered. For example, a single administration (i.e. a single dose) of an ophthalmic ointment includes a single strip, or multiple strips into the eyes of the patient. In some instances, one dose of the ophthalmic aqueous formulation described herein is one drop of the aqueous composition from the eye drop bottle. In some instances, one dose of the ophthalmic gel described herein is one drop of the gel composition from the eye drop bottle. In some instances, one dose of the ophthalmic ointment is one strip of the ointment composition dispensed through the nozzle of a dispersing tube.

In some embodiments of the disclosed method, the ophthalmic composition is stored below room temperature prior to first use. In some embodiments of the disclosed method, the ophthalmic composition is stored at between about 2 °C to about 10 °C prior to first use. In some embodiments of the disclosed method, the ophthalmic composition is stored at about 2 °C, about 3 °C, about 4 °C, about 5 °C, about 6 °C, about 7 °C, about 8 °C, about 9 °C, or about 10 °C prior to first use. In some embodiments of the disclosed method, the ophthalmic composition is stored at between about 4 °C to about 8 °C prior to first use.

In some embodiments of the disclosed method, the ophthalmic composition is stored at room temperature after first use. In some embodiments of the disclosed method, the ophthalmic composition is stored at between about 16 °C to about 26 °C after to first use. In some embodiments of the disclosed method, the ophthalmic composition is stored at about 16 °C, about 17 °C, about 18 °C, about 19 °C, about 20 °C, about 21 °C, about 22 °C, about 23 °C, about 24 °C, about 25 °C, or about 26 °C after to first use.

In some instances, the ophthalmic aqueous formulations are administered as follows: the lower lid of the eye to be administered was pulled down and a predetermined amount of the aqueous formulation (e.g. 1-3 drops) is applied to the inside of the eyelid. The ophthalmic tip of the dispensing mechanism does not touch any surface to avoid contamination and/or injury.

In some instances, the ophthalmic gel formulations are administered as follows: the lower lid of the eye to be administered was pulled down and a predetermined amount of gel (e.g. 1-3 drops) is applied to the inside of the eyelid. The ophthalmic tip of the dispensing mechanism does not touch any surface to avoid contamination and/or injury.

In some instances, the ophthalmic ointment formulations are administered as follows: the lower lid of the eye to be administered was pulled down and a small amount of ointment (approximately 0.635 cm (0.25 inches)) was applied to the inside of the eyelid. The ophthalmic tip of the dispensing mechanism does not touch any surface to avoid contamination and/or injury.

In some instances, the ophthalmic composition is administered at predetermined time intervals over an extended period of time. In some instances, the ophthalmic composition is administered once every day. In some instances, the ophthalmic composition is administered every other day. In some instances, the ophthalmic composition is administered over 1 week, 2 weeks, 1 month, 2 months, 3 months, 6 moths, 1 year, 2 years, 3 years, 4 years, 5 years, 6 years, 7 years, 8 years, 9 years, 10 years, 11 years, or 12-15 years.

In some instances, the ophthalmic composition is administered in doses having a dose-to-dose muscarinic antagonist or ophthalmic agent concentration variation of less than 50%, less than 40%, less than 30%, less than 20%, less than 10%, or less than 5%.

The number of times a composition is administered to an individual in need thereof depends on the discretion of a medical professional, the disorder, the severity of the disorder, and the individual's response to the formulation. In some instances, a composition disclosed herein is administered once to an individual in need thereof with a mild acute condition. In some instances, a composition disclosed herein is administered more than once to an individual in need thereof with a moderate or severe acute condition. In the case wherein the patient's condition does not improve, upon the doctor's discretion the administration of a muscarinic antagonist or an ophthalmic agent is administered chronically, that is, for an extended period of time, including throughout the duration of the patient's life in order to ameliorate or otherwise control or limit the symptoms of the patient's disease or condition.

In the case wherein the patient's condition does not improve, upon the doctor's discretion the administration of the muscarinic antagonist or ophthalmic agent is administered chronically, that is, for an extended period of time, including throughout the duration of the patient's life in order to ameliorate or otherwise control or limit the symptoms of the patient's disease or condition.

In the case wherein the patient's status does improve, upon the doctor's discretion the administration of the muscarinic antagonist or ophthalmic agent is given continuously; alternatively, the dose of drug being administered is temporarily reduced or temporarily suspended for a certain length of time (*i.e.,* a "drug holiday"). The length of the drug holiday varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, and 365 days. The dose reduction during a drug holiday is from 10%-100%, including by way of example only 10%, 15%, 20%, 25%, 30%, 35%, 40%, 45%, 50%, 55%, 60%, 65%, 70%, 75%, 80%, 85%, 90%, 95%, and 100%.

Once improvement of the patient's ophthalmic conditions has occurred, a maintenance muscarinic antagonist or ophthalmic agent dose is administered if necessary. Subsequently, the dosage or the frequency of administration, or both, is optionally reduced, as a function of the symptoms, to a level at which the improved disease, disorder or condition is retained. In certain instances, patients require intermittent treatment on a long-term basis upon any recurrence of symptoms.

The amount of a muscarinic antagonist or an ophthalmic agent that will correspond to such an amount will vary depending upon factors such as the particular compound, disease condition and its severity, according to the particular circumstances surrounding the case, including, *e.g.*, the specific muscarinic antagonist or ophthalmic agent being administered, the route of administration, the condition being treated, the target area being treated, and the subject or host being treated. The desired dose is presented in a single dose or as divided doses administered simultaneously (or over a short period of time) or at appropriate intervals.

In some instances, the initial administration is a particular muscarinic antagonist or ophthalmic agent and the subsequent administration a different formulation or muscarinic antagonist or ophthalmic agent.

### Kits/Articles of Manufacture

The invention relates to an ophthalmic product comprising a fluid-dispensing device comprising an internal filter or membrane, a reservoir and a dispensing tip fitted onto the reservoir, and an ophthalmic composition as described herein.

According to the invention, the ophthalmic compositions are presented in a dispenser device which contains one or more unit dosage forms containing a compound provided herein. In a further instance, the dispenser device is accompanied by instructions for administration. In yet a further instance, the dispenser is also accompanied with a notice associated with the container in form prescribed by a governmental agency regulating the manufacture, use, or sale of pharmaceuticals, which notice is reflective of approval by the agency of the form of the drug for human or veterinary administration. In another instance, such notice, for example, is the labeling approved by the U.S. Food and Drug Administration for prescription drugs, or the approved product insert. In yet another instance, compositions containing a compound provided herein formulated in a compatible pharmaceutical carrier are also prepared, placed in an appropriate container, and labeled for treatment of an indicated condition.

### Terminology

Unless defined otherwise, all technical and scientific terms used herein have the same meaning as is commonly understood by one of skill in the art to which the claimed subject matter belongs. It is to be understood that the foregoing general description and the following detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include," "includes," and "included," is not limiting.

As used herein, ranges and amounts is expressed as "about" a particular value or range. About also includes the exact amount. Hence "about 5 µg" means "about 5 µg" and also "5 µg." Generally, the term "about" includes an amount that is expected to be within experimental error.

The section headings used herein are for organizational purposes only and are not to be construed as limiting the subject matter described.

The terms "subject" and "individual", as included herein, are used interchangeably. None of the terms are to be interpreted as requiring the supervision of a medical professional (e.g., a doctor, nurse, physician's assistant, orderly, hospice worker).

### EXAMPLES

### Example 1 - Ophthalmic Formulations

Exemplary compositions for preparation of ophthalmic formulations are described in Tables 1-13.

**Table 1 -Preservative-Free Aqueous Solution Formulation (Atropine)**

| **Ingredient** | **Quantity (mg/g)** | **Concentration (wt%)** |
|---|---|---|
| Atropine | 0.01-0.5 | 0.001-0.05 (wt%) |
| Buffer agent and/or pD adjusting agent (e.g. , borates and/or DCl) | - | q.s. for pD=4.2-7.9 |
| Tonicity and/or Osmolarity adjustor (e.g. NaCl, mannitol, etc) | - | q.s. to 0.5-2.0 wt% |
| Deuterated Water | - | q.s. to 100 wt% |

**Table 2 -Preservative-Free Aqueous Solution Formulation (Atropine Sulfate)**

| **Ingredient** | **Quantity (mg/g)** | **Concentration (wt%)** |
|---|---|---|
| Atropine sulfate | 0.01-0.5 | 0.001-0.05 (wt%) |
| Buffer agent and/or pD adjusting agent (e.g. , borates and/or DCl) | - | q.s. for pD=4.2-7.9 |
| Tonicity and/or Osmolarity adjustor (e.g. NaCl, mannitol, etc) | - | q.s. to 0.5-2.0 wt% |
| Deuterated Water | - | q.s. to 100 wt% |

**Table 3 -Preservative-Free Aqueous Solution Formulation (Atropine Sulfate)**

| **Ingredient** | **Quantity (mg/g)** | **Concentration (wt%)** |
|---|---|---|
| Atropine sulfate | 0.05-0.15 | 0.005-0.015 (wt%) |
| Buffer agent and/or pD adjusting agent (e.g. , borates and/or DCl) | - | q.s. for pD=4.2-7.9 |
| Preservative (e.g. benzalkonium chloride, cetrimonium sodium perborate, etc.) | - | q.s. to prevent the growth of or to destroy microorganism introduced into the solution |
| Tonicity and/or Osmolarity adjustor (e.g. NaCl, mannitol, etc) | - | q.s. to 0.5-2.0 wt% |
| Deuterated Water | - | q.s. to 100 wt% |

**Table 4 -Preservative-Free Mucus Penetrating Particle Formulation (Atropine)**

| **Ingredient** | **Quantity (mg/g)** | **Concentration (wt%)** |
|---|---|---|
| Atropine | 0.01-0.5 | 0.001-0.05 (wt%) |
| Buffer agent and/or pD adjusting agent (e.g. , borates and/or DCl) | - | q.s. for pD=4.2-7.9 |
| Mucus penetrating particles | - | q.s. to formulate atropine at 0.001-0.05 wt% |
| Deuterated Water | - | q.s. to 100 wt% |

**Table 5 -Preservative-Free Mucus Penetrating Particle Formulation (Atropine Sulfate)**

| **Ingredient** | **Quantity (mg/g)** | **Concentration (wt%)** |
|---|---|---|
| Atropine sulfate | 0.01-0.5 | 0.001-0.05 (wt%) |
| Buffer agent and/or pD adjusting agent (e.g. , borates and/or DCl) | - | q.s. for pD=4.2-7.9 |
| Mucus penetrating particles | - | q.s. to formulate atropine at 0.001-0.05 wt% |
| Deuterated Water | - | q.s. to 100 wt% |

**Table 6 -Cellulose Gel Formulation (Atropine Sulfate)**

| **Ingredient** | **Quantity (mg/g)** | **Concentration (wt%)** |
|---|---|---|
| Atropine Sulfate | 0.01-0.5 | 0.001-0.05 (wt%) |
| Viscosity enhancing agent (e.g. hydroxypropyl methylcellulose) | 10-50 | **1-5** (wt%) |
| Buffer agent and/or pD adjusting agent (e.g. , sodium acetate and/or DCl) | - | q.s. for pD=4.2-7.9 |
| Stabilizer (e.g. EDTA, cyclodextrin, etc.) | - | q.s. for low degradation of atropine sulfate (e.g. less than 10%, 5% or 1% degradation) |
| Osmolarity modifier (e.g. NaCl) | - | q.s. 150-500 mOsm/L |
| Deuterated Water | - | q.s. to 100 wt% |

**Table 7 -Thermosetting Gel Formulation (Atropine Sulfate)**

| **Ingredient** | **Quantity (mg/g)** | **Concentration (wt%)** |
|---|---|---|
| Atropine sulfate | 0.01-0.5 | 0.001-0.05 (wt%) |
| Viscosity enhancing agent (e.g. poloxamer 407) | 100-250 | 10-25 (wt%) |
| Buffer agent and/or pD adjusting agent (e.g. , sodium acetate and/or DCl) | - | q.s. for pH=4.2-7.9 |
| Stabilizer (e.g. EDTA, cyclodextrin, etc.) | - | q.s. for low degradation of atropine sulfate (e.g. less than 10%, 5% or 1% degradation) |
| Osmolarity modifier (e.g. NaCl) | - | q.s. 150-500 mOsm/L |
| Deuterated Water | - | q.s. to 100 wt% |

**Table 8 - Reference Ointment Formulation (Atropine Sulfate)**

| **Ingredient** | **Quantity (g) for 1000 mL solution** | **Concentration in 1000 mL aqueous solution** |
|---|---|---|
| Atropine sulfate | 0.01-0.5 | 0.001-0.05 (wt%) |
| Dispersing agent (e.g. polyethyleneglycol, and/or polyethoxylated castor oil and/or C12-C20 alcohol | 10-200 | 1-20 (wt%) |
| Buffering agent pD adjusting agent (e.g. DCl) | - | q.s. for pD=4.2-7.9 |
| Stabilizer (e.g. EDTA, cyclodextrin, etc.) | - | q.s. for low degradation of atropine sulfate (e.g. less than 10%, 5% or 1% degradation) |
| Osmolarity modifier (e.g. NaCl) | - | q.s. 150-500 mOsm/L |
| Ointment base (e.g. wool wax and/or petrolatum and/or liquid paraffin) | | q.s. to 100 wt% |

**Table 9 - Reference Aqueous Solution Formulation**

| **Ingredient** | **Quantity (mg/g)** | **Concentration (wt%)** |
|---|---|---|
| Ophthalmic agent | 0.01-200 | 0.001-20 (wt%) |
| Buffer agent and/or pD adjusting agent (e.g. , borates and/or DCl) | - | q.s. for pD=4-8 |
| Tonicity and/or Osmolarity adjustor (e.g. NaCl, mannitol, etc) | - | q.s. to 0.5-2.0 wt% |
| Deuterated Water | - | q.s. to 100 wt% |

**Table 10 - Reference Aqueous Solution Formulation**

| **Ingredient** | **Quantity (mg/g)** | **Concentration (wt%)** |
|---|---|---|
| Ophthalmic agent | 0.01-50 | 0.001-5 (wt%) |
| Buffer agent and/or pD adjusting agent (e.g. , borates and/or DCl) | - | q.s. for pD=4-8 |
| Tonicity and/or Osmolarity adjustor (e.g. NaCl, mannitol, etc) | - | q.s. to 0.5-2.0 wt% |
| Deuterated Water | - | q.s. to 100 wt% |

**Table 11 - Reference Cellulose Gel Formulation**

| **Ingredient** | **Quantity (mg/g)** | **Concentration (wt%)** |
|---|---|---|
| Ophthalmic agent | 0.01-200 | 0.001-20 (wt%) |
| Viscosity enhancing agent (e.g. hydroxypropyl methylcellulose) | 10-50 | 1-5 (wt%) |
| Buffer agent and/or pD adjusting agent (e.g. , sodium acetate and/or DCl) | - | q.s. for pD=4-8 |
| Stabilizer (e.g. EDTA, cyclodextrin, etc.) | - | q.s. for low degradation of ophthalmic agent |
| Osmolarity modifier (e.g. NaCl) | - | q.s. 150-500 mOsm/L |
| Deuterated Water | - | q.s. to 100 wt% |

**Table 12 - Reference Thermosetting Gel Formulation**

| **Ingredient** | **Quantity (mg/g)** | **Concentration (wt%)** |
|---|---|---|
| Ophthalmic agent | 0.01-200 | 0.001-20 (wt%) |
| Viscosity enhancing agent (e.g. poloxamer 407) | 100-250 | 10-25 (wt%) |
| Buffer agent and/or pD adjusting agent (e.g. , sodium acetate and/or DCl) | - | q.s. for pH=4.2-7.9 |
| Stabilizer (e.g. EDTA, cyclodextrin, etc.) | - | q.s. for low degradation of ophthalmic agent |
| Osmolarity modifier (e.g. NaCl) | - | q.s. 150-500 mOsm/L |
| Deuterated Water | - | q.s. to 100 wt% |

**Table 13 - Reference Ointment Formulation**

| **Ingredient** | **Quantity (g) for 1000 mL solution** | **Concentration in 1000 mL aqueous solution** |
|---|---|---|
| Ophthalmic agent | 0.01-200 | 0.001-20 (wt%) |
| Dispersing agent (e.g. polyethyleneglycol, and/or polyethoxylated castor oil and/or C12-C20 alcohol | 10-200 | 1-20 (wt%) |
| Buffering agent pD adjusting agent (e.g. DCl) | - | q.s. for pD=4-8 |
| Stabilizer (e.g. EDTA, cyclodextrin, etc.) | - | q.s. for low degradation of ophthalmic agent |
| Osmolarity modifier (e.g. NaCl) | - | q.s. 150-500 mOsm/L |
| Ointment base (e.g. wool wax and/or petrolatum and/or liquid paraffin) | | q.s. to 100 wt% |

### Example 2 - Preparation of an Aqueous Solution Formulation Containing 0.01% Atropine in D₂O

### Stock 1% Solution

In a 100mL solution, 1 gram of atropine, and 0.77 g of NaCl (and other ingredients/components preferably in their dry state) are added along with a quantity sufficient to equal 100mL sterile deuterated water for injection. The solution is mixed in an appropriately sized beaker with a stir bar on a hot plate until all of the solid powders have dissolved and the solution has become clear with no visible particles. Next, the stir bar is removed, and the solution is poured into a filter bottle and vacuum filtered through a 0.22 micron polyethersulfone membrane filter into a sterile bottle. The filter top is removed from the sterile stock bottle and the stock bottle is capped for storage with a sterile bottle cap.

### Diluted 0.01% Solution

0.3mL of the 1% solution was combined with a quantity sufficient to achieve 30mL total of sterile 0.9% Sodium Chloride For Injection USP. The solution was thoroughly mixed. The pH of the solution was recorded. A 0.22 micron filter was placed on the tip of the syringe and the solution was aliquotted into separate sterile containers.

### Example 3 - Preparation of an Aqueous Solution Formulation Containing 0.01% Atropine Sulfate

### Stock 1% Solution

In a 100mL solution, 1 gram of atropine sulfate, and 0.77 g of NaCl (and other ingredients/components preferably in their dry state) were added along with a quantity sufficient to equal 100mL sterile water for injection. The solution was mixed in an appropriately sized beaker with a stir bar on a hot plate until all of the solid powders had dissolved and the solution became clear with no visible particles. Next, the stir bar was removed, and the solution was poured into a filter bottle and vacuum filtered through a 0.22 micron polyethersulfone membrane filter into a sterile bottle. The filter top was removed from the sterile stock bottle and the stock bottle was capped for storage with a sterile bottle cap.

### Diluted 0.01% Solution

0.3mL of the 1% solution was combined with a quantity sufficient to achieve 30mL total of sterile 0.9% Sodium Chloride For Injection USP. The solution was thoroughly mixed. The pH of the solution was recorded. A 0.22 micron filter was placed on the tip of the syringe and the solution was aliquoted into separate sterile containers.

### Example 4 - Stability Analysis

The stability of atropine solutions at different pHs and different storage temperatures was examined. Eighteen atropine solutions at six different pHs were prepared. Each of the atropine solutions was prepared as follows: 83.3 µg/mL free atropine in 0.01% BAK Cl, 0.9% NaCl, and 2.08 mM citric acid in D₂O, adjusted to the desired pH, in a 5mL dropper bottle (Adelphi) to a toal volume of 3.5mL. Next, the 18 atropine solutions were separated into 3 sets, with each set containing 6 different dropper bottles at 6 different pHs. Each set was stored at a different storage temperature. The three temperatures were 2-8 °C, 25°C with 60% relative humidity (RH), and 40°C with 75% RH. Each bottle was stored in a horizontal orientation.

Samples from each dropper bottle were taken at 4 different time intervals (at time of storage, 1 month from date of storage, 3 months, and 6 months) and stability and purity were examined (see Tables 14A-C, 15A-C, and 16A-C). The solutions remained clear and colorless throughout the duration of the experiment.

A separate stability experiment was also carried out using a 12 mL spray bottle (Adelphi). In this experiment, three atropine solutions were each prepared as follows: 83.3 µg/mL free atropine in 0.01% BAK Cl, 0.9% NaCl, 2.08 mM citric acid in D₂O, pH 5.8 (indicates the D₂O formulation). Each of the three atropine solutions was stored at a different temperature, 2-8 °C, 25°C with 60% RH, and 40°C with 75% RH, respectively. Each bottle was stored in a horizontal orientation.

Samples were taken from each spray bottle at 4 different time intervals (at time of storage, 2 months from date of storage, 5 months, and 8 months) and stability and purity were examined (see Tables 14D, 15D, and 16D). The solutions remained clear and colorless throughout the duration of the experiment.

**Table 14A - Atropine solutions at pH 5.4 and pH 5.6 and storage temperature at 2-8 °C**

| **Parameter** | **Time Point** | | | | | | | |
|---|---|---|---|---|---|---|---|---|
| | **Initial** | **T=1 Month** | **T=3 Months** | **T=6 Months** | **Initial** | **T=1 Month** | **T=3 Months** | **T=6 Months** |
| pH* | 5.35* | 5.35* | 5.40* | 5.32 | 5.58* | 5.53* | 5.56* | 5.49 |
| Weight Monitoring² | NA | +0.9 | -2.1 | -7.5 | NA | +1.2 | -8.0 | -13.7 |
| Potency³ (Assay) | 102.3 % | 104.7% | 101.1% | 100.1% | 102.5% | 104.3 % | 101.4% | 99.3% |

| Related Substances⁴ (% Area) | | | | | | | | |
|---|---|---|---|---|---|---|---|---|
| RRT 0.51-0.52 | ND | 0.08% | 0.08% | ND | ND | ND | ND | ND |
| Tropic Acid¹ | 0.02% | 0.07% | 0.06% | 0.05% | 0.04% | 0.08% | 0.08% | 0.08% |
| RRT 1.21 | 0.10% | ND | ND | ND | 0.10% | ND | ND | ND |
| RRT 1.75 | ND | 0.05% | ND | ND | ND | ND | ND | ND |
| RRT 2.39 | ND | 0.06% | ND | ND | ND | ND | ND | ND |
| Total Related Substances | 0.1% | 0.3% | 0.1% | 0.1% | 0.1% | 0.1% | 0.1% | 0.1% |

| BAK Impurities % Area⁵ | | | | | | | | |
|---|---|---|---|---|---|---|---|---|
| RRT 0.98 | ND | ND | ND | 0.10% | ND | ND | ND | 0.11% |
| RRT 1.05 | 0.75% | 0.86% | 0.80% | 0.69% | 0.75% | 0.87% | 0.82% | 0.70% |
| RRT 1.28 - 1.29 | 0.13% | 0.15% | 0.15% | 0.15% | 0.15% | 0.14% | 0.14% | 0.15% |
| RRT 1.53 | 0.19% | 0.06% | ND | ND | 0.18% | 0.06% | ND | ND |

| | | | | | | | | |
|---|---|---|---|---|---|---|---|---|
| * Indicates D₂O formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area | | | | | | | | |

**Table 14B - Atropine solutions at pH 5.8 and pH 6 and storage temperature at 2-8 °C**

| **Parameter** | **Time Point** | | | | | | | |
|---|---|---|---|---|---|---|---|---|
| | **Initial** | **T=1 Month** | **T=3 Months** | **T=6 Months** | **Initial** | **T=1 Month** | **T=3 Months** | **T=6 Months** |
| pH | 5.83* | 5.78* | 5.81* | 5.81 | 5.99* | 6.01* | 6.02* | 5.98 |
| Weight Monitoring² | NA | +1.2 | +0.8 | +.3 | NA | +1.1 | -0.4 | -3.6 |
| Potency³ (Assay) | 102.5 % | 104.4% | 101.3% | 98.7% | 102.6% | 104.2% | 100.7% | 98.7% |

| Related Substances⁴ (% Area) | | | | | | | | |
|---|---|---|---|---|---|---|---|---|
| RRT 0.63 | ND | ND | ND | ND | ND | 0.19% | ND | ND |
| Tropic Acid¹ | 0.05% | 0.10% | 0.12% | 0.14% | 0.06% | 0.13% | 0.15% | 0.18% |
| RRT 0.84 - 0.85 | ND | ND | ND | ND | ND | 0.13% | ND | ND |
| RRT 1.21 | 0.11% | ND | ND | ND | 0.09% | ND | ND | ND |
| RRT 1.36 | ND | ND | ND | ND | ND | 0.11% | ND | ND |
| Total Related Substances | 0.2% | 0.1% | 0.1% | 0.1% | 0.2% | 0.6% | 0.2% | 0.2% |

| BAK Impurities % Area⁵ | | | | | | | | |
|---|---|---|---|---|---|---|---|---|
| RRT 0.98 | ND | ND | ND | 0.10% | ND | ND | ND | 0.11% |
| RRT 1.05 | 0.74% | 0.87% | 0.82% | 0.69% | 0.75% | 0.88% | 0.82% | 0.69% |
| RRT 1.27 - 1.29 | 0.14% | 0.13% | 0.13% | 0.15% | 0.15% | 0.15% | 0.14% | 0.15% |
| RRT 1.53 | 0.20% | 0.06% | ND | ND | 0.19% | 0.07% | ND | ND |

| | | | | | | | | |
|---|---|---|---|---|---|---|---|---|
| * Indicates D₂O formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area | | | | | | | | |

**Table 14 C - Atropine solutions at pH 6.2 and pH 6.4 and storage temperature at 2-8 °C**

| **Parameter** | **Time Point** | | | | | | | |
|---|---|---|---|---|---|---|---|---|
| | **Initial** | **T=1 Month** | **T=3 Months** | **T=6 Months** | **Initial** | **T=1 Month** | **T=3 Months** | **T=6 Months** |
| pH | 6.25* | 6.18* | 6.19* | 6.14 | 6.41* | 6.44* | 6.43* | 6.40 |
| Weight Monitoring² | NA | +1.1 | 0.0 | -2.0 | NA | -0.5 | -0.1 | -1.3 |
| Potency³ (Assay) | 102.5 % | 104.4% | 101.1% | 98.2% | 102.6 % | 104.3% | 101.2% | 98.5% |

| Related Substances⁴ (% Area) | | | | | | | | |
|---|---|---|---|---|---|---|---|---|
| Tropic Acid¹ | 0.17% | 0.25% | 0.28% | 0.35% | 0.10% | 0.24% | 0.31% | 0.53% |
| RRT 1.21 | 0.09% | ND | ND | ND | 0.10% | ND | ND | ND |
| Total Related Substances | 0.3% | 0.3% | 0.3% | 0.4% | 0.2% | 0.2% | 0.3% | 0.5% |

| BAK Impurities % Area⁵ | | | | | | | | |
|---|---|---|---|---|---|---|---|---|
| RRT 0.98 | ND | ND | ND | 0.11% | ND | ND | ND | 0.1% |
| RRT 1.05 | 0.76% | 0.90% | 0.84% | 0.70% | 0.76% | 0.88% | 0.84% | 0.68% |
| RRT 1.28 - 1.29 | 0.14% | 0.14% | 0.12% | 0.15% | 0.13% | 0.15% | 0.14% | 0.16% |
| RRT 1.53 | 0.21% | 0.07% | ND | ND | 0.23% | 0.07% | ND | ND |

| | | | | | | | | |
|---|---|---|---|---|---|---|---|---|
| * Indicates D₂O formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area | | | | | | | | |

**Table 14D - Atropine solutions at pH 5.8 in a spray bottle storage container and storage temperature at 2-8 °C**

| **Parameter** | **Time Point** | | | |
|---|---|---|---|---|
| | **Initial** | **T=2 Month** | **T=5 Months** | **T=8 Months** |
| pH* | 5.80* | 5.85* | 5.82 | 5.85* |
| Potency² (Assay) | 102.6% | 102.9% | 101.5% | 102.2% |

| Related Substances³ (% Area) | | | | |
|---|---|---|---|---|
| RRT 0.52 | ND | 0.05% | ND | |
| Tropic Acid¹ | 0.06% | 0.08% | 0.09% | 0.10% |
| Total Related Substances | 0.1% | 0.1% | 0.1% | 0.1% |

| BAK Impurities % Area⁴ | | | | |
|---|---|---|---|---|
| RRT 0.97 | ND | ND | 0.13% | ND |
| RRT 1.05 | 0.88% | 0.83% | 0.69% | 0.64% |
| RRT 1.27 - 1.29 | 0.14% | 0.12% | 0.15% | 0.11% |
| RRT 1.51-1.53 | 0.13% | 0.08% | 0.05% | 0.07% |

| | | | | |
|---|---|---|---|---|
| * Indicates D₂O formulation ¹ Tropic acid identified by retention time marker at each time point ³90.0-110.0% of label claim ³Report unknown related substances ≥ 0.05% area ⁴Report related substances ≥ 0.05% area | | | | |

**Table 15A - Atropine solutions at pH 5.4 and pH 5.6 and storage temperature at 25°C / 60% RH**

| **Parameter** | **Time Point** | | | | | | | |
|---|---|---|---|---|---|---|---|---|
| | **Initial** | **T=1 Month** | **T=3 Months** | **T=6 Months** | **Initial** | **T=1 Month** | **T=3 Months** | **T=6 Months** |
| pH | 5.35* | 5.41* | 5.40* | 5.33 | 5.58* | 5.51* | 5.52* | 5.47 |
| Weight Monitoring² | NA | -13.2 | -18.7 | -37.1 | NA | -11.9 | -29.2 | -60.8 |
| Potency³ (Assay) | 102.3% | 104.5% | 101.1% | 98.8% | 102.5 % | 104.4% | 101.1% | 98.7% |

| Related Substances⁴ (% Area) | | | | | | | | |
|---|---|---|---|---|---|---|---|---|
| RRT 0.51-0.52 | ND | 0.08% | 0.08% | ND | ND | ND | ND | ND |
| Tropic Acid¹ | 0.02% | 0.17% | 0.32% | 0.60% | 0.04% | 0.24% | 0.45% | 0.83% |
| RRT 1.21 | 0.10% | ND | ND | ND | 0.10% | ND | ND | ND |
| Total Related Substances | 0.1% | 0.3% | 0.4% | 0.6% | 0.1% | 0.2% | 0.5% | 0.8% |

| BAK Impurities % Area⁵ | | | | | | | | |
|---|---|---|---|---|---|---|---|---|
| RRT 0.98 | ND | ND | ND | 0.11% | ND | ND | ND | 0.12% |
| RRT 1.05 | 0.75% | 0.79% | 0.63% | 0.38% | 0.75% | 0.81% | 0.65% | 0.38% |
| RRT 1.28 - 1.29 | 0.13% | 0.15% | 0.14% | 0.15% | 0.15% | 0.13% | 0.14% | 0.15% |
| RRT 1.53 | 0.19% | ND | ND | ND | 0.18% | ND | ND | ND |

| | | | | | | | | |
|---|---|---|---|---|---|---|---|---|
| * Indicates D₂O formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area | | | | | | | | |

**Table 15B - Atropine solutions at pH 5.8 and pH 6 and storage temperature at 25°C / 60% RH**

| **Parameter** | **Time Point** | | | | | | | |
|---|---|---|---|---|---|---|---|---|
| | **Initial** | **T=1 Month** | **T=3 Months** | **T=6 Months** | **Initial** | **T=1 Month** | **T=3 Months** | **T=6 Months** |
| pH | 5.83* | 5.78* | 5.79* | 5.79 | 5.99* | 6.01* | 6.01* | 5.96 |
| Weight Monitoring² | NA | -12.7 | -41.4 | -38.5 | NA | -7.7 | -17.1 | -41.1 |
| Potency³ (Assay) | 102.5 % | 104.3% | 101.0% | 97.9% | 102.6 % | 104.1% | 100.2% | 97.3% |

| Related Substances⁴ (% Area) | | | | | | | | |
|---|---|---|---|---|---|---|---|---|
| Tropic Acid¹ | 0.05% | 0.38% | 0.82% | 1.58% | 0.06% | 0.56% | 1.28% | 2.56% |
| RRT 0.84 | ND | ND | ND | ND | ND | 0.13% | ND | ND |
| RRT 1.21 | 0.11% | ND | ND | ND | 0.09% | ND | ND | ND |
| RRT 1.55 | ND | ND | ND | 0.06% | ND | ND | ND | 0.08% |
| Total Related Substances | 0.2% | 0.4% | 0.8% | 1.6% | 0.2% | 0.7% | 1.3% | 2.6% |

| BAK Impurities % Area⁵ | | | | | | | | |
|---|---|---|---|---|---|---|---|---|
| RRT 0.98 | ND | ND | ND | 0.12% | ND | ND | ND | 0.11% |
| RRT 1.05 | 0.74% | 0.81% | 0.65% | 0.38% | 0.75% | 0.79% | 0.64% | 0.38% |
| RRT 1.28 - 1.29 | 0.14% | 0.14% | 0.13% | 0.16% | 0.15%^{#} | 0.15%^{#} | 0.13%^{#} | 0.15%^{#} |
| RRT 1.53 | 0.20% | ND | ND | ND | 0.19%^{#} # | ND | ND | ND |

| | | | | | | | | |
|---|---|---|---|---|---|---|---|---|
| * Indicates D₂O formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area ^{#}RRT 1.27- 1.29 ^{##}RRT 1.53-1.55 | | | | | | | | |

**Table 15C - Atropine solutions at pH 6.2 and pH 6.4 and storage temperature at 25°C / 60% RH**

| **Parameter** | Time Point | | | | | | | |
|---|---|---|---|---|---|---|---|---|
| | **Initial** | **T=1 Month** | **T=3 Months** | **T=6 Months** | **Initial** | **T=1 Month** | **T=3 Months** | **T=6 Months** |
| pH | 6.25* | 6.20* | 6.16* | 6.08 | 6.41***** | 6.42* | 6.41***** | 6.4 |
| Weight Monitoring² | NA | -6.4 | -13.7 | -38.2 | NA | -8.1 | -15.9 | -44.9 |
| Potency³ (Assay) | 102.5 % | 103.9% | 99.6% | 96.2% | 102.6% | 102.8% | 98.1% | 92.9% |

| Related Substances⁴ (% Area) | | | | | | | | |
|---|---|---|---|---|---|---|---|---|
| Tropic Acid¹ | 0.17% | 0.87% | 1.93% | 3.63% | 0.10% | 1.27% | 3.23% | 6.34% |
| RRT 0.84 | ND | ND | ND | ND | ND | 0.05% | ND | ND |
| RRT 1.21 | 0.09% | ND | ND | ND | 0.10% | ND | ND | ND |
| RRT 1.55 | ND | ND | 0.06% | 0.10% | ND | ND | 0.07% | 0.14% |
| Total Related Substances | 0.3% | 0.9% | 2.0% | 3.7% | 0.2% | 1.3% | 3.3% | 6.5% |

| BAK Impurities % Area⁵ | | | | | | | | |
|---|---|---|---|---|---|---|---|---|
| RRT 0.98 | ND | ND | ND | 0.15% | ND | ND | ND | 0.10% |
| RRT 1.05 | 0.76% | 0.79% | 0.66% | 0.38% | 0.76% | 0.79% | 0.66% | 0.39% |
| RRT 1.27 | ND | ND | ND | ND | ND | ND | ND | 0.15% |
| RRT 1.28 - 1.29 | 0.14% | 0.14% | 0.14% | 0.15% | 0.13%^{#} | 0.13%^{#} | 0.14%^{#} | 0.15%^{#} |
| RRT 1.53 | 0.21% | ND | ND | ND | 0.23%^{#} # | ND | ND | ND |

| | | | | | | | | |
|---|---|---|---|---|---|---|---|---|
| * Indicates D₂O formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area ^{#}RRT 1.27- 1.29 ^{##}RRT 1.53-1.55 | | | | | | | | |

**Table 15D - Atropine solutions at pH 5.8 in a spray bottle storage container and storage temperature at 25°C / 60% RH**

| **Parameter** | **Time Point** | | | |
|---|---|---|---|---|
| | **Initial** | **T=2 Months** | **T=5 Months** | **T=8 Months** |
| pH* | 5.80* | 5.89* | 5.79* | 5.72* |
| Potency² (Assay) | 102.6% | 101.4% | 107.0% | 102.1% |

| Related Substances³ (% Area) | | | | |
|---|---|---|---|---|
| RRT 0.51-0.52 | ND | 0.05% | 2.06% | ND |
| Tropic Acid¹ | 0.06% | 0.54% | 1.38% | 2.22% |
| RRT 1.09 | ND | ND | ND | 0.06% |
| RRT 1.31 | ND | 0.06% | ND | ND |
| RRT 1.55 | ND | ND | 0.07% | 0.07% |
| Total Related Substances | 0.1% | 0.6% | 1.5% | 2.4% |

| BAK Impurities % Area⁴ | | | | |
|---|---|---|---|---|
| RRT 0.98 | ND | ND | 0.18% | ND |
| RRT 1.05 | 0.88% | 0.83% | 0.48% | 0.73% |
| RRT 1.27 - 1.29 | 0.14% | 0.15% | 0.17% | 0.15% |
| RRT 1.53 | 0.13% | ND | ND | ND |

| | | | | |
|---|---|---|---|---|
| * Indicates D₂O formulation ¹ Tropic acid identified by retention time marker at each time point ³90.0-110.0% of label claim ³Report unknown related substances ≥ 0.05% area ⁴Report related substances ≥ 0.05% area | | | | |

**Table 16A - Atropine solutions at pH 5.4 and pH 5.6 and storage temperature at 40°C / 75% RH**

| **Parameter** | **Time Point** | | | | | | | |
|---|---|---|---|---|---|---|---|---|
| | **Initial** | **T=1 Month** | **T=3 Months** | **T=6 Months** | **Initial** | **T=1 Month** | **T=3 Months** | **T=6 Months** |
| pH | 5.35* | 5.40* | 5.39* | 5.3 | 5.58* | 5.52* | 5.50* | 5.40 |
| Weight Monitoring² | NA | -11.4 | NA | -95.9 | NA | -13.9 | -77.0 | -59.2 |
| Potency³ (Assay) | 102.3% | 104.0% | 102.4% | 97.1% | 102.5 % | 103.7% | 99.1% | 95.7% |

| Related Substances⁴ (% Area) | | | | | | | | |
|---|---|---|---|---|---|---|---|---|
| RRT 0.50-0.52 | ND | 0.09% | 0.08% | ND | ND | ND | ND | ND |
| RRT 0.63 | ND | ND | ND | ND | ND | 0.11% | ND | ND |
| Tropic Acid¹ | 0.02% | 0.64% | 1.68% | 3.36% | 0.04% | 0.95% | 2.42% | 4.85% |
| RRT 1.21-1.22 | 0.10% | ND | 0.08% | 0.11% | 0.10% | ND | 0.06% | 0.10% |
| RRT 1.31 | ND | ND | ND | ND | ND | ND | ND | 0.16% |
| RRT 1.36 | ND | ND | ND | ND | ND | 0.13% | ND | ND |
| RRT 1.46 | ND | 0.07% | 0.05% | ND | ND | 0.06% | 0.05% | ND |
| RRT 1.53-1.55 | ND | 0.06% | 0.18% | 0.40% | ND | 0.06% | 0.20% | 0.46% |
| Total Related Substances | 0.1% | 0.9% | 2.1% | 3.9% | 0.1% | 1.3% | 2.7% | 5.6% |

| BAK Impurities % Area⁵ | | | | | | | | |
|---|---|---|---|---|---|---|---|---|
| RRT 0.98 | ND | ND | ND | 0.12% | ND | ND | ND | 0.12% |
| RRT 1.05 | 0.75% | 0.51% | 0.22% | ND | 0.75% | 0.52% | 0.23% | ND |
| RRT 1.27 - 1.29 | 0.13% | 0.12% | 0.15% | 0.16% | 0.15% | 0.13% | 0.12% | 0.16% |
| RRT 1.50 - 1.53 | 0.19% | ND | ND | 0.06% | 0.18%^{#} | ND | ND | 0.06%^{#} |

| | | | | | | | | |
|---|---|---|---|---|---|---|---|---|
| * Indicates **D₂O** formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area ^{#}RRT 1.55 | | | | | | | | |

**Table 16B - Atropine solutions at pH 5.8 and pH 6 and storage temperature at 40°C / 75% RH**

| **Parameter** | **Time Point** | | | | | | | |
|---|---|---|---|---|---|---|---|---|
| | **Initial** | **T=1 Month** | **T=3 Months** | **T=6 Months** | **Initial** | **T=1 Month** | **T=3 Months** | **T=6 Months** |
| pH | 5.83* | 5.81* | 5.75* | 5.68 | 5.99* | 5.98* | 5.99* | 5.84 |
| Weight Monitoring² | NA | -14.5 | -266.2 | -66.9 | NA | -11.9 | -40.1 | -61.9 |
| Potency³ (Assay) | 102.5% | 102.4% | 102.1% | 91.7% | 102.6 % | 101.6% | 94.3% | 86.9% |

| Related Substances⁴ (% Area) | | | | | | | | |
|---|---|---|---|---|---|---|---|---|
| RRT 0.59 | ND | ND | ND | ND | ND | 0.07% | ND | ND |
| Tropic Acid¹ | 0.05% | 1.78% | 4.44% | 8.95% | 0.06% | 2.82% | 7.18% | 13.46% |
| RRT 1.21-1.22 | 0.11% | ND | 0.07% | 0.09% | 0.09% | ND | 0.07% | 0.10% |
| RRT 1.46 | ND | 0.07% | 0.05% | ND | ND | 0.05% | 0.05% | ND |
| RRT 1.50 | ND | ND | ND | 0.06% | ND | ND | ND | ND |
| RRT 1.55-1.56 | ND | 0.10% | 0.28% | 0.56% | ND | 0.14% | 0.36% | 0.65% |
| Total Related Substances | 0.2% | 2.0% | 4.8% | 9.7% | 0.2% | 3.1% | 7.7% | 14.2% |

| BAK Impurities % Area⁵ | | | | | | | | |
|---|---|---|---|---|---|---|---|---|
| RRT 0.98 | ND | ND | ND | 0.12% | ND | ND | ND | 0.11% |
| RRT 1.05 | 0.74% | 0.51% | 0.22% | ND | 0.75% | 0.52% | 0.22% | ND |
| RRT 1.27 - 1.29 | 0.14% | 0.13% | 0.14% | 0.16% | 0.15% | 0.15% | 0.14% | 0.16% |
| RRT 1.50-1.53 | 0.20% | ND | 0.05% | 0.06% | 0.19% | ND | 0.06% | 0.05% |

| | | | | | | | | |
|---|---|---|---|---|---|---|---|---|
| * Indicates D₂O formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area | | | | | | | | |

**Table 16C - Atropine solutions at pH 6.2 and pH 6.4 and storage temperature at 40°C / 75% RH**

| **Parameter** | **Time Point** | | | | | | | |
|---|---|---|---|---|---|---|---|---|
| | **Initial** | **T=1 Month** | **T=3 Months** | **T=6 Months** | **Initia l** | **T=1 Month** | **T=3 Months** | **T=6 Months** |
| pH | 6.25* | 6.18* | 6.09* | 5.79 | 6.41* | 6.44* | 6.27* | 6.11 |
| Weight Monitoring² | NA | -12.0 | -28.1 | -55.8 | NA | -12.8 | -27.6 | -55.4 |
| Potency³ (Assay) | 102.5 % | 100.1% | 91.3% | 85.2% | 102.6 % | 97.1% | 83.9% | 71.8% |

| Related Substances⁴ (% Area) | | | | | | | | |
|---|---|---|---|---|---|---|---|---|
| RRT 0.34 | ND | ND | ND | 0.09% | ND | ND | ND | ND |
| Tropic Acid¹ | 0.17% | 4.20% | 10.23% | 14.87% | 0.10 % | 6.98% | 16.55% | 28.06% |
| RRT 0.90 | ND | ND | ND | 0.06% | ND | ND | ND | ND |
| RRT 1.21-1.22 | 0.09% | ND | 0.08% | 0.12% | 0.10 % | ND | 0.06% | 0.10% |
| RRT 1.35 | ND | ND | ND | ND | ND | ND | ND | 0.05% |
| RRT 1.46 | ND | 0.07% | 0.05% | ND | ND | 0.07% | ND | ND |
| RRT 1.55-1.56 | 0.21% | 0.17% | 0.43% | 0.64% | ND | 0.24% | 0.55% | 0.92% |
| RRT 1.77 | ND | ND | ND | 0.15% | ND | ND | ND | ND |
| Total Related Substances | 0.5% | 4.4% | 10.8% | 15.8% | 0.2% | 7.3% | 17.2% | 29.1% |

| BAK Impurities % Area⁵ | | | | | | | | |
|---|---|---|---|---|---|---|---|---|
| RRT 0.97 | ND | ND | ND | 0.26% | ND | ND | ND | 0.12% |
| RRT 1.05 | 0.76% | 0.50% | 0.23% | ND | 0.76 % | 0.50% | 0.25% | ND |
| RRT 1.28 - 1.29 | 0.14% | 0.13% | 0.14% | 0.18% | 0.13 % | 0.14% | 0.14% | 0.14% |
| RRT 1.50-1.53 | 0.21% | ND | 0.05% | 0.06% | 0.23 % | ND | 0.06% | 0.06% |

| | | | | | | | | |
|---|---|---|---|---|---|---|---|---|
| * Indicates D₂O formulation ¹ Tropic acid identified by retention time marker at each time point ²Report delta weight of bottle (mg) ³90.0-110.0% of label claim ⁴Report unknown related substances ≥ 0.05% area ⁵Report related substances ≥ 0.05% area | | | | | | | | |

**Table 16D - Atropine solutions at pH 5.8 in a spray bottle storage container and storage temperature at 40°C / 75% RH**

| **Parameter** | **Time Point** | | |
|---|---|---|---|
| | **Initial** | **T=2 Months** | **T=5 Months** |
| pH* | 5.80* | 5.80* | 5.77 |
| Potency² (Assay) | 102.6% | 99.7% | 95.8% |

| Related Substances³ (% Area) | | | |
|---|---|---|---|
| RRT 0.52 | ND | 0.05% | ND |
| Tropic Acid¹ | 0.06% | 3.25% | 8.94% |
| RRT 0.79 | ND | ND | 0.09% |
| RRT 1.10 | ND | 0.06% | ND |
| RRT 1.31 | ND | 0.08% | ND |
| RRT 1.55 | ND | 0.20% | 0.48% |
| Total Related Substances | 0.1% | 3.6% | 9.0% |

| BAK Impurities % Area⁴ | | | |
|---|---|---|---|
| RRT 1.05 | 0.88% | 0.80% | 0.41% |
| RRT 1.28 - 1.29 | 0.14% | 0.14% | 0.09% |
| RRT 1.53 | 0.13% | ND | ND |

| | | | |
|---|---|---|---|
| * Indicates D₂O formulation ¹ Tropic acid identified by retention time marker at each time point ³90.0-110.0% of label claim ³Report unknown related substances ≥ 0.05% area ⁴Report related substances ≥ 0.05% area | | | |

### Reference Example 5 - Preparation of an Aqueous Solution Formulation Containing 0.01% an ophthalmic agent in D₂O

### Stock 1% Solution

In a 100mL solution, 1 gram of an ophthalmic agent, and 0.77 g of NaCl (and other ingredients/components preferably in their dry state) are added along with a quantity sufficient to equal 100mL sterile deuterated water for injection. The solution is mixed in an appropriately sized beaker with a stir bar on a hot plate until all of the solid powders have dissolved and the solution has become clear with no visible particles. Next, the stir bar is removed, and the solution is poured into a filter bottle and vacuum filtered through a 0.22 micron polyethersulfone membrane filter into a sterile bottle. The filter top is removed from the sterile stock bottle and the stock bottle is capped for storage with a sterile bottle cap.

### Diluted 0.01% Solution

0.3mL of the 1% solution is combined with a quantity sufficient to achieve 30mL total of sterile 0.9% Sodium Chloride For Injection USP. The solution is thoroughly mixed. The pD of the solution is recorded. A 0.22 micron filter is placed on the tip of the syringe and the solution is aliquoted into separate sterile containers

### Example 6 - Dose Uniformity (10-Dose) - Preservative Free Fluid-Dispensing Device

To evaluate the dose-to-dose uniformity, preservative free fluid-dispensing devices containing the ophthalmic aqueous composition are stored upright for a predetermined period of time (e.g. 12 hours) prior to the start of the test. To simulate the recommended dosing of the product, 10 drops of the aqueous composition are dispensed from each device at predetermined time intervals (e.g. consecutively, every 1 minute, every 10 minutes, every hour or every 24 hours). All drops are dispensed into tared glass vials, capped, and stored at room temperature until analysis. Concentrations of atropine in the expressed drops are determined using a reverse-phase HPLC method.

### Example 7 - Dose Uniformity (10-Dose) - Fluid-Dispensing Device Comprising an Internal Filter

To evaluate the dose-to-dose uniformity, fluid-dispensing devices containing the ophthalmic aqueous composition are stored upright for a predetermined period of time (e.g. 12 hours) prior to the start of the test. Each of the fluid-dispensing devices comprises an internal filter. To simulate the recommended dosing of the product, 5 drops of the aqueous composition are dispensed from each device at predetermined time intervals (e.g. consecutively, every 1 minute, every 10 minutes, every hour or every 24 hours). All drops are dispensed into tared glass vials, capped, and stored at room temperature until analysis. Concentrations of atropine in the expressed drops are determined using a reverse-phase HPLC method.

### Example 8 - Effect of pH on Ophthalmic Acceptance in Guinea Pigs

A cohort of guinea pigs is administered 50 µL of preservative-free ophthalmic formulations having different pH values described herein. For example, ophthalmic formulations comprising H₂O or deuterated water (e.g., D₂O) are administered to the animals. Animal behavior is recorded at predetermined time intervals to evaluate the acceptance of the ophthalmic formulations

### Example 9 - In vivo Rabbit Eye Irritation Test

The exemplary compositions disclosed herein are subjected to rabbit eye irritation test to evalaute their safety profile. The test composition are tested for eye irritation test in New Zealand Rabbits (see for example Abraham M H, et al., Draize rabbit eye test compatibility with eye irritation thresholds in humans: a quantitative structure-activity relationship analysis. Toxicol Sci. 2003 December; 76(2):384-91. Epub 2003 Sep. 26; see also Gettings S D et al., A comparison of low volume, Draize and in vitro eye irritation test data. III. Surfactant-based formulations. Food Chem Toxicol. 1998 March; 36(3):209-31). The study involves single ocular administration into the right eye and the same volume of its placebo in the left eye of each of the three rabbits. Rabbits are examined immediately and after instillation of the compositions for 4, 24, 48 and 72 hours post instillation to note the signs/symptoms of eye irritation, if any. The test compositions show no sign of irritancy in cornea, iris and conjunctivae of the rabbit eyes.

### Example 10 - In vivo Testing of Ophthalmic Aqueous Formulation in Guinea Pigs

Focus deprivation myopia (FDM) is achieved using a latex shield to cover one eye. For defocus-induced myopia, a latex-made facemask was held in place by a rubber-band around the head of animals, leaving both eyes, the nose, mouth and ears freely exposed. A - 4.00 D lens is glued onto a plastic lens frame. The lens frame is then attached to the facemask around one eye by a fabric hook- and-loop fastener after the optical center of the lens was aligned with the pupil center. The lens is detached and cleaned on both sides with a water-wetted gauze at least once daily followed by reattachment to the facemask. All the animals are maintained on a cycle of 12-h illumination (500 Lux) and 12-h darkness during the experimental period

A cohort of guinea pigs at age of 3 weeks are randomly assigned to FDM (a facemask worn monocularly) or defocus-induced myopia (a -4.00 D lens worn monocularly) and control groups. The FDM groups were treated with the ophthalmic aqueous formulation, the ophthalmic carrier (without the opthalmic agent), or FDM-only. The defocus-induced myopia groups were treated with the ophthalmic aqueous formulation, the ophthalmic carrier (without the opthalmic agent), or defocus-only. The control groups were treated with the ophthalmic aqueous formulation, the ophthalmic carrier (without the opthalmic agent), or no treatment. Ocular biometric parameters are measured in both eyes of individual animals before and at 11 days of treatment

Biometric parameters (e.g. refraction, corneal curvature, and axial components of the eye) are measured by an optometrist, orthoptist, or ophthamologist with help from an animal care assistant during the light cycle (daytime) after removal of the facemask or lens. The optometrist, orthoptist, or ophthamologist is masked in regard to the treatment conditions for each animal.

Refraction is measured by retinoscopy after the pupil is completely dilated by topical administration of 1% cyclopentolate hydrochloride. The results of retinoscopy are recorded as the mean value of the horizontal and vertical meridians.

Corneal curvature is measured with a keratometer modified by attachment of an +8 D lens onto the anterior surface of the keratometer. A group of stainless steel balls with diameters from 5.5 to 11.0 mm are measured by the modified keratometer. Three readings are recorded for each measurement to provide a mean result. The radius of corneal curvature is then deduced from the readings on the balls with known radii.

A-scan ultrasonagraph is used to measure axial components of the eye (lens thickness and vitreous length and axial length). The conducting velocity was 1,723.3 m/s for measurement of the lens thickness and 1,540 m/s for measurement of the vitreous length as described previously. Each of the axial components is calculated as the mean of 10 repeated measurements.

### Example 11 - Safety and Efficacy Studies of Ophthalmic Aqueous Formulation

A clinical trial is performed to investigate the efficacy and safety of ophthalmic aqueous formulations described herein in patents with myopia. In some instances, the study is open-label, single blind, or double blind study. Patient selection criteria include myopic refraction of at least 1.0D in both eyes, and additional factors such as astigmatism, a documented myopic progression, age, sex, and/or health conditions.

The patients are randomized to receive 0.05%, 0.01%, or 0.001 atropine aqueous formulation formulated in either H₂O or deuterated water (e.g., D₂O) once nightly in both eyes. Allocation ratio in some instances is defined based the patient population.

The patients are evaluated on day 0 (baseline), day 14, day 30, and then at 2, 3, 4, 5, 6, 8, 10, 12, 18, 20, 24, and 36 months. At each visit, best-coorected distance logMar visual acuity (BCVA) is assessed by an optometrist, orthoptist, or ophthamologist using the Early Treatment Diabetic Retinopathy study chart. Near visual acuity is assessed using best-corrected distance spectable correction with a reduced logMar reading chart placed at 40cm under well-lit conditions. The near point of accommodation (NPA) is measured using a RAF rule using best-corrected distance spectable correction. Patients are instructed to move the target inwards till the N5 print becomes slightly blurred and then outwards till it just becomes clear. Accommodation amplitude is calculated as the inverse of NPA. Mesopic pupil size is measured with Procyon 3000 pupillometer. Photopic pupil size is measured using the Neuroptics pupillometer.

Cycloplegic autorefraction is determined 30 minutes after 3 drops of cyclopentolate 1% are administered at 5 minutes apart using a Canon RK-F1 autorefractor. A Zeiss IOL Master, a noncontact partial coherence interferometry, is used to measure the ocular axial length.

The primary outcome is myopia progression over the time period of the study. Safety is assessed by adverse events including allergic reactions, irritation, or development of blurring of vision in one or both eyes.

### Reference Example 12 - Preparation of an Ointment Formulation Containing Atropine Sulfate

Atropine sulfate is mixed with the dispersing agent (e.g. polyethyleneglycol) under heating and sonication and this mixture is further thoroughly mixed with a molten ointment base (e.g. a mixture of wool wax, white petrolatum, and liquid paraffin). The mixture is placed in a pressure vessel, and sterilized at 125 °C for 30-45 minutes and cooled to room temperature. In another instance, autoclaving is conducted under nitrogen. The resulting ophthalmic ointment is aseptically filled into pre-sterilized containers (e.g. tubes).

### Example 13 - Atropine-Mucus Penetrating Particle Composition

A 0.01% atropine-mucus penetrating particle composition was prepared utilizing a milling procedure. An aqueous dispersion containing atropine particles and an MPP-enabling mucus penetrating agent was milled with grinding medium until particle size was reduced to approximately 200nm with a polydispersity index less than 0.15 as measured by dynamic light scattering. Additional agents such as preservatives are also added during the milling procedure. Subsequently, the atropine-MPP composition is stored at temperatures of between about 15°C and about 25°C.

### Example 14 - Atropine Sulfate-Mucus Penetrating Particle Composition

A 0.01% atropine sulfate-mucus penetrating particle composition was prepared utilizing a milling procedure. An aqueous dispersion containing atropine particles and an MPP-enabling mucus penetrating agent was milled with grinding medium until particle size was reduced to approximately 200nm with a polydispersity index less than 0.15 as measured by dynamic light scattering. Additional agents such as preservatives are also added during the milling procedure. Subsequently, the atropine-MPP composition is stored at temperatures of between about 15°C and about 25°C.

## Claims

1. An ophthalmic product, comprising:
a) a fluid-dispensing device comprising an internal filter or membrane, a reservoir and a dispensing tip fitted onto the reservoir; and
b) an ophthalmic composition comprising from about 0.001 wt% to about 0.05 wt% of a muscarinic antagonist and deuterated water, at a pD of from about 4.2 to about 7.9, in the reservoir;
wherein the muscarinic antagonist is atropine or atropine sulfate;
wherein the ophthalmic composition is dispensable from the dispensing tip into an eye of an individual in need thereof, and wherein the dispensed ophthalmic composition is preservative-free.

2. The ophthalmic product of claim 1, wherein the ophthalmic composition has a pD of one of: less than 7.3, less than 7.2, less than 7.1, less than 7, less than 6.8, less than 6.5, less than 6.4, less than 6.3, less than 6.2, less than 6.1, less than 6, less than 5.9, less than 5.8, less than 5.2, or less than 4.8 after an extended period of time under storage condition.

3. The ophthalmic product of claim 1, wherein the ophthalmic composition comprises one of: less than 1%, less than 0.5%, less than 0.4%, less than 0.3%,
less than 0.2%, less than 0.1 %, less than 0.01%, or less than 0.001% of a preservative.

4. The ophthalmic product of claim 1, wherein the ophthalmic composition is preservative-free.

5. The ophthalmic product of claim 1, wherein the internal filter or membrane is located within the fluid-dispensing device at a position capable of removing a preservative from the ophthalmic composition prior to dispensing the ophthalmic composition into the eye of the individual.

6. The ophthalmic product of claim 1, wherein the internal filter or membrane is located within the fluid-dispensing device at a position capable of removing a microorganism and/or an endotoxin from the ophthalmic composition prior to dispensing the ophthalmic composition into the eye of the individual.

7. The ophthalmic product of claim 1, wherein the internal filter or membrane comprises cellulose acetate, cellulose nitrate, nylon, polyether sulfone (PES), polypropylene (PP), polyvinyl difluoride (PVDF), silicone, polycarbonate, or a combination thereof.

8. The ophthalmic product of claim 1, wherein the ophthalmic composition comprises one of: at least 80 %, at least 85 %, at least 90 %, at least 93 %, at least 95 %, at least 97 %, at least 98 %, or at least 99 % of the muscarinic antagonist based on initial concentration after extended period of time under storage condition.

9. The ophthalmic product of claim 1, wherein the ophthalmic composition has a potency of one of: at least 80 %, at least 85 %, at least 90 %, at least 93 %, at least 95 %, at least 97 %, at least 98 %, or at least 99 % after extended period of time under storage condition.

10. The ophthalmic product of any one of claims 2, 8, and 9, wherein the extended period of time is one of: about 1 week, about 2 weeks, about 3 weeks, about 1 month, about 2 months, about 3 months, about 4 months, about 5 months, about 6 months, about 8 months, about 10 months, about 12 months, about 18 months, about 24 months, about 36 months, about 4 years, or about 5 years.

## Patentansprüche

1. Ophthalmisches Produkt, das Folgendes umfasst:
a) eine Fluid-Abgabevorrichtung, die einen internen Filter oder eine interne Membran, einen Vorratsbehälter und eine auf den Vorratsbehälter aufgesetzte Abgabespitze umfasst, und
b) eine ophthalmische Zusammensetzung, die etwa 0,001 Gew.-% bis etwa 0,05 Gew.-% eines Muskarinrezeptor-Antagonisten und deuteriertes Wasser mit einem pD von etwa 4,2 bis etwa 7,9 im Vorratsbehälter;
wobei der Muskarinrezeptor-Antagonist Atropin oder Atropinsulfat ist;
wobei die ophthalmische Zusammensetzung aus der Abgabespitze in ein Auge eines bedürftigen Individuums abgegeben werden kann und wobei die abgegebene ophthalmische Zusammensetzung frei von Konservierungsmitteln ist.

2. Ophthalmisches Produkt nach Anspruch 1, wobei die ophthalmische Zusammensetzung nach einem längeren Zeitraum unter Lagerbedingungen einen der folgenden pD aufweist: weniger als 7,3, weniger als 7,2, weniger als 7,1, weniger als 7, weniger als 6,8, weniger als 6,5, weniger als 6,4, weniger als 6,3, weniger als 6,2, weniger als 6,1, weniger als 6, weniger als 5,9, weniger als 5,8, weniger als 5,2 oder weniger als 4,8.

3. Ophthalmisches Produkt nach Anspruch 1, wobei die ophthalmische Zusammensetzung eines der Folgenden umfasst: weniger als 1 %, weniger als 0,5 %, weniger als 0,4 %, weniger als 0,3 %, weniger als 0,2 %, weniger als 0,1 %, weniger als 0,01 % oder weniger als 0,001 % eines Konservierungsmittels.

4. Ophthalmisches Produkt nach Anspruch 1, wobei die ophthalmische Zusammensetzung frei von Konservierungsmitteln ist.

5. Ophthalmisches Produkt nach Anspruch 1, wobei sich der interne Filter oder die interne Membran innerhalb der Fluid-Abgabevorrichtung an einer Stelle befindet, die es ermöglicht, dass ein Konservierungsmittel aus der ophthalmischen Zusammensetzung entfernt werden kann, bevor die ophthalmische Zusammensetzung in das Auge des Individuums abgegeben wird.

6. Ophthalmisches Produkt nach Anspruch 1, wobei sich der interne Filter oder die interne Membran innerhalb der Fluid-Abgabevorrichtung an einer Stelle befindet, die es ermöglicht, dass ein Mikroorganismus und/oder Endotoxin aus der ophthalmischen Zusammensetzung entfernt werden können, bevor die ophthalmische Zusammensetzung in das Auge des Individuums abgegeben wird.

7. Ophthalmisches Produkt nach Anspruch 1, wobei der interne Filter oder die interne Membran Celluloseacetat, Cellulosenitrat, Nylon, Polyethersulfon (PES), Polypropylen (PP), Polyvinyldifluorid (PVDF), Silikon, Polycarbonat oder eine Kombination davon umfasst.

8. Ophthalmisches Produkt nach Anspruch 1, wobei die ophthalmische Zusammensetzung bezogen auf die Anfangskonzentration nach einem längeren Zeitraum unter Lagerbedingungen eines der Folgenden umfasst: zumindest 80 %, zumindest 85 %, zumindest 90 %, zumindest 93 %, zumindest 95 %, zumindest 97 %, zumindest 98 % oder zumindest 99 % des Muskarinrezeptor-Antagonisten.

9. Ophthalmisches Produkt nach Anspruch 1, wobei die ophthalmische Zusammensetzung nach einem längeren Zeitraum unter Lagerbedingungen eine der folgenden Wirkstärken aufweist: zumindest 80 %, zumindest 85 %, zumindest 90 %, zumindest 93 %, zumindest 95 %, zumindest 97 %, zumindest 98 % oder zumindest 99 %.

10. Ophthalmisches Produkt nach einem der Ansprüche 2, 8 und 9, wobei der längere Zeitraum einer der folgenden ist: etwa 1 Woche, etwa 2 Wochen, etwa 3 Wochen, etwa 1 Monat, etwa 2 Monate, etwa 3 Monate, etwa 4 Monate, etwa 5 Monate, etwa 6 Monate, etwa 8 Monate, etwa 10 Monate, etwa 12 Monate, etwa 18 Monate, etwa 24 Monate, etwa 36 Monate, etwa 4 Jahre oder etwa 5 Jahre.

## Revendications

1. Produit ophtalmique, comprenant :
a) un dispositif de distribution de fluide comprenant un filtre ou une membrane interne, un réservoir et un embout de distribution monté sur le réservoir ; et
b) une composition ophtalmique comprenant d'environ 0,001 % en poids à environ 0,05 % en poids d'un antagoniste muscarinique et d'eau deutérée, à un pD d'environ 4,2 à environ 7,9, dans le réservoir ;
dans lequel l'antagoniste muscarinique est l'atropine ou le sulfate d'atropine ;
dans lequel la composition ophtalmique peut être distribuée à partir de l'embout de distribution dans un œil d'un individu qui en a besoin, et dans lequel la composition ophtalmique distribuée est sans conservateur.

2. Produit ophtalmique selon la revendication 1, dans lequel la composition ophtalmique présente un pD : inférieur à 7,3, inférieur à 7,2, inférieur à 7,1, inférieur à 7, inférieur à 6,8, inférieur à 6,5, inférieur à 6,4, inférieur à 6,3, inférieur à 6,2, inférieur à 6,1, inférieur à 6, inférieur à 5,9, inférieur à 5,8, inférieur à 5,2 ou inférieur à 4,8 après une période de temps prolongée dans des conditions de stockage.

3. Produit ophtalmique selon la revendication 1, dans lequel la composition ophtalmique comprend : moins de 1 %, moins de 0,5 %, moins de 0,4 %, moins de 0,3 %, moins de 0,2 %, moins de 0,1 %, moins de 0,01 % ou moins de 0,001 % d'un conservateur.

4. Produit ophtalmique selon la revendication 1, dans lequel la composition ophtalmique est exempte de conservateur.

5. Produit ophtalmique selon la revendication 1, dans lequel le filtre ou la membrane interne est situé(e) à l'intérieur du dispositif de distribution de fluide dans une position capable d'éliminer un conservateur de la composition ophtalmique avant de distribuer la composition ophtalmique dans l'œil de l'individu.

6. Produit ophtalmique selon la revendication 1, dans lequel le filtre ou la membrane interne est situé(e) à l'intérieur du dispositif de distribution de fluide dans une position capable d'éliminer un micro-organisme et/ou une endotoxine à partir de la composition ophtalmique avant de distribuer la composition ophtalmique dans l'œil de l'individu.

7. Produit ophtalmique selon la revendication 1, dans lequel le filtre ou la membrane interne comprend de l'acétate de cellulose, du nitrate de cellulose, du nylon, de la polyéther sulfone (PES), du polypropylène (PP), du difluorure de polyvinyle (PVDF), de la silicone, du polycarbonate, ou une combinaison de ceux-ci.

8. Produit ophtalmique selon la revendication 1, dans lequel la composition ophtalmique comprend au moins 80 %, au moins 85 %, au moins 90 %, au moins 93 %, au moins 95 %, au moins 97 %, au moins 98 % ou au moins 99 % de l'antagoniste muscarinique sur la base de la concentration initiale après une période de temps prolongée dans des conditions de stockage.

9. Produit ophtalmique selon la revendication 1, dans lequel la composition ophtalmique présente une activité de : au moins 80 %, au moins 85 %, au moins 90 %, au moins 93 %, au moins 95 %, au moins 97 %, au moins 98 % ou au moins 99 % après une période de temps prolongée dans des conditions de stockage.

10. Produit ophtalmique selon l'une quelconque des revendications 2, 8 et 9, dans lequel la période de temps prolongée est une parmi : environ 1 semaine, environ 2 semaines, environ 3 semaines, environ 1 mois, environ 2 mois, environ 3 mois, environ 4 mois, environ 5 mois, environ 6 mois, environ 8 mois, environ 10 mois, environ 12 mois, environ 18 mois, environ 24 mois, environ 36 mois, environ 4 ans ou environ 5 ans.
